(19) Europäisches Patentamt

European Patent Office

Office européen des brevets

(11) **EP 1 655 038 A1**

(12) **EUROPEAN PATENT APPLICATION**
published in accordance with Art. 158(3) EPC

(43) Date of publication:
**10.05.2006 Bulletin 2006/19**

(51) Int Cl.:
*A61K 47/24* (1990.01)     *A61K 47/26* (1990.01)
*A61K 47/28* (1990.01)     *A61K 47/36* (1990.01)
*A61K 47/42* (1990.01)     *A61P 35/00* (2000.01)
*A23L 1/302* (1985.01)     *A23L 1/00* (1968.09)

(21) Application number: **04748262.5**

(22) Date of filing: **30.07.2004**

(86) International application number:
**PCT/JP2004/011291**

(87) International publication number:
**WO 2005/011632 (10.02.2005 Gazette 2005/06)**

(84) Designated Contracting States:
**CH DE FR GB LI**

(30) Priority: **01.08.2003  JP  2003285432**
**26.03.2004  JP  2004093872**

(71) Applicant: **National Institute of Advanced Industrial Science and Technology Tokyo 100-8921 (JP)**

(72) Inventors:
• **YAMAZAKI, Noboru**
  **Tsukuba-shi (JP)**
• **TSURUSHIMA, Hideo**
  **Tsukuba-shi (JP)**
• **KOJIMA, Shuuji**
  **Tsukuba-shi (JP)**

(74) Representative: **HOFFMANN EITLE Arabellastrasse 4 81925 München (DE)**

(54) **TARGET-DIRECTED AND ENTERIC ABSORPTION-CONTROLLED LIPOSOME HAVING SUGAR CHAIN AND CANCER REMEDY AND DIAGNOSTIC CONTAINING THE SAME**

(57)    An object of the present invention is to provide a liposome having a sugar chain bonded thereto, the sugar chain having an activity of specifically binding to various lectins (sugar chain recognizing proteins) present on the cell surfaces of various tissues and thus being capable of actually distinguishing a cell and a tissue *in vivo,* thereby efficiently delivering a medicinal drug or a gene.

**Description**

Technical Field

The present invention relates to a liposome that can be used as a therapeutic drug delivery system for recognizing target cells/tissues such as cancer cells and for sending a medicinal drug and a gene locally to an affected part and used as a cell/tissue sensing probe for a diagnostic purpose, both being applicable in the medical/pharmaceutical fields regarding pharmaceutical products and cosmetics. Particularly, the present invention relates to a liposome modified by a sugar chain excellent in intestinal absorption and a liposome preparation prepared by encapsulating a liposome having a medicinal drug and a gene therein.

Background Art

The realization of a "drug delivery system (DDS) for delivering drugs or genes intentionally and intensively to cancer cells or target tissues" has been set as one of the specific goals of the U.S. National Nanotechnology Initiative (NNI). The Nanotechnology/Materials Strategy of the Council for Science and Technology Policy in Japan also focuses research on "Medical micro systems/materials, Nanobiology for utilizing and controlling biological mechanisms," and one of the five year R & D targets is "Establishment of basic seeds in health/life-lengthening technologies such as biodynamic materials and pinpoint treatments." However, even in view of these goals the incidence and morbidity of cancers become higher year after year, along with a progressive aging of the population, and a serious need for the development of a targeting DDS material which is a novel treatment material exists. Targeting DDS nano-structured materials for other diseases also come under the spotlight because they have no side effects, and their market size of over 10 trillion yen is anticipated in the near future. Further, it is expected that these materials will be utilized in medical diagnosis as well as medical treatments.

The therapeutic effect of a drug will be achieved only if the drug reaches a specific target region and acts thereupon. If the drug reaches a non-target region, undesirable side effects may result. Thus, the development of a drug delivery system that allows drugs to be used effectively and safely is also desired. In a drug delivery system, the targeting DDS can be defined as a concept of delivering a drug to a "necessary region in a body," in a "necessary amount" and for a "necessary time-period." A liposome is a noteworthy particulate carrier regarded as a representative material for a targeting DDS. While a passive targeting method based on modification of lipid type, composition ratio, size, or surface charge of liposomes has been developed to impart a targeting function to this particle, this method is still insufficient and required to be improved in many respects. An active targeting method has also been researched in an attempt to achieve a sophisticated targeting function. While the active targeting method referred to as a "missile drug" is conceptually ideal, it has not been accomplished in Japan and abroad, and future developments are expected. This method is designed to provide ligands bonded to the membrane surface of a liposome that will be specifically recognized and bound by a receptor residing on the cell-membrane surface of a target tissue, thereby achieving active targeting. The cell-membrane surface receptor ligands include antigens, antibodies, peptides, glycolipids, and glycoproteins.

It is revealing that the sugar chain of glycolipids and glycoproteins bears an important role as an information molecules in various communications between cells, such as in the creation or morphogenesis of tissues, in the proliferation or differentiation of cells, in the biophylaxis or fecundation mechanism, and in the creation and metastasis of cancers.

Further, research on various types of lectins (sugar-recognizing protein) such as selectin, DC-SIGN, DC-SGNR, a C-type lectin including colectin and mannose binding lectin, an I-type lectin including siglec, a P-type lectin including a mannose-6-phosphate receptor, an R-type lectin, an L-type lectin, an M-type lectin, and galectin, which serve as receptors on cell-membrane surfaces of target tissues, has been proposed to serve as receptors for sugar chains having different molecular structures that may be used as noteworthy new DDS ligands (Yamazaki, N., Kojima, S., Bovin, N. V., Andre, S., Gabius, S. and H. -J. Gabius. *Adv. Drug Delivery Rev.* 43:225-244 (2000); Yamazaki, N., Jigami, Y., Gabius, H. -J., and S. Kojima. *Trends in Glycoscience and Glycotechnology* 13:319-329 (2001); http://www.gak.co.jp/TTGG/71PDF/yamazaki.pdf).

Liposomes having ligands bonded to their external membrane surface have been actively researched in order to provide a DDS material for delivering drugs or genes selectively to a target region, such as cancer. While these liposomes bind to target cells in vitro, most of them do not exhibit adequate targeting to intended target cells or tissues in vivo (Forssen, E. and M. Willis. *Adv. Drug Delivery Rev.* 29:249-271 (1998); Takahashi, T. and M. Hashida. *Today's DDS/Drug Delivery System,* Iyaku Journal Co., Ltd. (Osaka, Japan), 159-167 (1999)). While some research has been conducted on liposomes incorporating glycolipids having sugar chains, for use as a DDS material, these liposomes were evaluated only in vitro, and little progress has been reported for similar research on liposomes incorporating glycoproteins having sugar chains (DeFrees, S. A., Phillips, L., Guo, L. and S. Zalipsky. *J. Am. Chem. Soc.* 118:6101-6104 (1996); Spevak, W., Foxall, C., Charych, D. H., Dasqupta, F. and J. O. Nagy. *J. Med. Chem.* 39:1918-1020 (1996); Stahn, R.,

Schafer, H., Kernchen, F. and J. Schreiber. *Glycobiology* 8:311-319 (1998); Yamazaki, N., Jigami, Y., Gabius, H. -J., and S. Kojima. *Trends in Glycoscience and Glycotechnology* 13:319-329 (2001); http://www.gak.co.jp/TTGG/71PDF/ yamazaki.pdf). As above, systematic research into liposomes having a wide variety of sugar chains, on the glycolipids or glycoproteins bonded to the liposomes, including preparative methods and in vivo analyses thereof, is pending and represents an important challenge to be progressed in future.

Leukemia is one of the cancers whose pathological analysis and therapy are mostly advanced. The therapy of leukemia is performed primarily based on chemotherapy. Since cancer may possibly be reduced (cured) by chemotherapy, chemotherapy is currently applied intensively, with the result that severe side effect is accompanied.

Recently, it has been reported that the number of elderly leukemic patients increases with an increase of the elderly persons. In acute myelocytic leukemia, not less than 60 year-old patients come to occupy the half of the patients. However, the recent progress of leukemia therapy is not always conducible to improvement of therapeutic achievement for the elderly leukemic patients. This is considered as follows. One is that unfavorable symptoms such as chromosomal abnormality and emergence of drug resistant leukemia cells are frequently observed in elderly patients. Another is that infectious disease complication and myelopathy ascribed to administration of anticancer drugs take place and the organs become less tolerable against damage caused by anticancer drugs. For these reasons, intensive chemotherapy cannot be applied to elderly persons at present in the same manner as that applied to younger persons.

To solve these problems, methods for improving response to anticancer drugs, and a molecule-targeting drug have been developed. Of them, development of a drug delivery system (hereinafter referred to as "DDS"), which brings an ideal drug distribution (delivery), is considered as one of the most important solutions. If a medicinal drug is desirably delivered by the DDS, the degree of damage that the organs suffer can be reduced and cytotoxic damage that the leukemia cells suffer can be enhanced, leading to an improvement of therapeutic achievement of elderly leukemic patients.

Further, in research on new types of DDS materials, it is an important challenge to develop a DDS material capable of being orally administered in the easiest and cheapest way. For example, when a peptide medicine is orally administered, it is subject to enzymolysis and may be only partially absorbed in the intestine due to its water solubility, high molecular weight, and low permeability in the mucosa of small intestine. As an alternative, a ligand-bonded liposome is getting attention as a potential DDS material for delivering high molecular-weight medicines or genes into the blood stream through the intestine (Lehr, C.-M. *J. Controlled Release* 65:19-29 (2000)). However, results from research into an intestinal absorption-controlled liposome, using a sugar chain as the ligand, have not been reported.

We have filed a patent application of a sugar-modified liposome wherein a sugar chain is bound to the liposome through a linker protein, the sugar chain is selected from Lewis X trisaccharide, sialyl Lewis X tetrasaccharide, 3'-sialyllactosamine trisaccharide, and 6'-sialyllactosamine trisaccharide, tris (hydroxymethyl) aminomethane is optionally bound to the liposome membrane and/or linker protein and hydrophilized, and an intestinal absorption-controlled liposome wherein the liposome is modified with a sugar chain selected from lactose disaccharide, 2'-fucosyllactose trisaccharide, difucosyllactose tetrasaccharide and 3-fucosyllactose trisaccharide and the sugar chian may be bound to the liposome through a linker protein.

Disclosure of the Invention

An object of the present invention is to provide a liposome to which a sugar chain bonded, the sugar chain having a specific binding activity to various types of lectins (sugar chain recognition protein) present in cell surfaces of various types of tissues, and the liposome being capable of recognizing cells and tissues *in vivo,* thereby efficiently delivering a medicinal drug or a gene to the cells and tissues. Another object of the present invention is to provide a therapeutic drug containing the liposome. Still another object of the present invention is to provide a highly stable liposome.

To attain the objects mentioned above, the present inventors have conducted studies on the composition of a liposome. As the result, they obtained a highly stable liposome. They further conducted various experimental studies on the type and binding density of the sugar chain to be bonded to the surface of a liposome, linker proteins and a compound hydrophilizing the liposome. As a result, they found that the accuracy of targeting of a liposome to each tissue is actually and successfully controlled by the structure of a sugar chain. In addition, they found that the amount of liposome delivered to each tissues is successfully increased by hydrating a liposome surface and/or a linker protein with a predetermined hydrophilic compound and further controlling the density of a sugar chain to be bonded to the liposome, thereby attaining efficient delivery of a medicinal drug or a gene to a target cell and tissue. Based on these findings, they accomplished the present invention.

The present inventors further conducted intensive studies on application of the liposome obtained in this manner to practical therapy for a disease. As a result, they found that such a liposome is successfully applied to treatments for various diseases of tissues and organs by varying the type of sugar chain bonded onto the surface of the liposome. Based on the finding, they accomplished the present invention.

The present inventors succeeded in reducing the toxicity of an anticancer drug, doxorubicin, whose toxicity has been a matter of concern, by encapsulating it in a liposome. However, this achievement is not sufficient because although the safety of the drug increases, cytotoxicity against leukemic cells decreases. Then, they contrived to bind a sugar chain to a liposome such that a liposome is actively directed to leukemia cells. In this manner, they accomplished a DDS.

To describe more specifically, the present invention is directed to the following items 1 to 79.

1. A liposome modified by a sugar chain having a sugar chain bonded to the membrane of the liposome.

2. The liposome modified by a sugar chain according to 1., wherein a constitutional lipid of the liposome comprises phosphatidylcholines (0 to 70% by mole), phosphatidylethanolamines (0 to 30% by mole); not less than one lipid (0 to 30% by mole) selected from the group consisting of phosphatidic acids, long-chain alkyl phosphates and dicetyl phosphates; not less than one lipid (0 to 40% by mole) selected from the group consisting of gangliosides, glycolipids, phosphatidylglycerols, and sphingomyelins; and cholesterols (0 to 70% by mole).

3. The liposome modified by a sugar chain according to 2., wherein the at least one lipid selected from the group consisting of gangliosides, glycolipids, phosphatidylglycerols, sphingomyelins and cholesterols gathers on the surface of the liposome to form a raft.

4. The liposome modified by a sugar chain according to any of 1. to 3., wherein the sugar chain controlled in type and density is bonded.

5. The liposome modified by a sugar chain according to any of 1. to 4., wherein the liposome has a particle diameter of 30 to 500 nm.

6. The liposome modified by a sugar chain according to 5., wherein the liposome has a particle diameter of 50 to 350 nm.

7. The liposome modified by a sugar chain according to any of 1. to 6., wherein the liposome has a zeta potential of -50 to 10 mV.

8. The liposome modified by a sugar chain according to 7., wherein the liposome has a zeta potential of -40 to 0 mV.

9. The liposome modified by a sugar chain according to 8., wherein the liposome has a zeta potential of -30 to -10 mV.

10. The liposome modified by a sugar chain according to any of 1. to 9., wherein the sugar chain is bonded to the membrane of the liposome via a linker protein.

11. The liposome modified by a sugar chain according to 10., wherein the linker protein is a protein derived from the living body.

12. The liposome modified by a sugar chain according to 11., wherein the linker protein is a protein derived from a human.

13. The liposome modified by a sugar chain according to 12., wherein the linker protein is a human serum protein.

14. The liposome modified by a sugar chain according to 11., wherein the linker protein is human serum albumin or bovine serum albumin.

15. The liposome modified by a sugar chain according to any of 1. to 14., wherein the linker protein is bonded onto a raft formed of at least one lipid selected from the group consisting of gangliosides, glycolipids, phosphatidylglycerols, sphingomyelins and cholesterols formed on the surface of the liposome.

16. The liposome modified by a sugar chain according to any of 1. to 15., hydrophilized by binding a hydrophilic compound to the membrane of the liposome and/or the linker protein.

17. The liposome modified by a sugar chain according to 16., wherein the hydrophilic compound is a low molecular weight substance.

18. The liposome modified by a sugar chain according to 16. or 17., wherein the hydrophilic compound rarely causes steric hindrance to the sugar chain and does not prevent proceeding of a reaction of recognizing the sugar chain by a lectin on the membrane surface of a target cell.

19. The liposome modified by a sugar chain according to any of 16. to 18., wherein the hydrophilic compound has a hydroxide group.

20. The liposome modified by a sugar chain according to any of 16. to 19., wherein the hydrophilic compound is an amino alcohol.

21. The liposome modified by a sugar chain according to any of 16. to 20., wherein the hydrophilic compound binds directly to the surface of the liposome membrane.

22. The liposome modified by a sugar chain according to 16., hydrophilized by a hydrophilic compound, wherein the hydrophilic compound is represented by the general formula (1):

$$X\text{-}R1(R2OH)n \qquad (1)$$

where R1 denotes a C 1 to C40 linear or branched hydrocarbon chain; R2 is absent or represents a C1 to C40 linear or branched hydrocarbon chain; X represents a reactive functional group directly binding to a liposome lipid or a linker protein, or binding to a divalent crosslinking agent; and n is a natural number.

23. The liposome modified by a sugar chain according to 16., hydrophilized by a hydrophilic compound, wherein the hydrophilic compound is represented by the general formula (2):

$$H_2N\text{-}R3\text{-}(R4OH)n \qquad (2)$$

where R3 denotes a C 1 to C40 linear or branched hydrocarbon chain; R4 is absent or represents a C1 to C40 linear or branched hydrocarbon chain; $H_2N$ represents a reactive functional group directly binding to a liposome lipid or a linker protein, or binding to a divalent crosslinking agent; and n is a natural number.

24. The liposome modified by a sugar chain according to 16., hydrophilized by a hydrophilic compound, wherein the hydrophilic compound is represented by the general formula (3):

$$H_2N\text{-}R5\text{-}(OH)n \qquad (3)$$

where R5 denotes a C1 to C40 straight and branched hydrocarbon chain; $H_2N$ represents a reactive functional group directly binding to a liposome lipid or a linker protein, or binding to a divalent crosslinking agent; and n is a natural number.

25. The liposome modified by a sugar chain according to 16., wherein the membrane of the liposome and/or the linker protein are hydrophilized by covalently bonding a hydrophilic compound being a tris(hydroxyalkyl)aminoalkane to the membrane of the liposome and/or the linker protein.

26. The liposome modified by a sugar chain according to 25., wherein the membrane of the liposome and/or the linker protein are hydrophilized by covalently bonding, to the membrane of the liposome and/or the linker protein, a hydrophilic compound selected from the group consisting of tris(hydroxymethyl)aminoethane, tris(hydroxyethyl) aminoethane, tris(hydroxypropyl)aminoethane, tris(hydroxymethyl)aminomethane, tris(hydroxyethyl)aminomethane, tris(hydroxypropyl)aminomethane, tris(hydroxylmethyl)aminopropane, tris(hydroxyethyl)aminopropane, and tris(hydroxypropyl)aminopropane.

27. The liposome modified by a sugar chain according to any of 1. to 26., wherein the liposome modified by a sugar chain is targeted to a lectin selected from the group consisting of a selectin, DC-SIGN, DC-SGNR, a C-type lectin including colectin and mannose binding lectin, an I-type lectin including siglec, a P-type lectin including a mannose-6-phosphate receptor, an R-type lectin, an L-type lectin, an M-type lectin, and galectin, all serving as a receptor present on the cellular membrane of each tissue.

28. The liposome modified by a sugar chain according to 28., wherein the liposome modified by a sugar chain is targeted to a selectin selected from the group consisting of E-selectin, P-selectin, and L-selectin.

29. The liposome modified by a sugar chain according to any of 1 to 28., wherein a binding density of the sugar chain bonded to the liposome is 1 to 30000 per liposome particle when the linker protein is used, and 1 to 500000 per liposome particle when the linker protein is not used.

30. The liposome modified by a sugar chain according to any of 1. to 28., wherein a binding density of the sugar chain bonded to the liposome is 1 to 60 per protein molecule to be bonded to the liposome.

31. The liposome modified by a sugar chain according to any of 1. to 30., wherein the liposome is capable of being absorbed from the intestinal tract.

32. The liposome modified by a sugar chain according to any of 1. to 31., having high targetability to a tissue or an organ selected from the group consisting of blood, liver, spleen, lung, brain, small intestinal tract, heart, thymus gland, kidney, pancreas, muscle, large intestinal tract, bone, bone marrow, eyes, cancer tissue, inflammatory tissue and lymph node.

33. The liposome modified by a sugar chain according to any of 1. to 32., wherein the sugar chain is bonded to the membrane of the liposome and selected from the group consisting of α-1,2-mannobiose disaccharide, α-1,3-mannobiose disaccharide, α-1,4-mannobiose disaccharide, α-1,6-mannobiose disaccharide, α-1,3/α-1,6-mannotriose trisaccharide, oligomannose-3 pentasaccharide, oligomannose-4b hexasaccharide, oligomannose-5 heptasaccharide, oligomannose-6 octasaccharide, oligomannose-7 nonasaccharide, oligomannose-8 decasaccharide, and oligomannose-9 undecasaccharide, 3'-sialyllactose trisaccharide, 6'-sialyllactose trisaccharide, 3'-sialyllactosamine trisaccharide, 6'-sialyllactosamine trisaccharide, Lewis X trisaccharide, sialyl Lewis X tetrasaccharide, lactose disaccharide, 2'-fucosyllactose trisaccharide, difucosyllactose tetrasaccharide, and 3-fucosyllactose trisaccharide.

34. A liposome preparation prepared by encapsulating a medicinal drug or a gene in the liposome according to any of 1. to 33.

35. The liposome preparation according to 34., wherein the medicinal drug is selected from the group consisting of medicinal drugs for tumors such as an anticancer drug based on an alkylating compound, metabolic antagonist, plant derived anticancer drug, anticancer antibiotic, BRM, cytokine, anticancer drug based on a platinum complex, immunotherapeutic drug, hormonal anticancer drug, and monoclonal antibody; medicinal drugs for the central nerve; medicinal drugs for the peripheral nervous system/sensory organ; therapeutic drugs for a respiratory disease; medicinal drugs for a circulatory organ; medicinal drugs for a digestive organ; hormonal medicinal drugs; medicinal drugs for a urinary/genital organ; medicinal drugs for external application; vitamins/analeptics; medicinal drugs for blood and body fluid; metabolic medicine; antibiotic/chemotherapeutic agents; medicinal drugs for medical check; anti-inflammatory agents; medicinal drugs for eye disorder; medicinal drugs for the central nervous system; medicinal drugs for the autoimmune system; medicinal drugs for the circulatory organ; medicinal drugs for lifestyle-related diseases such as diabetes and hyperlipemia; various oral, pulmonary, transdermal or transmucosal drugs; adenocortical hormones; immunosuppressive agents; antibiotics; anti-viral agents; neovascularization inhibitors; cytokines; chemokines; anti-cytokine antibodies; anti-chemokine antibodies; anti-cytokine/chemokine receptor antibodies; nucleic acid preparations involved in gene therapy such as siRNA, miRNA, smRNA, antisense ODN and DNA; neuroprotective factors; and various antibody medicines.

36. The liposome preparation according to claim 34 or 35, which is an oral preparation.

37. The liposome preparation according to 34. or 35., which is a parenteral preparation.

38. The liposome preparation according to 35., wherein the medicinal drug comprising a liposome modified by a sugar chain is doxorubicin.

39. An anticancer drug comprising the liposome preparation according to 35., wherein the medicinal drug is a drug for a tumor.

40. The anticancer drug according to 39. which is to be orally administered.

41. The anticancer drug according to 39. which is to be parenterally administered.

42. A liposome whose membrane is hydrophilized and which has no sugar chain bonded onto surface thereof

43. The liposome according to 42., wherein a constitutional lipid of the liposome comprises phosphatidylcholines (0 to 70% by mole), phosphatidylethanolamines (0 to 30% by mole); not less than one lipid (0 to 30% by mole) selected from the group consisting of phosphatidic acids, long-chain alkyl phosphates and dicetyl phosphates; not less than one lipid (0 to 40% by mole) selected from the group consisting of gangliosides, glycolipids, phosphatidylglycerols, and sphingomyelins; and cholesterols (0 to 70% by mole).

44. The liposome according to 42. or 43., further comprising a protein.

45. The liposome according to any of 42. to 44., wherein the membrane of the liposome and/or the linker protein are hydrophilized by binding a hydrophilic compound thereto.

46. The liposome according to 45., wherein the hydrophilic compound is a low molecular weight substance.

47. The liposome according to 45. or 46., wherein the hydrophilic compound rarely causes steric hindrance to the sugar chain and does not prevent proceeding of a reaction of recognizing the sugar chain by a lectin on the membrane surface of a target cell.

48. The liposome according to any of 45. to 47., wherein the hydrophilic compound has a hydroxide group.

49. The liposome according to any of claims 45 to 48, wherein the hydrophilic compound is an amino alcohol.

50. The liposome according to any of 45. to 49., wherein the hydrophilic compound binds directly to the surface of the liposome membrane.

51. The liposome according to 45., wherein the hydrophilic compound of a low molecular weight has at least one OH group.

52. The liposome according to 45., wherein the hydrophilic compound is represented by the general formula (1):

$$X\text{-}R1(R2OH)n \qquad (1)$$

where R1 denotes a C1 to C40 linear or branched hydrocarbon chain; R2 is absent or represents a C1 to C40 linear or branched hydrocarbon chain; X represents a reactive functional group directly binding to a liposome lipid or a linker protein or binding to a divalent crosslinking agent; and n is a natural number.

53. The liposome according to 45., wherein the hydrophilic compound is represented by the general formula (2):

$$H_2N\text{-}R3\text{-}(R4OH)n \qquad (2)$$

where R3 denotes a C1 to C40 linear or branched hydrocarbon chain; R4 is absent or represents a C1 to C40 linear or branched hydrocarbon chain; $H_2N$ represents a reactive functional group directly binding to a liposome lipid or a linker protein or binding to a divalent crosslinking agent; and n is a natural number.

54. The liposome according to 45., wherein the hydrophilic compound is represented by the general formula (3):

$$H_2N\text{-}R5\text{-}(OH)n \qquad (3)$$

where R5 denotes a C1 to C40 linear or branched hydrocarbon chain; $H_2N$ represents a reactive functional group directly binding to a liposome lipid or a linker protein or binding to a divalent crosslinking agent; and n is a natural number.

55. The liposome according to 45., wherein the membrane of the liposome and/or the linker protein are hydrophilized by covalently bonding a hydrophilic compound being a tris(hydroxyalkyl)aminoalkane to the membrane of the liposome and/or the linker protein.

56. The liposome according to 55., wherein the membrane of the liposome is hydrophilized by covalently bonding, to the membrane of the liposome, a hydrophilic compound selected from the group consisting of tris(hydroxymethyl) aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxylmethyl)aminopropane, tris(hydroxyethyl)aminopropane, and tris(hydroxypropyl)aminopropane.

57. A liposome preparation prepared by encapsulating a medicinal drug or a gene in the liposome according to any of 42. to 56.

58. The liposome preparation according to 57., wherein the medicinal drug is selected from the group consisting of medicinal drugs for tumors such as an anticancer drug based on an alkylating compound, metabolic antagonist, plant derived anticancer drug, anticancer antibiotic, BRM, cytokine, anticancer drug based on a platinum complex, immunotherapeutic drug, hormonal anticancer drug, and monoclonal antibody; medicinal drugs for the central nerve; medicinal drugs for the peripheral nervous system/sensory organ; therapeutic drugs for a respiratory disease; medicinal drugs for a circulatory organ; medicinal drugs for a digestive organ; hormonal medicinal drugs; medicinal drugs for a urinary/genital organ; medicinal drugs for external application; vitamins/analeptics; medicinal drugs for blood and body fluid; metabolic medicine; antibiotic/chemotherapeutic agents; medicinal drugs for medical check; anti-inflammatory agents; medicinal drugs for eye disorder; medicinal drugs for the central nervous system; medicinal drugs for the autoimmune system; medicinal drugs for the circulatory agent; medicinal drugs for lifestyle-related diseases such as diabetes and hyperlipemia; various oral, pulmonary, transdermal or transmucosal drugs; adenocortical hormones; immunosuppressive agents; antibiotics; anti-viral agents; neovascularization inhibitors; cytokines; chemokines; anti-cytokine antibodies; anti-chemokine antibodies; anti-cytokine/chemokine receptor antibodies; nucleic acid preparations involved in gene therapy such as siRNA, miRNA, smRNA, antisense ODN and DNA; neuroprotective factors; and various antibody medicines.

59. The liposome preparation according to 57. or 58., being a preparation for peroral administration.

60. The liposome preparation according to 57. or 58., which is a parenteral preparation.

61. An anticancer drug comprising the liposome preparation according to 58., wherein the medicinal drug is a drug for a tumor.

62. The liposome preparation according to 61., wherein the medicinal drug is doxorubicin.

63. The anticancer drug according to 61. or 62., which is to be orally administered.

64. The anticancer drug according to 61. or 62., which is to be parentally administered.

65. A cosmetic composition comprising a liposome preparation prepared by encapsulating a cosmetic in the liposome modified by a sugar chain according to any of 1. to 33.

66. The cosmetic composition according to 65., which is a preparation to be transdermally administered.

67. The cosmetic composition according to 65. or 66., wherein the cosmetic is vitamin A or vitamin E.

68. A food composition comprising a liposome preparation prepared by encapsulating a food in the liposome modified by a sugar chain according to any of 1. to 33.

69. The food composition according to 68., which is a preparation for peroral administration.

70. The food composition according to 68. or 69., wherein the food is a nutritional supplement.

71. The food composition according to 69. or 70., wherein the food is vitamin A or vitamin E.

72. A cosmetic composition comprising a liposome preparation prepared by encapsulating a cosmetic in the liposome according to any of 42. to 56.

73. The cosmetic composition according to 72., being a preparation for transdermal administration.

74. The cosmetic composition according to 72. or 73, wherein the cosmetic is vitamin A or vitamin E.

75. A food composition comprising a liposome preparation prepared by encapsulating a food in the liposome according to any of 42. to 56.

76. The food composition according to 75., which is a preparation for oral administration.

77. The food composition according to 75. or 76., wherein the food is a nutritional supplement.

78. The food composition according to 76. or 77., wherein the food is vitamin A or vitamin E.

The present specification includes the contents described in the specifications and/or the drawings of JP Patent Application Nos. 2003-285432 and 2004-093872 based on which the priority of the present application is claimed.

Brief Description of the Drawings

Figure 1 is a schematic illustration showing the structure of a liposome having $\alpha$-1,2-mannobiose disaccharide bonded thereto;

Figure 2 is a schematic illustration showing the structure of a liposome having α-1,3-mannobiose disaccharide bonded thereto;

Figure 3 is a schematic illustration showing the structure of a liposome having α-1,4-mannobiose disaccharide bonded thereto;

Figure 4 is a schematic illustration showing the structure of a liposome having α-1,6-mannobiose disaccharide bonded thereto;

Figure 5 is a schematic illustration showing the structure of a liposome having α-1,3/α-1,6-mannotriose trisaccharide bonded thereto;

Figure 6 is a schematic illustration showing the structure of a liposome having oligomannose-3 pentasaccharide-bonded thereto;

Figure 7 is a schematic illustration showing the structure of a liposome having oligomannose-4b hexasaccharide bonded thereto;

Figure 8 is a schematic illustration showing the structure of a liposome having oligomannose-5 heptasaccharide bonded thereto;

Figure 9 is a schematic illustration showing the structure of a liposome having oligomannose-6 octasaccharide bonded thereto;

Figure 10 is a schematic illustration showing the structure of a liposome having oligomannose-7 nonasaccharide bonded thereto;

Figure 11 is a schematic illustration showing the structure of a liposome having oligomannose-8 decasaccharide bonded thereto;

Figure 12 is a schematic illustration showing the structure of a liposome having oligomannose-9 undecasaccharide bonded thereto;

Figure 13 is a graph showing the distribution amounts of 13 types of liposome complexes in blood 60 minutes after intravenous injection;

Figure 14 is a graph showing the distribution amounts of 13 types of liposome complexes in the liver 60 minutes after intravenous injection;

Figure 15 is a graph showing the distribution amounts of 13 types of liposome complexes in the spleen 60 minutes after intravenous injection;

Figure 16 is a graph showing the distribution amounts of 13 types of liposome complexes in the lung 60 minutes after intravenous injection;

Figure 17 is a graph showing the distribution amounts of 13 types of liposome complexes in the brain 60 minutes after intravenous injection;

Figure 18 is a graph showing the distribution amounts of 13 types of liposome complexes in the cancer tissue 60 minutes after intravenous injection;

Figure 19 is a graph showing the distribution amounts of 13 types of liposome complexes in the lymph node 60 minutes after intravenous injection;

Figure 20 is a graph showing the distribution amounts of 13 types of liposome complexes in the thymus gland 60 minutes after intravenous injection;

Figure 21 is a graph showing the distribution amounts of 13 types of liposome complexes in the heart 60 minutes after intravenous injection;

Figure 22 is a graph showing the distribution amounts of 13 types of liposome complexes in the small intestinal tract 60 minutes after intravenous injection;

Figure 23 is a schematic illustration showing a structure of a liposome having 3'-sialyllactose trisaccharide bounded thereto;

Figure 24 is a schematic illustration showing a structure of a liposome having 6'-sialyllactose trisaccharide bounded thereto;

Figure 25 is a schematic illustration showing a structure of a liposome having 3'-sialyllactosamine trisaccharide bounded thereto;

Figure 26 is a schematic illustration showing a structure of a liposome having 6'-sialyllactosamine trisaccharide bounded thereto;

Figure 27 is a graph showing the delivery amount of 4 types of liposome complexes in the blood 10 minutes after injection into the intestinal tract;

Figure 28 is a graph showing the delivery amount of 4 types of liposome complexes in the blood 10 minutes after injection into the intestinal tract;

Figure 29 is a graph showing the delivery amount of 4 types of liposome complexes in the blood 10 minutes after injection into the intestinal tract;

Figure 30 is a graph showing the delivery amount of 4 types of liposome complexes in the blood 10 minutes after injection into the intestinal tract;

Figure 31 is a graph showing the delivery amount of 4 types of liposome complexes in the blood 10 minutes after injection into the intestinal tract;

Figure 32 is a schematic illustration of a liposome having a tris(hydroxymethyl)aminomethane (used as a comparative compound) bonded thereto;

Figure 33 is a schematic illustration showing the structure of a liposome having Lewis X trisaccharide bonded thereto;

Figure 34 is a schematic illustration showing the structure of a liposome having sialyl Lewis X tetrasaccharide bonded thereto;

Figure 35 is a schematic illustration showing the structure of a liposome modified by lactose disaccharide;

Figure 36 is a schematic illustration showing the structure of a liposome modified by 2'-fucosyllactose trisaccharide;

Figure 37 is a schematic illustration showing the structure of a liposome modified by difucosyllactose tetrasaccharide;

Figure 38 is a schematic illustration showing the structure of a liposome modified by 3-fucosyllactose trisaccharide;

Figure 39 is a graph showing the carcinostatic effect on mice carrying cancer by injection of a liposome having $\alpha$-1,6-mannobiose disaccharide bonded thereto to the caudal vein;

Figure 40 is a photograph showing the carcinostatic effect on mice carrying cancer by injection of a liposome having $\alpha$-1,6-mannobiose disaccharide bonded thereto to the caudal vein;

Figure 41 is a graph showing the carcinostatic effect on mice carrying cancer by oral injection of a liposome having $\alpha$-3-fucosyllactose trisaccharide bonded thereto;

Figure 42 is a photograph showing the carcinostatic effect on mice carrying cancer by oral injection of a liposome having $\alpha$-3-fucosyllactose trisaccharide bonded thereto;

Figure 43 is a graph showing the results of a test of increasing the cytotoxicity of L-Dox and L-Dox-SLX by interferon $\alpha$;

Figure 44 is a graph showing the results of a test of increasing the cytotoxicity of L-Dox-SLX by an L-selectin neutralizing antibody at the time IFN is added;

Figure 45 is a graph showing change in concentration of doxorubicin in blood with time in a mouse carrying cancer;

Figure 46 is a graph showing change in concentration of doxorubicin in a tumor with time in a mouse carrying cancer;

Figure 47 is a micrograph showing distribution of doxorubicin in tumor tissue and cells in a mouse carrying cancer;

Figure 48 is a graph showing the carcinostatic effect of a drug on mice carrying cancer by caudal vein injection;

Figure 49 is a photograph showing the carcinostatic effect of a drug on mice carrying cancer by caudal vein injection;

Figure 50 is a graph showing the weight of tumor for each control group, showing the carcinostatic effect of drugs in mice carrying cancer by caudal vein injection;

Figure 51 is a graph showing the blood retention of liposomes hydrophilized by two types of compounds in comparison with a liposome unhydrophilized in mice carrying cancer 5 minutes after caudal vein injection;

Figure 52 is a photograph of the limb of an RA mouse having inflammation;

Figure 53 is a graph showing the results of therapy by prednisolone encapsulated DDS intravenous injection to an RA mouse;

Figure 54 is a graph showing the results of therapy by prednisolone encapsulated DDS oral injection to an RA mouse; and

Figure 55 is a graph showing the results of therapy by administrating prednisolone in an appropriate amount to an RA mouse.

Best Mode for Carrying Out the Invention

The present invention will be described in more detail below.

The present invention is directed to a targeting liposome and an intestinal-absorption controlled liposome having various sugar chains on the surface. In the present invention, the term "targeting" refers to an ability of a substance to reach a specific target site such as a predetermined tissue, organ, or a lesion e.g., cancer, when the substance is injected *in vivo* and then to be taken up by the site. The term "intestinal-absorption controlled" refers to a nature of a substance of being absorbed *in vivo* via the intestinal tract, in other words, "absorbability from the intestinal tract", which further includes a property of controlling the absorption rate and degree.

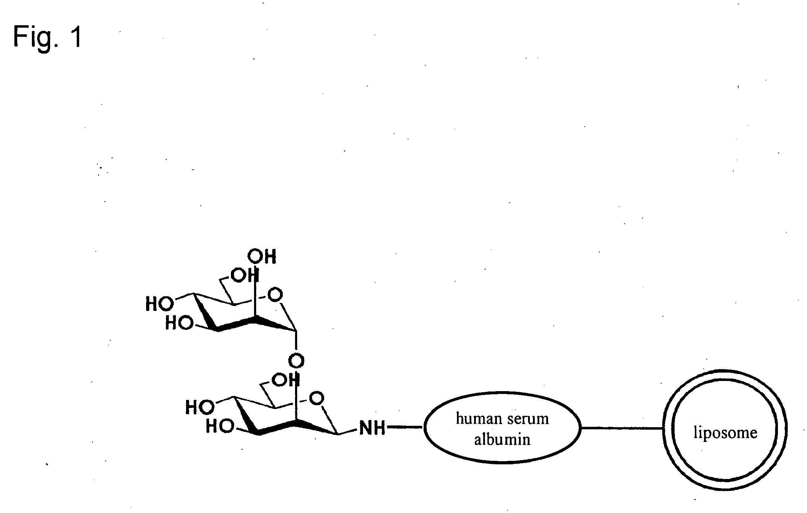

The "liposome" generally refers to a closed vesicle composed of a lipid layer, which is a membrane-form lipid assembly and an aqueous layer formed in the lipid layer. The liposome of the present invention has sugar chains bonded onto surface, that is, the lipid layer, as shown in Figures 1 to 12, 23 to 26 and 32 to 38. The sugar chain may be bonded to the lipid layer of a liposome directly or covalently via a linker, which is a biological protein including a protein derived from a human, such as human serum albumin. The sugar chain bonded herein is limited in type and density.

Various types of sugar chains may be used depending upon the tissue, organ or cancer to which an intestinal-absorption controlled liposome and a targeting liposome according to the present invention are targeted.

It is known that E-selectin and P-selectin, which express on the vascular endothelial cells of a lesion, strongly bind to sialyl Lewis X, which is a sugar chain expressing on the cellular membrane of leukocytes. To the liposome of the invention, the sialyl Lewis X sugar chain or an analogous sugar chain, which is reactive to a protein such as a lectin (e.g., E-selectin, P-selectin) having a sugar chain binding site, is bonded while being limited in type and density. It is considered that the liposomes are specifically accumulated to a lesional site such as cancer whose vascular endothelial cells express E-selectin and P-selectin. In the sites expressing E-selectin and P-selectin, inflammation and vascularization take place. In the blood vessel of these sites, the intercellular space of the endothelial cells is enlarged. Therefore, the accumulated liposomes are conceivably diffused from the intercellular space to the lesional site and its peripheral region. The liposomes thus diffused are taken up (through endocytosis) by the cells of the lesional site and the peripheral region and intracellularly release a drug encapsulated therein. In this mechanism, the liposomes produce an effect on inflammatory diseases or cancer.

As the sugar chain to be bonded to the liposome of the present invention, mention may be made of a sugar chain reactive to a protein, such as E-selectin and P-selectin, having a sugar chain binding site as mentioned above. The E-selectin and P-selectin herein refer to various types of lectins (sugar-chain recognizing proteins), which include C-type lectins such as selectin, DC-SIGN, DC-SGNR, colectin, mannose binding protein, I-type lectins such as siglec, P-type lectins such as a mannose-6-phosphate receptor, R-type lectin, L-type lectin, M-type lectin, and galectin. The target cells to which the liposome of the present invention is directed vary in type of protein, such as lectin, having a sugar-chain binding site expressing depending upon the cells. Therefore, a liposome capable of targeting specifically to a predetermined cell can be obtained by selecting a type of sugar chain depending upon the type of protein.

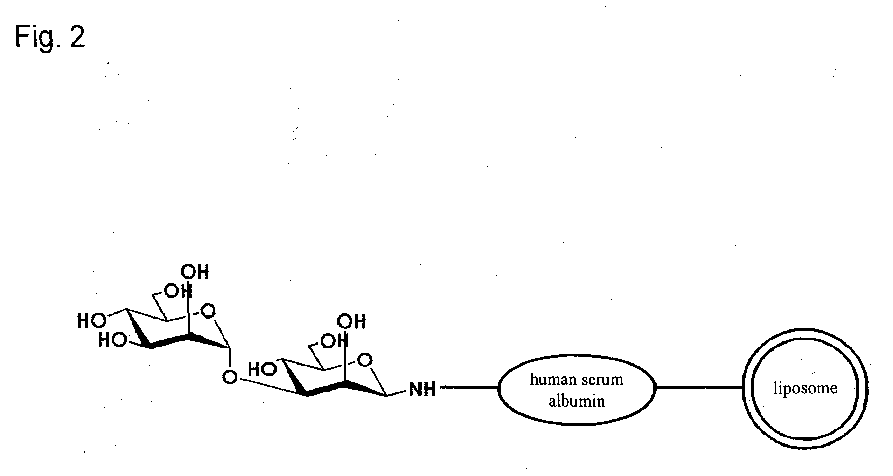

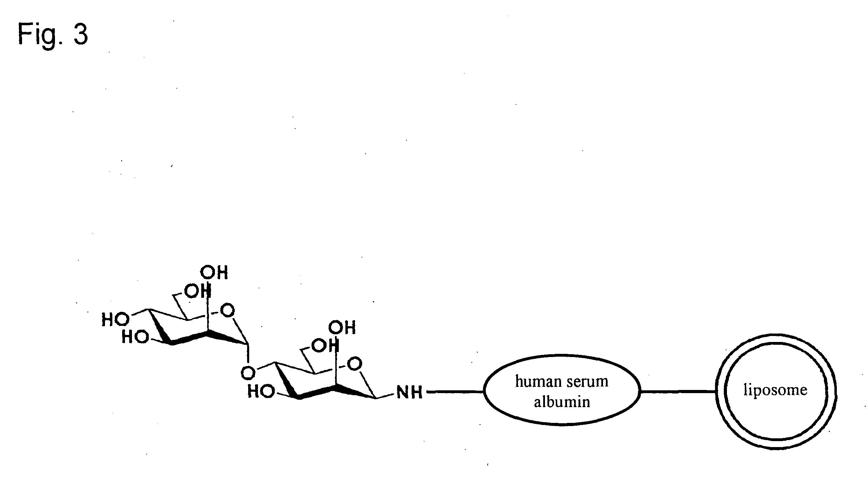

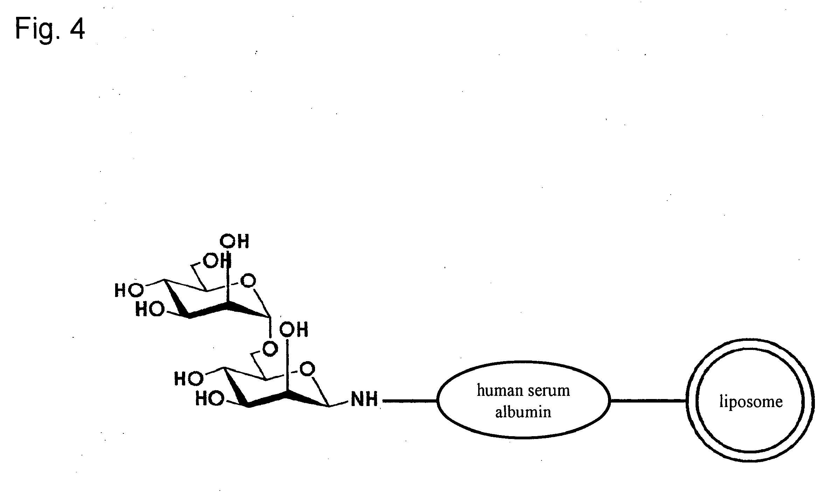

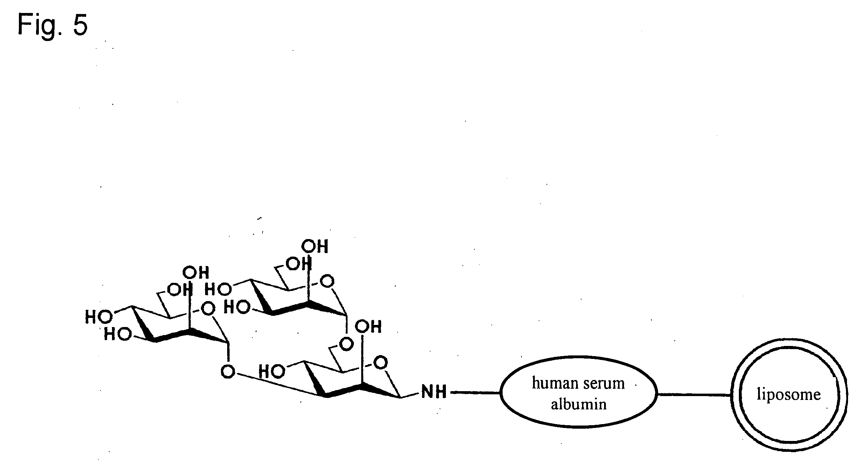

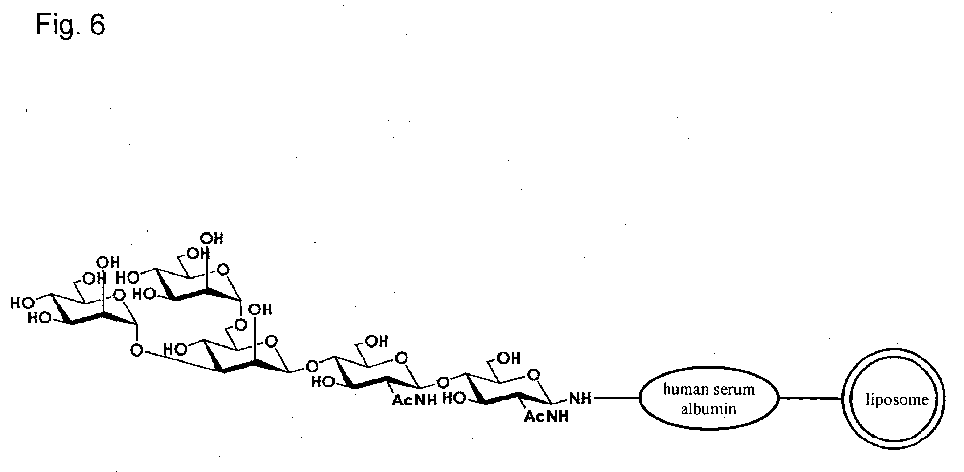

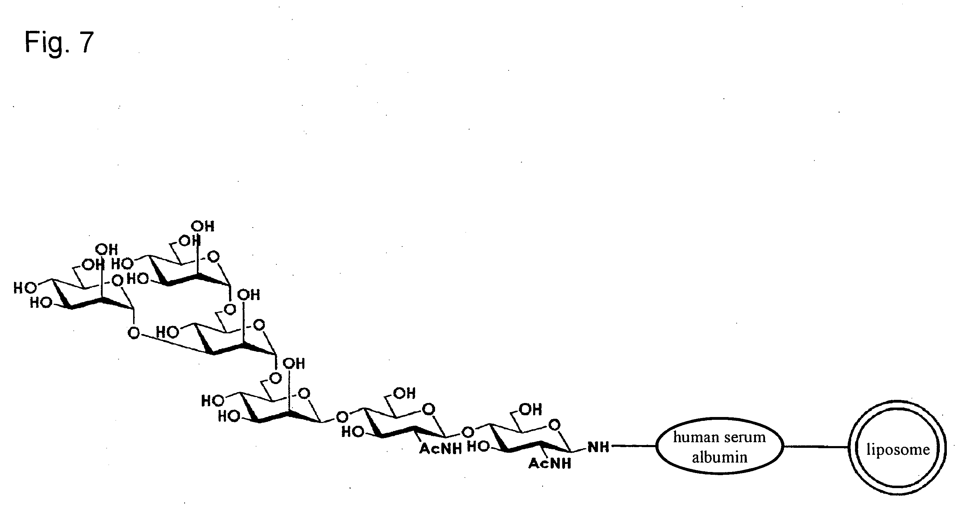

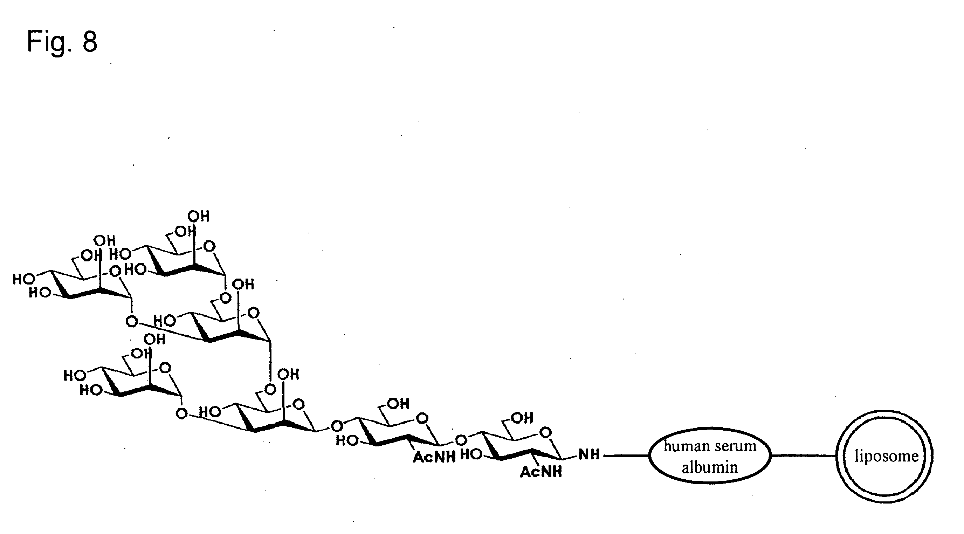

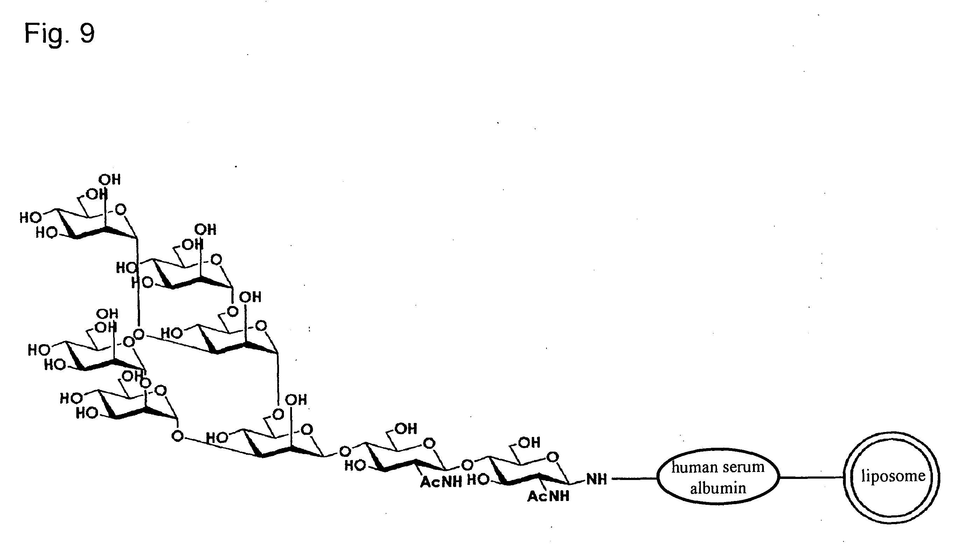

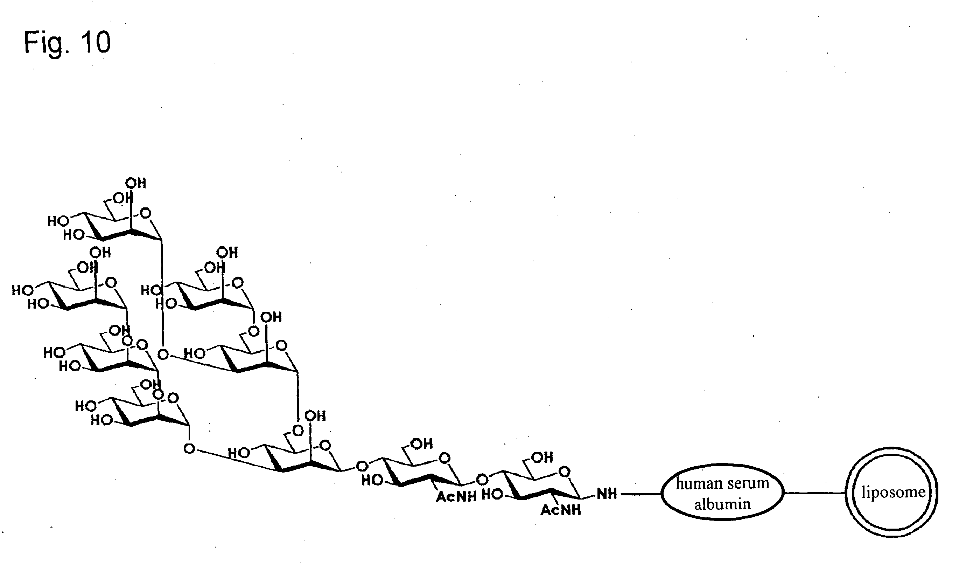

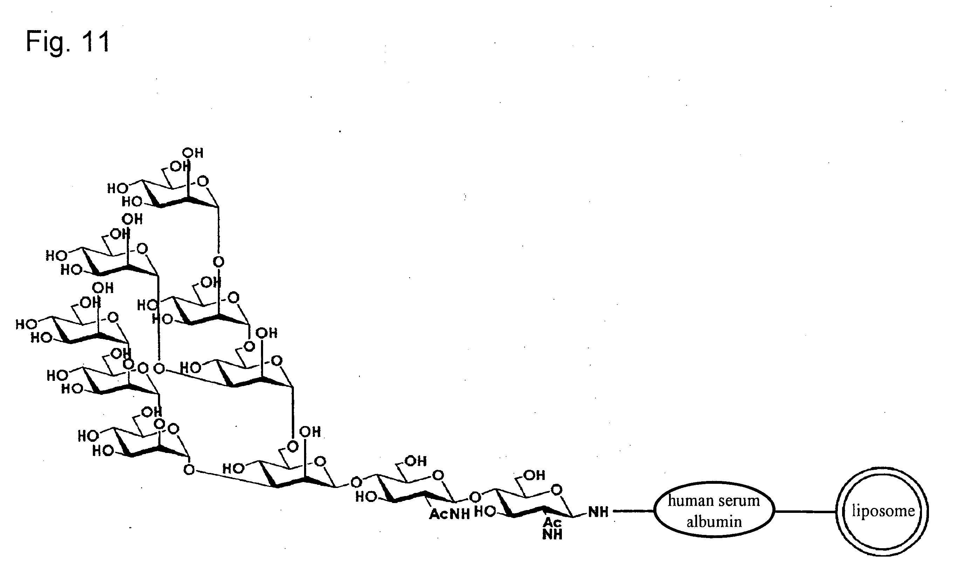

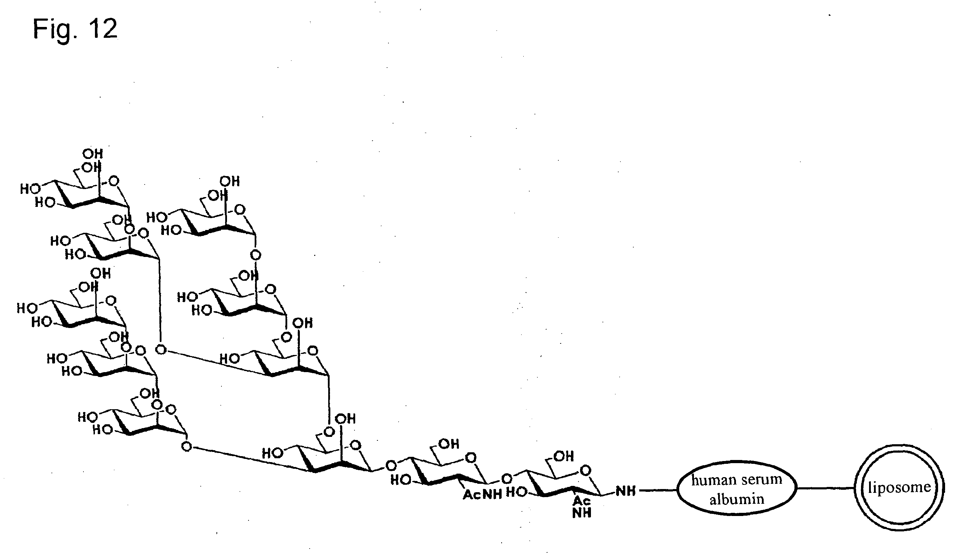

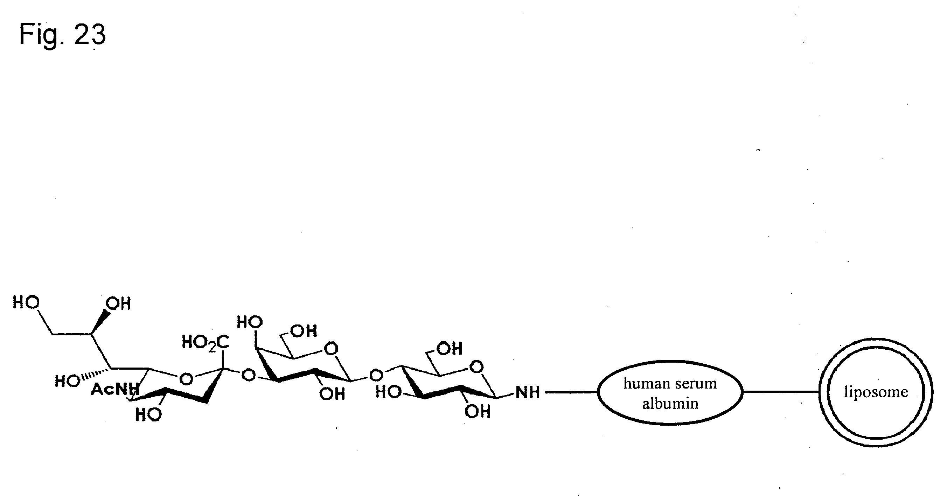

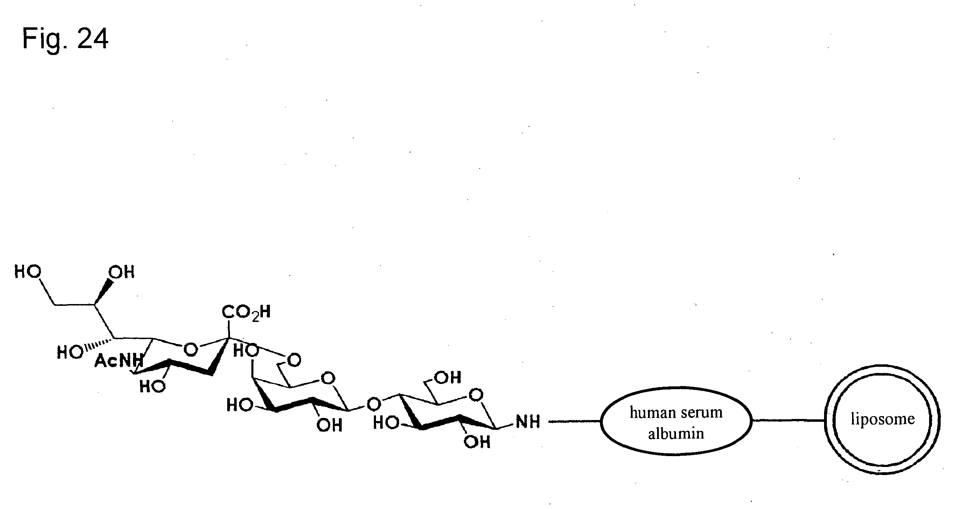

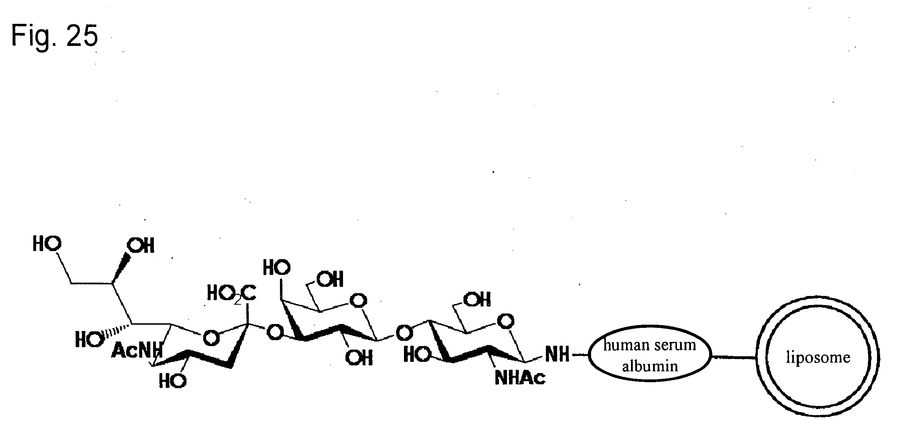

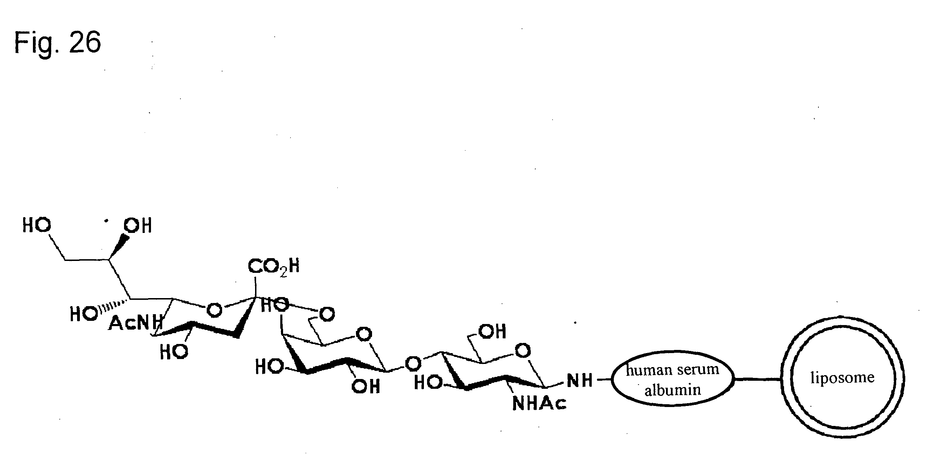

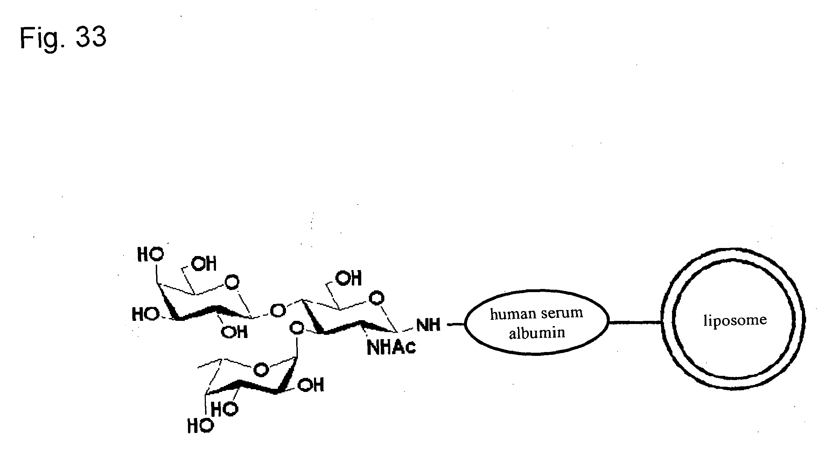

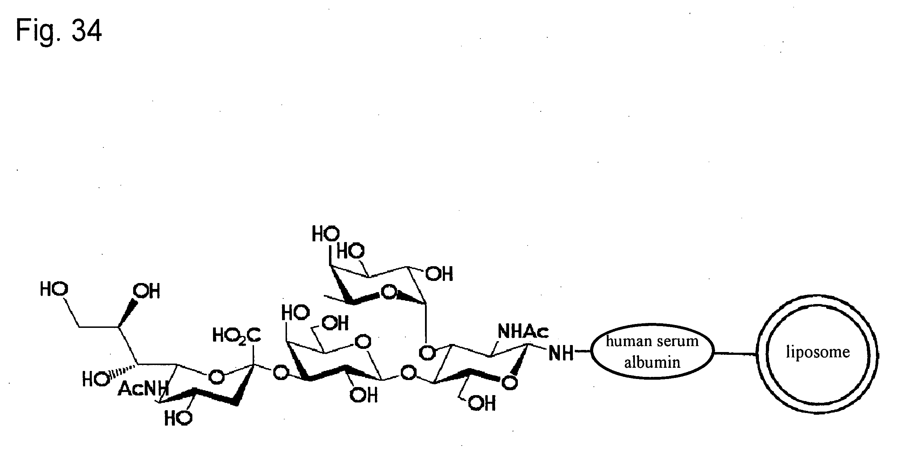

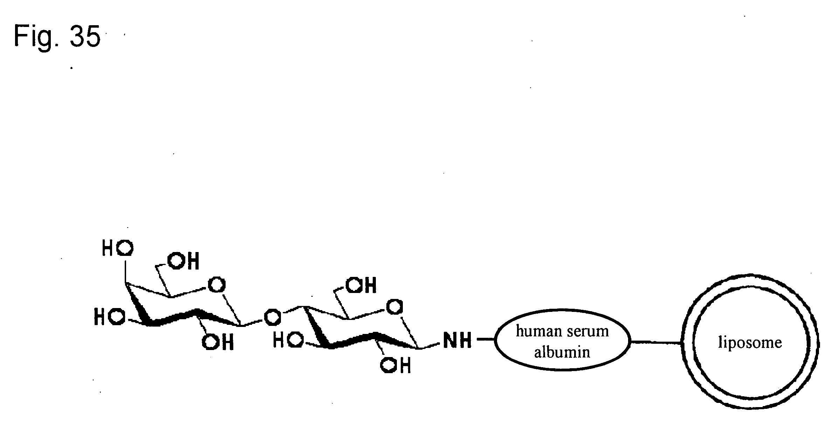

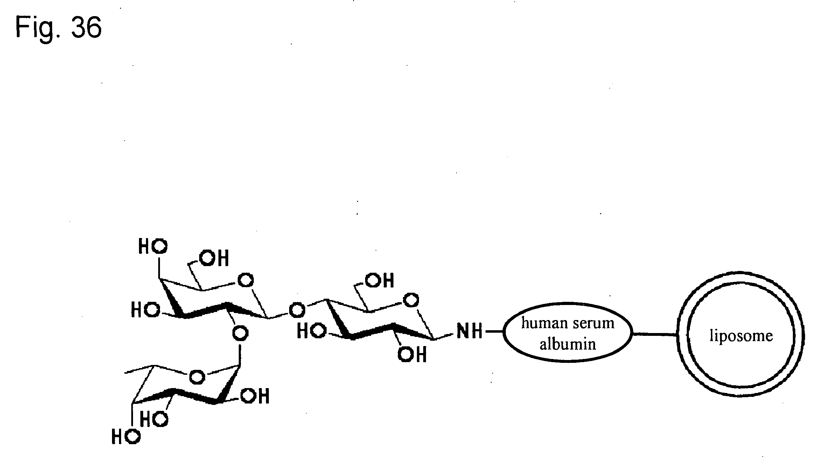

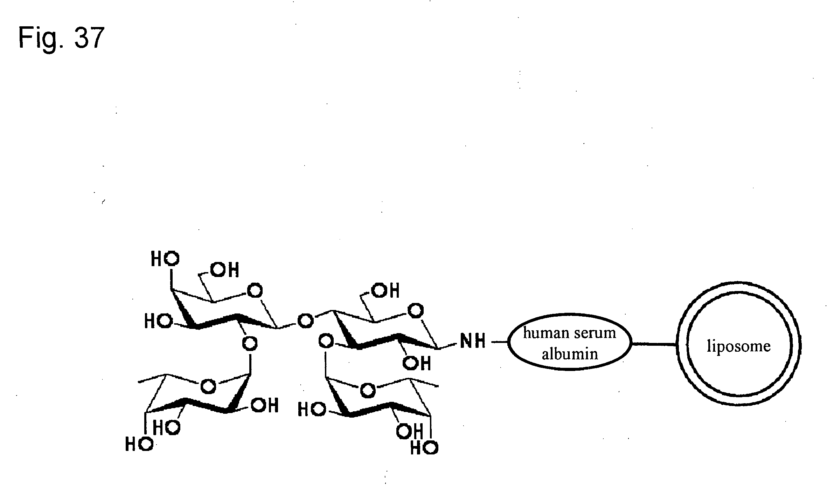

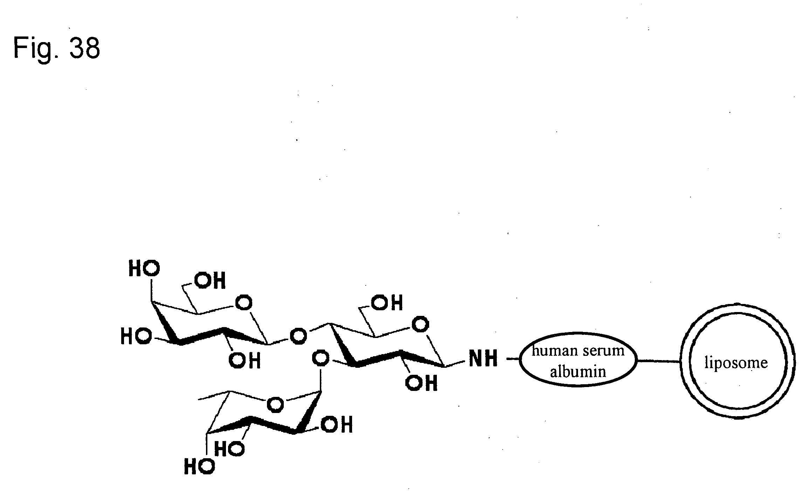

Examples of the intestinal-absorption controlled liposome include 3'-sialyllactose trisaccharide (whose structure is shown in Figure 23. (hereinafter, described in the same manner)), 6'-sialyllactose trisaccharide (Figure 24), 3'-sialyllactosamine sugar chain (Figure 25), and 6'-sialyllactosamine sugar chain (Figure 26). Examples of the targeting liposome include $\alpha$-1,2-mannobiose disaccharide (Figure 1), $\alpha$-1,3-mannobiose disaccharide (Figure 2), $\alpha$-1,4-mannobiose disaccharide (Figure 3), $\alpha$-1,6-mannobiose disaccharide (Figure 4), $\alpha$-1,3/$\alpha$-1,6-mannotriose trisaccharide (Figure 5), oligomannose-3 pentasaccharide (Figure 6), oligomannose-4b hexasaccharide (Figure 7), oligomannose-5 heptasaccharide (Figure 8), oligomannose-6 octasaccharide (Figure 9), oligomannose-7 nonasaccharide (Figure 10), oligomannose-8 decasaccharide (Figure 11), oligomannose-9 undecasaccharide (Figure 12), Lewis X trisaccharide (Figure 33), sialyl Lewis X tetrasaccharide (Figure 34), lactose disaccharide (Figure 35), 2'-fucosyllactose trisaccharide (Figure 36), difucosyllactose tetrasaccharide (Figure 37), and 3-fucosyllactose trisaccharide (Figure 38).

The present inventors have made an effort intensively with a view to improving membrane stability of the liposome to be used in the present invention, preventing leakage of a medicinal drug and a gene encapsulated in the liposome, hydrophilizing the membrane surface of the liposome, binding a protein with various densities, and binding a sugar chain with various densities. As a result, they prepared liposomes constituted of various lipids and glycolipids as mentioned below. Examples of the lipids constituting the liposome of the present invention include phosphatidylcholines, phosphatidylethanolamines, phosphatidic acids, long-chain alkyl phosphates, dicetyl phosphate, gangliosides, glycolipids, phosphatidylglycerols, sphingomyelins, and cholesterols. Examples of the phosphatidylcholines preferably include dimyristoylphosphatidylcholine, dipalmitoylphosphatidylcholine, and distearoylphosphatidylcholine. Examples of phosphatidylethanolamines preferably include dimyristoylphosphatidyl ethanolamine, dipalmitoylphosphatidylethanolamine, and distearoyl phosphatidylethanolamine. Examples of the phosphatidic acids or long-chain alkyl phosphates preferably include dimyristoylphosphatidic acid, dipalmitoylphosphatidic acid, distearoylphosphatidic acid, and dicetylphosphoric acid. Examples of the gangliosides preferably include ganglioside GM1, ganglioside GD1a, and ganglioside GT1b. Examples of glycolipids preferably include galactosylceramide, glycosylceramide, lactosylceramide, phosphatide, and globoside. Examples of the phosphatidylglycerols preferably include dimyristoylphosphatidylglycerol, dipalmitoylphosphatidylglycerol, and distearoylphosphatidylglycerol. Of them, a phosphatidic acid, a long-chain alkyl phosphate, ganglioside, glycolipid, or cholesterol is desirably added as a constitutional lipid since they have an effect of increasing stability of a liposome.

Examples of the lipid constituting the liposome of the present invention include phosphatidylcholines (0 to 70% by mole), phosphatidylethanolamines (0 to 30% by mole); not less than one lipid (0 to 30% by mole) selected from the group consisting of phosphatidic acids, long-chain alkyl phosphates and dicetyl phosphates; not less than one lipid (0 to 40% by mole) selected from the group consisting of gangliosides, glycolipids, phosphatidylglycerols, and sphingomyelins; and cholesterols (0 to 70% by mole). The liposome itself can be produced in accordance with a customary method. Examples of such a customary method include a thin-film method, reverse layer vaporization method, ethanol injection method, and dehydration-rehydration method.

Furthermore, the particle diameter of a liposome may be controlled by an ultrasonic irradiation method, extrusion method, French press method, or homogenization method. A method of producing the liposome of the present invention will be more specifically described. First, a mixed micelle is prepared by a lipid containing components such as a phosphatidylchlorin, cholesterol, a phosphatidylethanolamine, phosphatidic acid, ganglioside, glycolipid or phosphatidylglycerol, and sodium cholate serving as a surfactant.

Of them, addition of a phosphatidic acid or a long-chain alkyl phosphate such as dicetylphosphate is essential to negatively charge a liposome. Addition of a phosphatidylethanolamine is essential for a hydrophilic reaction site.

Addition of a ganglioside, glycolipid or phosphatidylglycerol is essential for a binding site of a linker protein. At least one lipid selected from the group consisting of gangliosides, glycolipids, phosphatidylglycerols, sphingomyelins and cholesterols assembles in a liposome and functions as a scaffold (raft) for biding a linker protein thereto. The liposome of the present invention is further stabilized by forming such a raft to which a protein is to be bonded. In other words, the present invention includes a liposome in which a raft is formed of at least one lipid selected from the group consisting of gangliosides, glycolipids, phosphatidylglycerols, sphingomyelins and cholesterols to bind a linker protein thereto.

The mixed micelle thus obtained is subjected to ultrafiltration to prepare a liposome.

As the liposome to be used in the present invention, a general liposome may be used. However, the surface of the liposome is desirably hydrophilized. After a liposome is prepared as described above, the surface of the liposome is hydrophilized. The hydrophilization of the liposome surface is performed by binding a hydrophilic compound thereto As a compound that may be used in hydrophilization, use may be made of a low molecular weight hydrophilic compound, preferably a low molecular weight hydrophilic compound having at least one OH group, more preferably, a low molecular weight hydrophilic compound having at least two OH groups. Furthermore, mention may be made of a low molecular weight hydrophilic compound having at least one amino group, in other words, a hydrophilic compound having at least one OH group and at least one amino group. Since a hydrophilic compound has a low molecular weight, it rarely causes steric hindrance to a sugar chain and thus does not prevent proceeding of a sugar-chain molecular recognition reaction by a lectin on the surface of a target cell membrane. Furthermore, examples of the hydrophilic compound do not include a sugar chain capable of binding a lectin used for targeting to a specific target such as a lectin in the liposome modified by a sugar chain of the present invention. As an examples of such a hydrophilic compound, use may be made of amino alcohols such as tris(hydroxyalkyl)aminoalkane including tris(hydroxymethyl) aminomethane, more specifically, tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxylmethyl)aminopropane, tris(hydroxyethyl)aminopropane, and tris(hydroxypropyl)aminopropane. Furthermore, a low molecular weight compound having an OH group to which an amino group is introduced may be used as a hydrophilic compound according to the present invention. Such a compound is not limited and, for example, cellobiose may be mentioned which has an amino group introduced into a sugar chain to which a lectin is not bonded. The surface of a liposome is hydrophilized by applying a divalent crosslinking agent and tris(hydroxymethyl)aminomethane onto a lipid, phosphatidylethanolamine of a liposome membrane. A hydrophilic compound is represented by the following general formulas (1), (2) and (3).

$$X-R1(R2OH)n \qquad \text{Formula (1)}$$

$$H_2N-R3-(R4OH)n \qquad \text{Formula (2)}$$

$$H_2N-R5(OH)n \qquad \text{Formula (3)}$$

In the formula, R1, R3 and R5 represent a linear or branched hydrocarbon chain of C1 to C40, preferably C1 to C20, further preferably C1 to C10; and R2 and R4 are absent or represent a linear or branched hydrocarbon chain of C1 to C40, preferably C1 to C20, further preferably C1 to C10. X represents a reactive functional group directly binding to a liposome lipid or a divalent crosslinking agent. Examples of such a functional group include COOH, NH, $NH_2$, CHO, SH, NHS-ester, maleimide, imidoester, active halogen, EDC, pyridyldisulfide, azidephenyl, and hydrazide. A reference symbol n represents a natural number.

The surface of a liposome hydrophilized by such a hydrophilic compound is covered with a thin layer of the hydrophilic compound. However, since the thickness of the cover layer of the hydrophilic compound is low, even in the case of a liposome having a sugar chain bonded thereto, the reactivity of the sugar chain is not suppressed.

The hydrophilization of a liposome may be performed by a method (JP Patent Publication (Kokai) No. 2000-302685) of producing a liposome by using a phospholipid, which is prepared by covalently bonding polyethylene glycol, polyvinyl alcohol and an anhydrous maleic acid copolymer.

Of them, hydrophilization of a liposome surface is particularly preferably performed by use of tris(hydroxymethyl) aminomethane.

The hydrophilization method using tris(hydroxymethyl)aminomethane of the present invention is preferable compared to a conventional method using polyethylene glycol in several points. For example, in the case of the present invention where targeting is performed by use of a molecular recognition function of a sugar chain which is bonded onto a liposome, tris(hydroxymethyl)aminomethane is particularly preferably used, since tris(hydroxymethyl)aminomethane is a low molecular weight substance compared to a conventionally used high molecular weight substance such as polyethylene glycol, it rarely causes steric hindrance to the sugar chain, and does not prevent proceeding of a sugar-chain molecular recognition reaction performed by a lectin (sugar chain recognizing protein) on the membrane surface of a target cell.

Furthermore, the liposome of the present invention has a satisfactory size distribution, composition, and dis-

person properties after completion of the hydrophilization treatment, and excellent long-term storage stability and *in-vivo* stability. Therefore, the liposome of the present invention is preferably used for producing a preparation.

To hydrophilize a liposome surface by tris(hydroxymethyl)aminomethane, first a divalent reagent such as bissulfosuccinimidylsuberate, disuccinimidyl glutarate, dithiobissuccinimidylpropionate, disuccinimidylsuberate, 3,3'-dithiobissurfosuccinimidylpropionate, ethylene glycol bissuccinimidylsuccinate, or ethylene glycol bissulfosuccinimidylsuccinate, is added to a liposome solution, which is obtained in accordance with a customary method using a lipid such as dimyristoylphosphatidylethanolamine, dipalmitoylphosphatidyl ethanolamine, or distearoylphosphatidylethanolamine to react them, thereby bonding the divalent regent to the lipid such as dipalmitoylphosphatidyl ethanolamine on the liposome membrane; and then tris(hydroxymethyl)aminomethane is reacted with one of the bonds of the divalent reagent, thereby bonding tris(hydroxymethyl)aminomethane to the liposome surface.

The liposome hydrophilized in this manner is extremely stable *in vivo.* Since it has a long *in vivo* half-life, even if a targeting sugar chain having targetability is not bonded as described later, the liposome can be preferably used as a drug carrier in the drug delivery system. The present invention includes a liposome hydrophilized with a low molecular weight compound in its surface.

The present invention includes a liposome hydrophilized with a hydrophilic compound as mentioned above and having no sugar chain bonded thereto. Such a hydrophilized liposome is advantageous since the stability of the liposome itself is high and the recognition of a sugar chain by the liposome is enhanced when a sugar chain is bonded to it.

The liposome of the present invention has a constitutional lipid containing phosphatidylcholines (0 to 70% by mole), phosphatidylethanolamines (0 to 30% by mole); not less than one lipid (0 to 30% by mole) selected from the group consisting of phosphatidic acids, long-chain alkyl phosphates and dicetyl phosphates; not less than one lipid (0 to 40% by mole) selected from the group consisting of gangliosides, glycolipids, phosphatidylglycerols, and sphingomyelins; and cholesterols (0 to 70% by mole).

The present invention includes a method of hydrophilizing a liposome by bonding a hydrophilic compound as mentioned above to a liposome. Furthermore, a hydrophilized liposome having no sugar chain bonded thereto is included. A targeting liposome or an intestinal tract absorbable liposome according to the present invention can be produced by bonding a sugar chain to the liposome having no sugar bonded thereto.

In the present invention, any one of the sugar chains mentioned above may be directly bonded to the liposome prepared in the aforementioned manner and further bonded to the liposome via a linker protein. In this case, the type of a sugar chain to be bonded to a liposome is not limited to a single type. A plurality of types of sugar chains may be bonded. The plurality of types of sugar chains may bind to different lectins commonly present on the cellular surface of the same tissue or organ or different lectins present on the cellular surface of different tissues and organs. If the former case of sugar chain is selected, the liposome can be surely directed to a predetermined target tissue or organ. If the latter case of sugar chain is selected, a single type of liposome can be directed to a plurality of targets. In this way, multi-purpose targeting liposome can be obtained.

To bind a sugar chain to a liposome, a linker protein and/or the sugar chain are mixed in producing the liposome, thereby bonding the sugar chain on the surface of the liposome while producing the liposome. Alternatively, it is rather desirable to separately prepare a liposome, linker protein and sugar chain(s) and then bind the linker protein and/or the sugar chain to the liposome after completion of liposome production. This is because the binding density of the sugar chains can be controlled by bonding a linker protein and/or a sugar chain to the liposome.

Direct binding of a sugar chain to a liposome can be performed by the method described below.

A liposome is produced by mixing a sugar chain in the form of a glycolipid or by binding a sugar chain to a phospholipid of a liposome after completion thereof while controlling the density thereof.

When a sugar chain is bonded by use of a linker protein, it is preferable to use a protein derived from the living body, in particular, a protein derived from a human. The protein derived from the living body is not particularly limited and includes a protein present in blood such as albumin, and a physiologically active substance present *in vivo,* such as animal serum albumin including human serum albumin (HSA) and bovine serum albumin (BSA). Particularly when human serum albumin is used, it has been experimentally confirmed to show a large uptake by mice tissues.

The liposome of the present invention is extremely stable and post treatment can be easily applied to the liposome. More specifically, a protein, a linker protein and a sugar chain can be easily bonded to the liposome after completion of the production thereof. Accordingly, a large amount of liposomes are produced, and then, various types of liposomes can be desirably produced by bonding different types of proteins, a linker protein(s) and a sugar chain(s) to the liposomes in accordance with the purposes.

To the liposome of the present invention, a sugar chain is bonded via a linker protein or directly to a lipid constituting the liposome. The liposome of the present invention has a conjugated polysaccharide ligand such as a glycolipid and glycoprotein and hydrophilized with a low molecular weight compound.

When a targeting liposome according to the present invention is used as a pharmaceutical drug as is described later, it must contain a compound having a medicinal effect. The compound having a medicinal effect is encapsulated in the liposome or bonded to the surface of the liposome. Alternatively, a protein having a medicinal effect may be used as a linker protein. In this case, such a protein may serve not only as a linker protein for bonding a liposome and a sugar chain but also the protein having a medicinal effect. As the protein having a medicinal effect, physiologically active proteins may be mentioned.

A sugar chain may be bonded to a liposome via a linker protein by the method mentioned below.

First, a protein is bonded to the surface of a liposome. The liposome is treated with an oxidizing agent such as $NaIO_4$, $Pb(O_2CCH_3)_4$, or $NaBiO_3$ to oxidize a ganglioside present on the membrane surface of the liposome. Then, the linker protein is bonded to the ganglioside on the liposome surface by a reductive amination using a reagent such as $NaBH_3CN$ or $NaBH_4$. It is preferable that the linker protein is also hydrophilized by bonding a compound having a hydroxyl group to the linker protein. More specifically, a compound such as tris(hydroxymethyl)aminomethane for use in hydrophilization mentioned above may be bonded to a linker protein on a liposome by use of a divalent reagent such as bissulfosuccinimidylsuberate, disuccinimidylglutarate, dithiobissuccinimidylpropionate, disuccinimidylsuberate, 3,3'-dithiobissulfosuccinimidylpropionate, ethylene glycol bissuccinimidylsuccinate, or ethylene glycol bissulfosuccinimidylsuccinate.

To describe this more specifically, one of the ends of a divalent crosslinking reagent is bonded to all amino groups of a linker protein. Then, the reduced terminals of sugar chains are glycosylaminated to prepare a sugar chain glycosylamine compound. The amino groups of the sugar chains are bonded to the other unreacted terminals of part of the divalent crosslinking reagent bonded onto the liposome.

The covalent bond formed between a sugar chain and/or a hydrophilic compound and a liposome, or the covalent bond formed between a sugar chain and/or a hydrophilic compound and a linker protein can be cleaved when the liposome is taken in a cell. More specifically, when a linker protein and a sugar chain are covalently bonded via a disulfide bond, the disulfide bond is intracellularly reduced to cleave the sugar chain. When the sugar chain is cleaved, the surface of the liposome becomes hydrophobic. As a result, the liposome bonds to the biomembrane, causing disturbance of the membrane stability, and releases a medicinal drug contained therein.

Subsequently, using the unreacted terminals (constituting major part) of the divalent reagent remaining intact on the surface of the protein on the sugar-chain bonded liposome membrane, hydrophilization treatment is performed. More specifically, the unreacted terminals of the divalent reagent bonded to the protein on the liposome are reactively bonded with a hydrophilic compound such as tris(hydroxymethyl)aminomethane, thereby hydrophilizing the entire surface of the liposome.

The hydrophilization of the surface of a liposome and a linker protein improves delivery of the liposome to various types of tissues and blood retention and delivery of the liposome to various types of tissues for the reasons below. It is conceivable that the hydrophilization of the surfaces of the liposome and the linker protein causes tissues to recognize the portion except for the sugar chain as an *in-vivo* moisture content, with the result that other tissues except for a target tissue fail to recognize the liposome and only the sugar chain is recognized by a lectin (sugar chain recognition protein) of the target tissue.

Subsequently, the sugar chain is bonded to a linker protein on the liposome. This is performed by glycosylaminating the reduced terminal of a saccharide constituting the sugar chain by an ammonium salt such as $NH_4HCO_3$ or $NH_2COONH_4$, and then, bonding the linker protein bonded onto liposome membrane to the saccharide glycosylaminated in the above by use of a divalent reagent such as bissulfosuccinimidylsuberate, disuccinimidylglutarate, dithiobissuccinimidylpropionate, disuccinimidylsuberate, 3,3'-dithiobissulfosuccinimidylpropionate, ethylene glycol bissuccinimidylsuccinate, or ethylene glycol bissulfosuccinimidylsuccinate. As a result, the liposomes shown in Figures 1 to 12, 23 to 26, 33 to 38 can be obtained. These sugar chains are commercially available.

The particle size of the liposome of the present invention and the liposome having a sugar chain, etc., bonded thereto falls within the range of 30 to 500 nm, and preferably 50 to 350 nm. The liposome of the present invention is desirably charged negatively. The liposome, if negatively charged, can prevent interaction with other negatively charged cells *in vivo.* The zeta potential of the surface of the liposome of the present invention is -50 to 10 mV, preferably -40 to 0 mV, and more preferably -30 to -10 mV at 37°C as measured in a physiological saline.

When a sugar chain is bonded, the binding density of the sugar chain falls within the range 1 to 60 chains per molecule of a linker protein, preferably 1 to 40, and further preferably 1 to 20. When a linker protein is used, the binding density falls within the range of 1 to 30000 per liposome particle, preferably 1 to 20000, and further preferably, 1 to 10000; 100 to 30000, preferably 100 to 20000, and further preferably 100 to 10000; or 500 to 30000, preferably 500 to 20000, and further preferably 500 to 10000. When no linker protein is used, the number of sugar chains that may be bonded is 1 to 500000 per liposome particle, preferably 1 to 300000, and further preferably, 1 to 100000 or more, in maximum.

In the present invention, the targetability to a target cell and tissue can be controlled by selecting the structure and amount of sugar chain to be bonded in various manners.

The controlled intestinal absorption can be increased by selecting the type and amount of sugar chain to be bonded. More specifically, by binding not only a sugar chain increasing the controlled intestinal absorption but also a sugar chain capable of targeting to a predetermined tissue or organ to a liposome, a liposome having two characteristics: targetability to a predetermined tissue or organ and controlled intestinal absorption can be prepared.

As described above, the lectin to which the targeting liposome of the present invention is specifically bonded is determined based on the type and amount of sugar chain to be bonded. In this mechanism, the liposome of the present invention reaches the predetermined tissue or organ. Alternatively, by selecting the structure and amount of sugar chain to be bonded, the liposome can be delivered to a lesion site such as a cancer tissue.

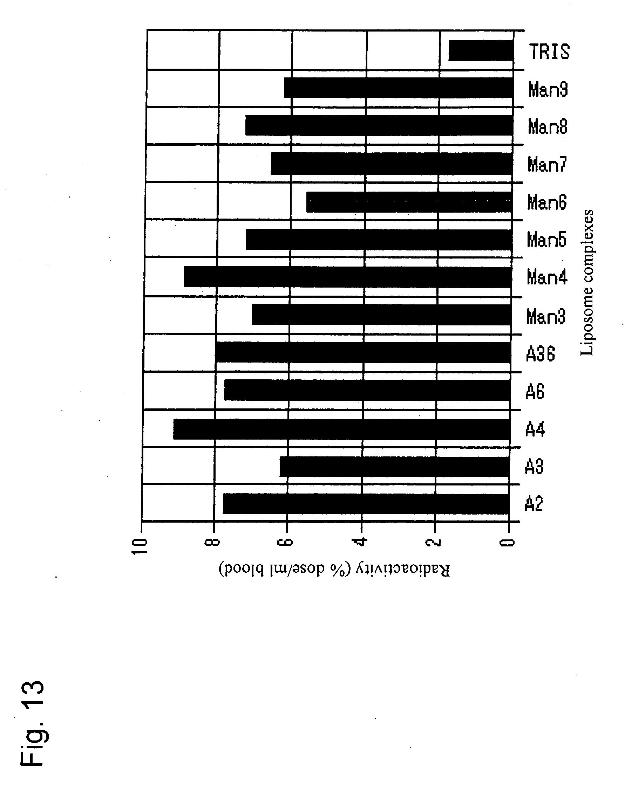

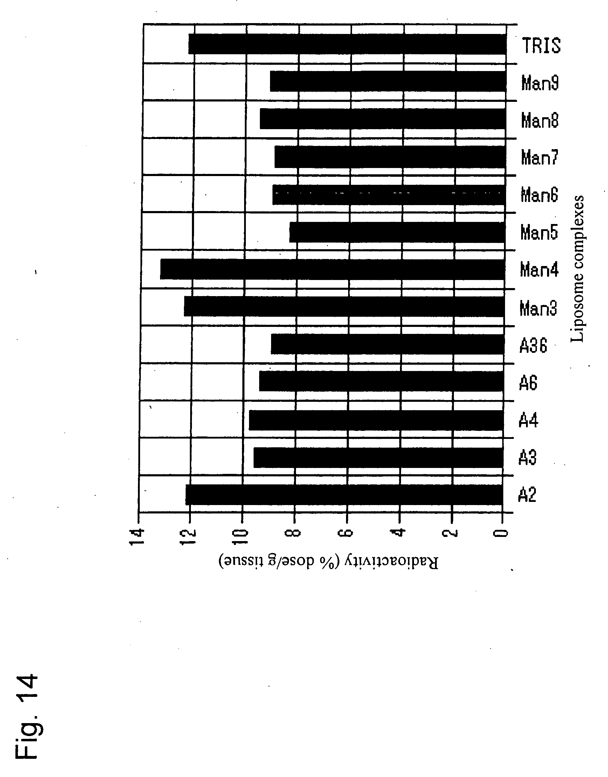

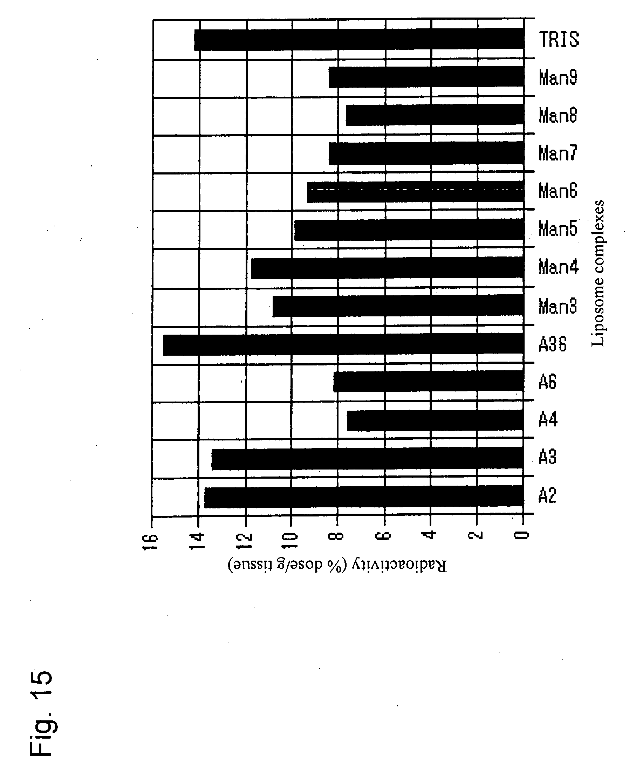

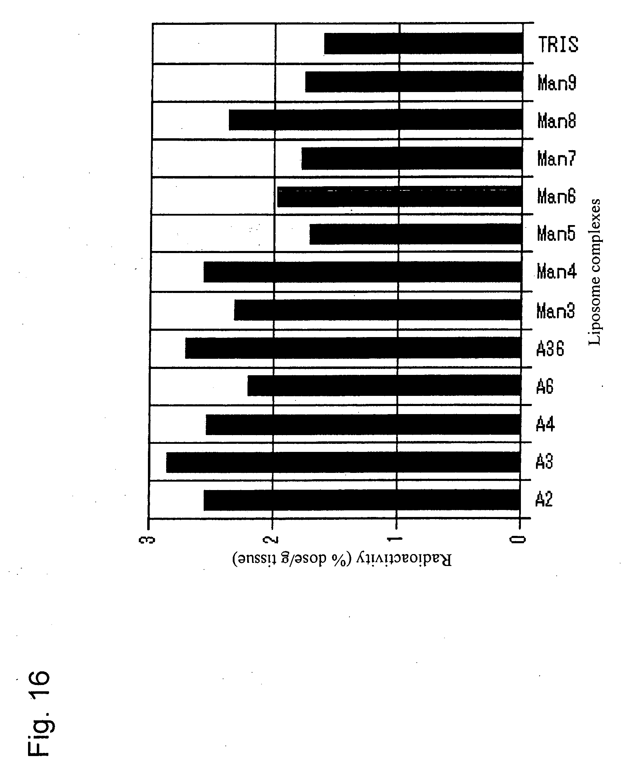

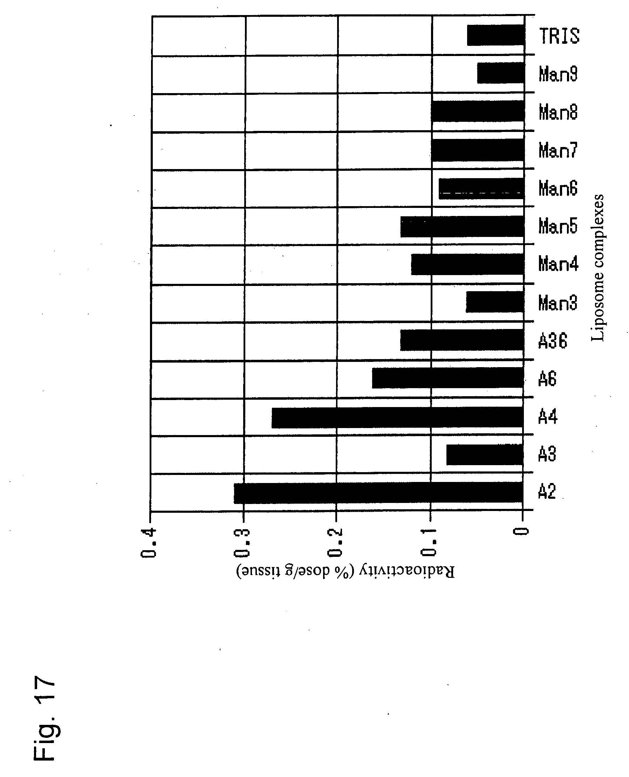

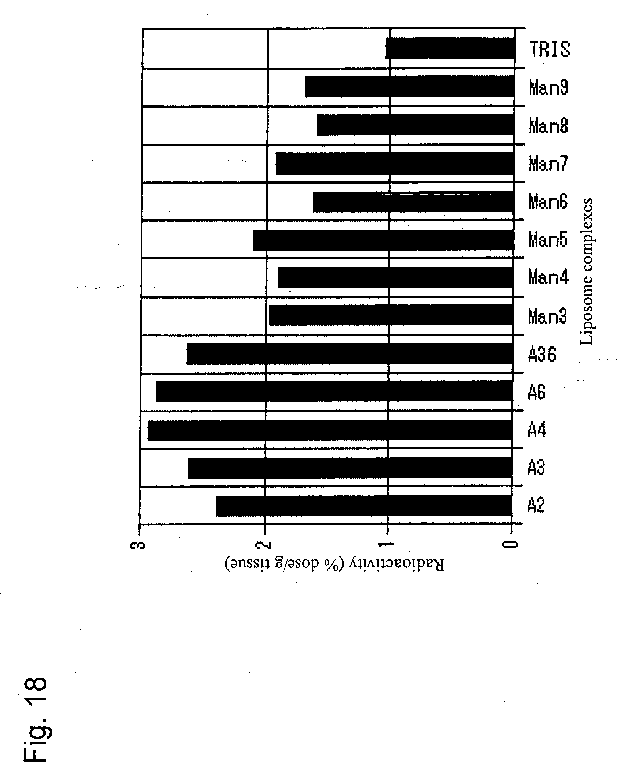

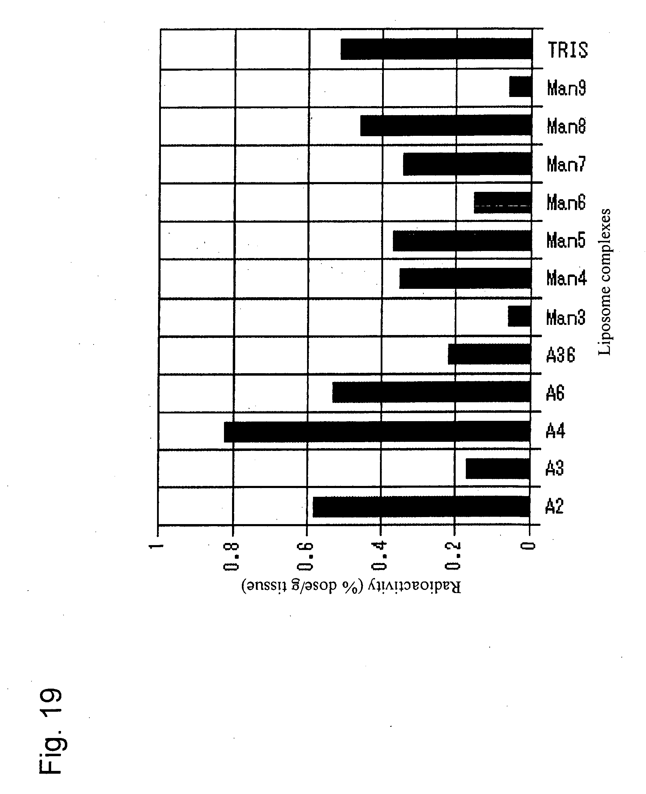

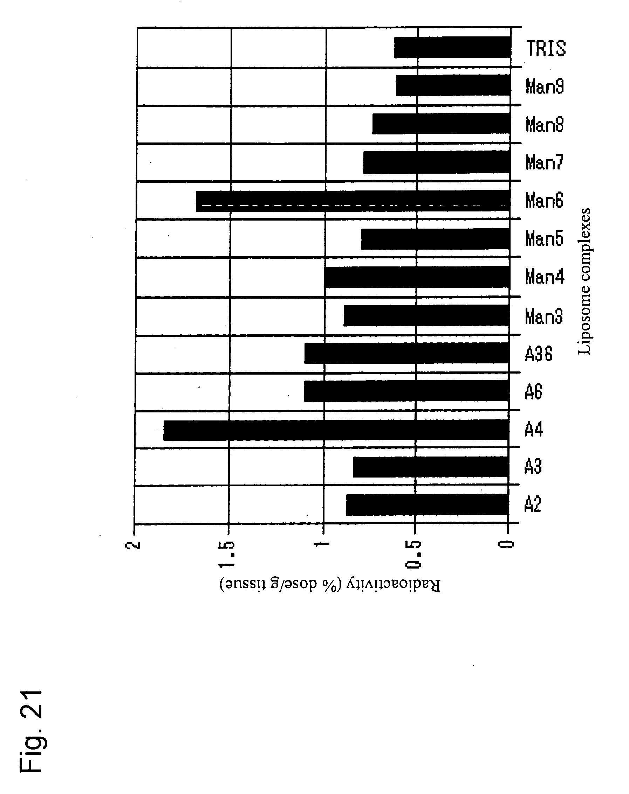

In the present invention, the targetability of a liposome to a target cell and tissue can be controlled by selecting the structure and amount of the sugar chain to be bonded. Depending upon a sugar chain, the liposome of the present invention is directed to tissues or organs such as blood, liver, spleen, lung, brain, small intestinal tract, heart, thymus gland, kidney, pancreas, muscle, large intestinal tract, bone, bone marrow, cancer tissue, inflammatory tissue and lymph node. For example, as shown in Figures 13, 16, 17, 18, 21 and 22 (showing Examples), liposomes having $\alpha$-1,2-mannobiose disaccharide, $\alpha$-1,3-mannobiose disaccharide, $\alpha$-1,4-mannobiose disaccharide, $\alpha$-1,6-mannobiose disaccharide, $\alpha$-1,3/$\alpha$-1,6-mannotriose trisaccharide, oligomannose-3 pentasaccharide, oligomannose-4b hexasaccharide, oligomannose-5 heptasaccharide, oligomannose-6 octasaccharide, oligomannose-7 nonasaccharide, oligomannose-8 decasaccharide, and oligomannose-9 undecasaccharide bonded thereto all have high targetability to blood, lung, brain, cancer tissue, heart, and small intestinal tract. Furthermore, as shown in Figure 14, liposomes having $\alpha$-1,2-mannobiose disaccharide, oligomannose-3 pentasaccharide, and oligomannose-4b hexasaccharide bonded thereto have high targetability to the liver. Furthermore, as shown in Figure 15, liposomes having $\alpha$-1,2-mannobiose disaccharide, $\alpha$-1,3-mannobiose disaccharide, and $\alpha$-1,3/$\alpha$-1,6-mannotriose trisaccharide bonded thereto have high targetability to the spleen. Moreover, as shown in Figure 19, liposomes having $\alpha$-1,2-mannobiose disaccharide, $\alpha$-1,4-mannobiose disaccharide, and $\alpha$-1,6-mannobiose disaccharide bonded thereto have high targetability to the lymph node. In addition, a liposome having oligomannose-6 octasaccharide bonded thereto has a high targetability to the thymus gland.

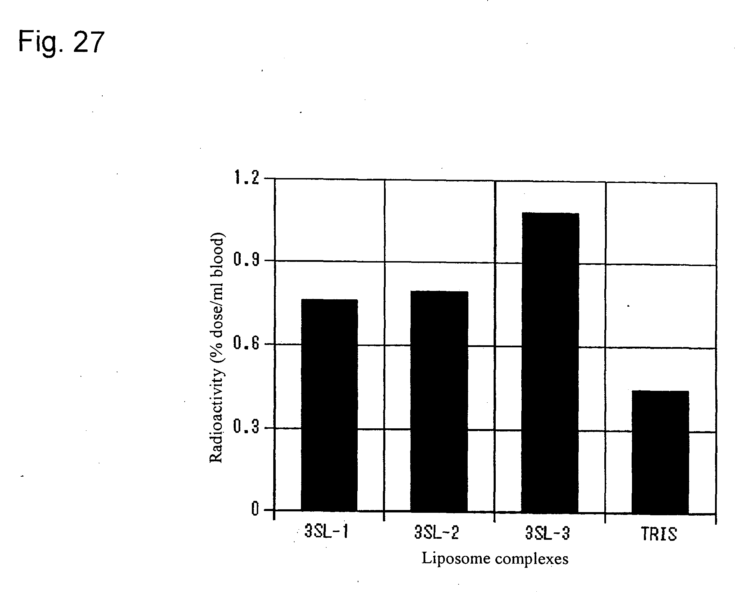

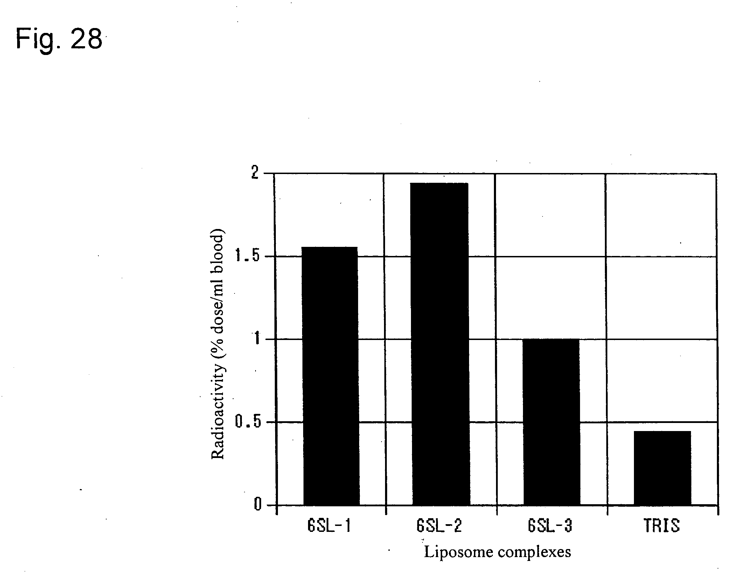

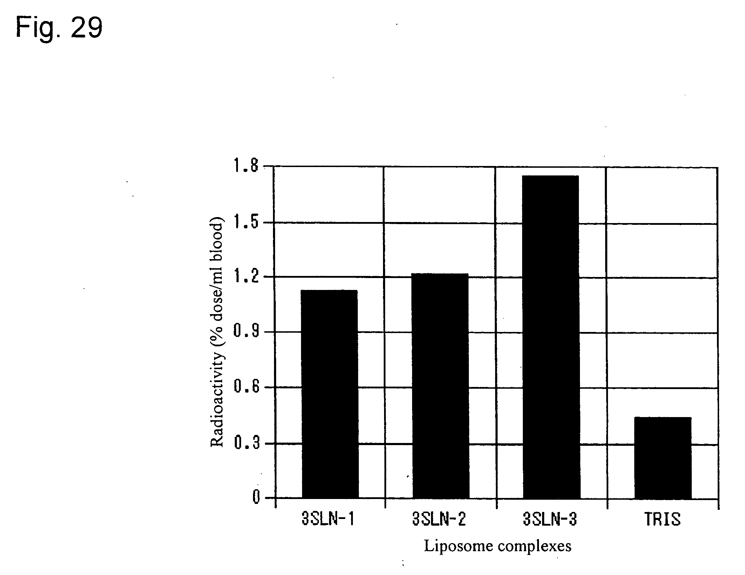

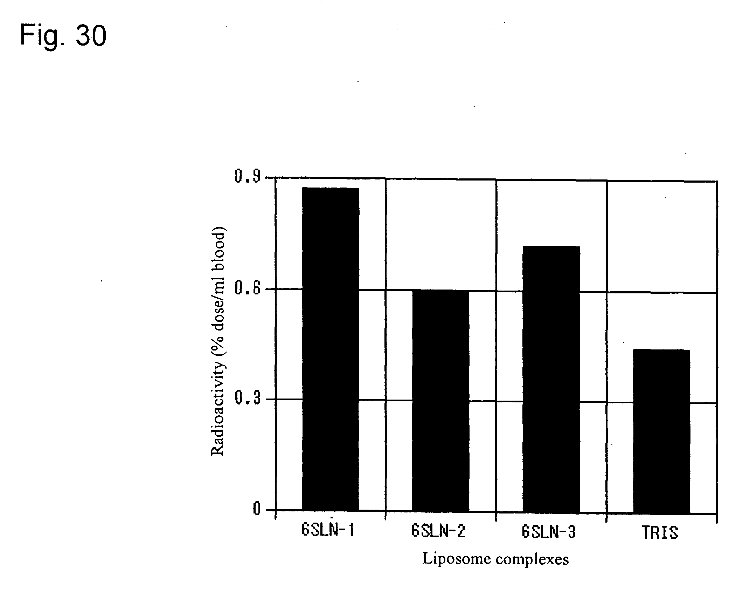

The liposomes shown in Figures 23 to 26 of the invention generally show extremely high intestinal absorption. When the density of a sugar chain bonded to each of the liposomes is controlled, the intestinal absorption can be controlled and a medicinal drug can be more efficiently delivered to a target site, and a side effect of the medicinal drug can be reduced. For example, Figures 27 to 30 (showing Examples) show the deliver rate (intestinal absorption) of the liposome from the intestinal tract into blood in the cases where the amount of sugar chain bonded to each of 4 types of liposome modified by a sugar chains is varied in three levels.

Note that the amount of sugar chain to be bonded to a linker protein bonded liposome is changed by bonding the sugar chains of three levels (1) 50 $\mu$g, 2) 200 $\mu$g and 3) 1 mg). According to the results of Examples, in the cases of sugar chains of 6' sialyllactose trisaccharide and 6'-sialyllactosamine, as the sugar-chain density increases, the intestinal absorption gradually decreases. Conversely, in the cases of sugar chains of 3'-sialyllactose trisaccharide and 3'-sialyllactosamine, the intestinal absorption increases. These facts demonstrate that the intestinal absorption varies depending upon the amount of sugar chain bonded onto a liposome for each type of sugar chain. Therefore, the intestinal absorption can be controlled by appropriately setting the amount of sugar chain bonded onto a liposome for each type of sugar chain.

When a compound having a medicinal effect is encapsulated in a liposome according to the present invention, the liposome is delivered to a predetermined tissue or organ, taken into the cells of the tissue and organ, releases the compound having a medicinal effect and exert the medical effect. In the case of a liposome having a sugar chain bonded thereto, the liposome reaches a predetermined tissue or organ depending upon the targetability of the sugar chain. Furthermore, even in the case of a liposome having no sugar chain bonded thereto, the liposome can reach a predetermined tissue and organ, since the liposome is stable *in vivo,* and therefore, the half-life of the liposome *in-vivo* is long.

The compound having a medicinal effect is not particularly limited and a wide variety of known proteins and pharmaceutical compounds can be used. When a medicinal compound for treating a predetermined disease such as an anticancer drug is contained in the liposome of the present invention, the liposome can be used as a therapeutic drug for the predetermined disease. As a compound having a medicinal effect to be encapsulated in the liposome of the present invention, mention may be made of gene therapeutic drugs such as DNA, RNA, and siRNA.

Examples of medicinal compounds to be encapsulated in the liposome of the present invention include medicinal drugs for tumors such as an anticancer based on an alkylating compound, metabolic antagonist, plant derived anticancer drug, anticancer antibiotic, BRM, cytokine, anticancer drug based on a platinum complex, immunotherapeutic drug, hormonal anticancer drug, and monoclonal antibody; medicinal drugs for the central nerve; medicinal drugs for the peripheral nervous system/sensory organ; therapeutic drugs for a respiratory disease; medicinal drugs for a circulatory organ; medicinal drugs for a digestive organ; hormonal medicinal drugs; medicinal drugs for a urinary/genital organ; medicinal drugs for external application; vitamins/analeptics; medicinal drugs for blood and body fluid; metabolic medicine; antibiotic/chemotherapeutic agents; medicinal drugs for medical check; anti-inflammatory agents; medicinal drugs for eye disorder; medicinal drugs for the central nervous system; medicinal drugs for the autoimmune system; medicinal drugs for the circulatory organ; medicinal drugs for lifestyle-related diseases such as diabetes and hyperlipemia; various oral, pulmonarg, transdermal or transmucosal drugs; adenocortical hormones; immunosuppressive agents; antibiotics; anti-viral agents; neovascularization inhibitors; cytokines; chemokines; anti-cytokine antibodies; anti-chemokine antibodies; anti-cytokine/chemokine receptor antibodies; nucleic acid preparations involved in gene therapy such as siRNA, miRNA, smRNA, antisense ODN and DNA; neuroprotective factors; and various antibody medicines.

Examples of the medicinal drugs for tumors include alkylating agent such as nitrogen mustard-N-oxide hydrochloride, cyclofosfamide, ifosfamide, brusfan, nimustine hydrochloride, mitobronitol, melphalan, dacarbazine, ranimustine, and estramustine sodium phosphate, metabolic antagonists such as mercaptopurine, thioinosine (mercaptopurineriboside), methotrexate, enocitabine, cytarabine, ancitabine hydrochloride (cyclocytidine hydrochloride), fluorouracil, 5-FU, tegafur, doxifluridine, and carmofur; plant-derived anticancer drugs such as alkaloids including etoposide, vinblastine sulfate, vincristine sulfate, vindesine sulfate, paclitaxel, taxol, irinotecan hydrochloride, and nogitecan hydrochloride; anticancer antibiotics such as actinomycin D, mitomycin C, chromomycin A3, bleomycin hydrochloride, bleomycin sulfate, peplomycin sulfate, daunorubicin hydrochloride, doxorubicin hydrochloride, aclarubicin hydrochloride (acrasinomycin A), pirarubicin hydrochloride, epirubicin hydrochloride, and neocarcinostatin, and others including mitoxantrone hydrochloride, carboplatin, cisplatin, L-asparaginase, aceglatone, procarbazine hydrochloride, tamoxifen citrate, ubenimex, lenthinan, sizofiran, medroxyprogesterone acetate, fosfestrol, mepitiostane, and epitiostanol. The present invention may include derivatives of the aforementioned medicinal drugs.

The liposome of the present invention containing a medicinal drug as mentioned above can be used in therapy for diseases such as cancer and inflammation. The term "cancer" used herein includes all types of neoplastic diseases such as tumors and leukemia. When a liposome modified by a sugar chain according to the present invention containing such a medicinal drug is administered, the medicinal drug is accumulated on the carcinogenic and inflammatory sites, compared to the case where the medicinal drug is solely administered. More specifically, it is accumulated twice, preferably 5 times or more, further preferably 10 times or more and particularly preferably 50 times or more as large as the amount of single administration.

Note that a compound having a medicinal effect may be encapsulated in a liposome or bonded onto the surface of the liposome. For example, a protein may be bonded to the surface of the liposome in the same manner as in binding a linker protein. Other compounds may be bonded by a known method with the help of a functional group contained therein. Such a compound is encapsulated in a liposome by the method below. More specifically, a medicinal drug may be encapsulated in a liposome by a known method, and for example, a liposome is formed using a solution containing a medicinal drug or the like and a lipid such as a phosphatidylcholine, phosphatidylethanolamine, phosphatidic acid, long-chain alkyl phosphate, ganglioside, glycolipid, or phosphatidylglycerols, and cholesterol, thereby encapsulating the medicinal drug or the like in the liposome.

Therefore, the liposome preparation of the present invention obtained by encapsulating a medicinal drug or a gene that may be subjected to therapy or diagnosis therein is selectively controlled so as to be delivered to a cancer tissue, inflammatory tissue or other tissues. Thus, it is possible to apply the medicinal drug or diagnostic drug intensively to a target cell or tissue, thereby increasing the efficacy of the drug. On the other hand, it is possible to reduce the amount of the drug taken into other cells or tissues, thereby mitigating a side effect.

The liposome or liposome modified by a sugar chain of the present invention may be administered as a pharmaceutical composition in various forms. Examples of such administration forms include instillation by an eyedrop; peroral administration by a tablet, capsule, granule, powder, and syrup; and parenteral administration by injection, drip infusion, and suppository. These compositions are produced by known methods and contain carriers, diluents and excipients generally used in the field of medicinal preparation. As carriers and excipients for tablets, use may be made of gelation agents, lactose, and magnesium stearate. Injection agents are prepared by dissolving, suspending or emulsifying the sugar chain bonded liposomes of the present invention in an aseptic aqueous or oily solution that is generally used in an injection agent. As the aqueous solution for injection, use may be made of physiological saline and isotonic solutions containing glucose and other auxiliary agents. In this case, an appropriate dissolution auxiliary agent(s), such as an alcohol, polyalcohol e.g., propylene glycol, and nonionic surfactant may be used simultaneously. As the oily solution, use may be made of sesame oil and soybean oil. As the dissolution auxiliary agent, use may be simultaneously made of benzyl benzoate and benzyl alcohol.

The administration route of a pharmaceutical composition of the present invention is not limited and includes instillation, peroral administration, intravenous injection and intramuscular injection. The administration amount can be appropriately determined depending upon significance of the disease. In the present invention, a pharmaceutically effective amount of composition is administered to a patient. The phrase "a pharmaceutically effective amount is administered" refers to administering a medicinal drug in an appropriate amount for treating a disease. The administration number of times of a pharmaceutical composition of the present invention is appropriately selected depending upon the symptom of a patient.

The liposome having a sugar chain on the surface is suitable for parenteral administration (intravenous injection) and peroral administration. The administration amount may be from one in tens to one in thousands relative to a conventional non-targeting liposome preparation. More specifically, the amount of medicinal drug to be contained in a liposome may be 0.0001 to 1000 mg, preferably 0.0001 to 10 mg, and further preferably, 0.0001 to 0.1 mg per body weight (kg). The liposome of the present invention includes a liposome not containing a targeting sugar chain but binding a hydrophilic compound thereto. Since such a liposome is hydrophilized, it has excellent *in-vivo* stability and a long half-life. Therefore, the liposome produces a sufficient effect in a low content.

When the pharmaceutical composition of the present invention is used for diagnosis, a labeling compound such as a fluorescent pigment, or radioactive compound is bonded to liposomes. When the liposomes tagged with a labeling compound are bonded to a lesion, the labeling compound is taken in lesion cells. Therefore, a disease can be detected and diagnosed based on the presence of the targeting compound as an index.

Furthermore, the liposome of the present invention having a cosmetic or a fragrance encapsulated therein or bonded thereto may be used as a cosmetic composition. The term "cosmetic" refers to a product "which is generally used by applying and rubbing, dispersing it or by analogous manner, in order to make a human body clean, beautiful, and more attractive, change facial appearance, and maintain the skin or hair healthy and which acts mildly to a human body". In the present invention, the term "cosmetic" includes not only general cosmetics but also quasi drugs defined as a product acting mildly, not used in therapy or prophylaxis of a disease, and not directed to having an effect on the structure and function of a body. Such a cosmetic may include those acting on cells such as the skin cells and activating the cells.

Examples of the cosmetics include those applied to the skin, hair, head hair, and head skin.

More specifically, a cosmetic may contain a skin whitener (whitening agent) such as magnesium phosphate-ascrobate, kojic acid, placenta extract, arbutin, and ellagic acid; vitamins such as vitamin A, vitamin B, vitamin C and vitamin E; hormones such as estrogen, estradiol, estrone, ethinylestradiol, cortisone, hydrocortisone, prednisone; skin tonic agents such as citric acid, tartaric acid, lactic acid, aluminium chloride, aluminium sulfate, potassium sulfate, alum, aluminium chlorohydroxyl allantorin, aluminium dihydroxy allantoin, zinc paraphenol sulfonate, zinc sulfate; hair-growth accelerating agents such as cantharis tincture, capsaicin tincture, ginger tincture, swertiae herba extract, garlic extract, hinokitiol, carpronium chloride, and pentadecanoic acid glyceride; others such as elastin, collagen, chamomile extract, glycyrrhiza extract, β-glycyrrhetic acid, glycyrrhizinic acid, γ-orizanol, calcium pantothenate, pantothenyl ethyl ether, and amino acids. Other than these, the cosmetic may contain compounds capable of activating cells physiologically active proteins such as interferons, interleukins, lysozyme, lactoferrin, and transferrin.

Besides these, use may be made of cosmetics described in #New Cosmetic Science", 2nd ed. Edited by Takeo Mitsui, published by Nanzando, January 18, 2002; and "Fragrance Science"-Theory and Reality-, 4th ed., written by Takeo Tamura, Hiroshi, Hirota, published by Fragrance Journal, June 30, 2001.

The composition containing a liposome according to the present invention having a cosmetic encapsulated therein or bonded thereto stays on the surface of the skin, where the cosmetic contained in the liposome is released and produces an effect on the skin surface. Alternatively, the liposome by itself is transdermally absorbed. When the liposome reaches the stratum corneum or the tissue under the stratum corneum, it is absorbed into the cells of the tissue, releases the cosmetic, which produces a medicinal effect.

A sugar chain may not be bonded to the liposome that is used in a cosmetic composition. In this case, the liposome stays on the skin or the underneath the skin and releases a cosmetic. Alternatively, a sugar chain may be bonded to the liposome so as to be accumulated specifically onto a target of an inflammatory site of the skin.

The cosmetic composition of the present invention may contain an aqueous composition and an oily composition usually contained as cosmetics in addition to the liposome described above. Examples of the aqueous composition include a moisturizing agent, thickening agent, and alcohol. Examples of the moisturizing agent include glycerin, propylene glycol, and polyhydric alcohol. Examples of the thickening agent include gum tragacanth, pectin, and alginic acid salt. Examples of the alcohol include, ethanol, and isopropanol. Examples of the oily component include olive oil, camellia oil, castor oil, solder, oleic acid, solid paraffin, ceresin, wax, Vaceline, liquid paraffin, silicon oil, synthetic ester oil and synthetic ether.

Furthermore, the liposome of the present invention having a functional food, nutritional supplementary food, or health supplementary food encapsulated therein or bonded thereto may be used as a food composition. The functional food, nutritional food or health supplementary food is not particularly limited and any food may be used as long as it is designed, processed and converted to be efficiently exhibit its function after absorption.

Examples of the functional food, nutritional food or health supplementary food may include ginkgo leaves, echinacea, sawfish coconut palm, St John's Wort. valerian, Black cohosh, milk thistle, evening primrose, grape seed extract, bilberry, feverfew, Japanese angelica root, soybean, French coast pine, garlic, ginseng, tea, ginger, agaricus, Fomes yucatensis, Merto purple, AHCC, yeast β-glucan, Grifola frondosa, propolis, beer yeast, cereals, plum, chlorella, barley young leaves, green juice, vitamins, collagen, Glcosamin, mulberry leaves, Rooibos tea, amino acids, the royal jelly, shiitake mycelia extract, Spirulina, Densichi carrot, cress, plant fermentation food, DHA, EPA, ARA, seaweed, cabbage, aloe, Nikko maple, hop, the extract of oyster, pycnogenol and sesame seeds, etc. These may be directly contained in a liposome and extracts are processed and contained in a liposome. A food composition containing liposomes may be orally taken. The liposomes that are used may not have a sugar chain bonded thereto and may have a sugar chain bonded thereto for enhancing absorption from the intestinal tract or targeting a predetermined tissue or organ. When the liposome of the present invention is administered as a food composition, it may be processed in the form of liquid beverage, gel food, and solid food. Alternatively, it may be processed in the form of tablets, granules, and the like.

The food composition of this invention can be used as a functional food, nutritional or health supplementary food in accordance with types of foods that liposomes contain. For instance, liposome including DHA can be used as an effective functional food, nutritional or health supplementary food for improving a slight senile dementia and amnesia.

The present invention will be explained in more detail by way of Examples, which should not be construed as limiting the present invention.

EXAMPLE 1

Preparation of Liposomes

Liposomes were prepared through an improved type of cholate dialysis based on a previously reported method (Yamazaki, N., Kodama, M. and H.-J. Gabius. *Methods Enzymol.* 242:56-65 (1994)). More specifically, 46.9 mg of sodium cholate was added to 45.6 mg of lipid mixture consisting of dipalmitoylphosphatidylcholine, cholesterol, dicetylphosphate, ganglioside and dipalmitoylphosphatidylethanolamine at a mole ratio of 35:40:5:15:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffer solution (pH 8.4), and was subjected to a supersonic treatment to obtain a clear micelle suspension. Then, this micelle suspension was subjected to ultrafiltration by using a PM 10 membrane (Amicon Co., USA) and a PBS buffer solution (pH 7.2) to prepare 10 ml of a uniform liposome (average size of 100 nm).

EXAMPLE 2

Hydrophilization of Lipid Membrane Surface ofLiposomes

10 ml of the liposome solution prepared in Example 1 was subjected to ultrafiltration by using an XM 300 membrane (Amicon Co., USA) and a CBS buffer solution (pH 8.5) to adjust the pH of the solution to 8.5. Then, 10 ml of bis (sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the reaction between the BS3 and the dipalmitoylphosphatidyletanolamine of the lipid on the liposome membrane. This liposome solution was then subjected to ultrafiltration by using an XM 300 membrane and a CBS buffer solution (pH 8.5). Then, 40 mg of tris (hydroxymethyl) aminomethane dissolved in 1 ml of CMS buffer solution (pH 8.5) was added to 10 ml of the liposome solution. The obtained solution was stirred at 25°C for 2 hours, and stirred at 7°C for one night to complete the reaction between the BS3 bonded to the lipid on the liposome membrane and the tris (hydroxymethyl) aminomethane. In this manner, the hydroxyl groups of the tris (hydroxymethyl) aminomethane were coordinated on the dipalmitoylphosphatidyletanolamine of the lipid on the liposome membrane to achieve the hydrophilization of the lipid membrane surface of the liposome

EXAMPLE 3

Bonding of Human Serum Albumin (HSA) to Membrane Surface of Liposomes

Human serum albumin (HSA) was bonded to the membrane surface of the liposome through a coupling reaction method based on a previously reported method (Yamazaki, N., Kodama, M. and H. -J. Gabius. *Methods Enzymol.* 242: 56-65 (1994)). More specifically, the reaction was carried out through a two-stage reaction method. That is, 43 mg of sodium metaperiodate dissolved in 1 ml of TAPS buffer solution (pH 8.4) was added to 10 ml of the liposome obtained in Example 2, and the obtained solution was stirred at room temperature for 2 hours to periodate-oxidize the ganglioside on the membrane surface of the liposome. Then, the solution was subjected to ultrafiltration by using an XM 300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 $\mu$l of 2M $NaBH_3CN$ was added to the PBS buffer solution (pH 8.0), and the obtained solution was stirred at 10°C for one night to bond the HSA to the liposome membrane surface through a coupling reaction between the HSA and the ganglioside on the liposome. Then, 10 ml of HSA-bonded liposome solution was obtained through an ultrafiltration using an XM 300 membrane and a CBS buffer solution (pH 8.5).

EXAMPLE 4

Bonding of α-1,2-mannobiose Disaccharide to Human Serum Albumin (HSA) Bonded on Liposome Membrane Surfaces

50 μg of α-1,2-mannobiose disaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 μm to complete an amination reaction at the reduction terminal of the sugar chain and obtain 50 μg of glycosylamine compound of the α-1,2-mannobiose disaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 μg of the glycosylamine compound of the α-1,2-mannobiose disaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the α-1,2-mannobiose disaccharide to the DTSSP on the human serum albumin bonded on the liposome membrane surface. In this manner, 2 ml of a liposome (referred to as A2), in which α-1,2-mannobiose disaccharide is bonded to the liposome through human serum albumin (FIG. 1) (total lipid mass: 2 mg, total protein mass: 200 μg, average particle size: 100 nm), were obtained.

EXAMPLE 5

Bonding of α-1,3-mannobiose Disaccharide to Human Serum Albumin (HSA) Bonded on Liposome Membrane Surfaces

50 μg of α-1,3-mannobiose disaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 μm to complete an amination reaction at the reduction terminal of the sugar chain and obtain 50 μg of glycosylamine compound of the α-1,3-mannobiose disaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 μg of the glycosylamine compound of the α-1,3-mannobiose disaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the α-1,3-mannobiose disaccharide to the DTSSP on the human serum albumin bonded on the liposome membrane surface. In this manner, 2 ml of a liposome (referred to as A3), in which α-1,3-mannobiose disaccharide is bonded to the liposome through human serum albumin (FIG. 2) (total lipid mass: 2 mg, total protein mass: 200 μg, average particle size: 100 nm), were obtained.

EXAMPLE 6

Bonding of α-1,4-mannobiose Disaccharide to Human Serum Albumin (HSA) Bonded on Liposome Membrane Surfaces

50 μg of α-1,4-mannobiose disaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 μm to complete an amination reaction at the reduction terminal of the sugar chain and obtain 50 μg of glycosylamine compound of the α-1,4-mannobiose disaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 μg of the glycosylamine compound of the alpha-1,4-mannobiose disaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the α-1,4-mannobiose disaccharide to the DTSSP on the human serum albumin bonded on the liposome membrane surface. In this manner, 2 ml of a liposome (referred to as A4), in which α-1,4-mannobiose disaccharide is bonded to the liposome through human serum albumin (FIG. 3) (total lipid mass: 2 mg, total protein mass: 200 μg, average particle size: 100 nm), were obtained.

EXAMPLE 7

Bonding of α-1,6-mannobiose Disaccharide to Human Serum Albumin (HSA) Bonded on Liposome Membrane Surfaces

50 μg of α-1,6-mannobiose disaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 μm to complete an amination reaction at the reduction terminal of the sugar chain and obtain 50 μg of glycosylamine compound of the α-1,6-mannobiose disaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 μg of the glycosylamine compound of the α-1,6-mannobiose disaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the α-1,6-mannobiose disaccharide to the DTSSP on the human serum albumin bonded on the liposome membrane surface. In this manner, 2 ml of a liposome (referred to as A6), in which α-1,6-mannobiose disaccharide is bonded to the liposome through human serum albumin (FIG. 4) (total lipid mass: 2 mg, total protein mass: 200 μg, average particle size: 100 nm), were obtained.

EXAMPLE 8

Bonding of α-1,3/α-1,6-mannotriose trisaccharide to Human Serum Albumin (HSA) Bonded on Liposome Membrane Surfaces

50 μg of α-1,3/α-1,6-mannotriose trisaccharide (Calbiochem Co.., USA) was added to 0.5 ml of water solution having 0.25 g ofNH4HCO3 dissolved therein, and the obtained solution was stirred at 37°C for 3 days. Then, the solution was filtered by using a filter of 0.45 μm to complete an amination reaction at the reduction terminal of the sugar chain and obtain 50 μg of glycosylamine compound of the α-1,3/α-1,6-mannotriose trisaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 μg of the glycosylamine compound of the α-1,3/α-1,6-mannotriose trisaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the α-1,3/α-1,6-mannotriose trisaccharide to the DTSSP on the human serum albumin bonded on the liposome membrane surface. In this manner, 2 ml of a liposome (referred to as A36), in which α-1,3/α-1,6-mannotriose trisaccharide is bonded to the liposome through human serum albumin (FIG. 5) (total lipid mass: 2 mg, total protein mass: 200 μg, average particle size: 100 nm), were obtained.

EXAMPLE 9

Bonding of Oligomannose-3 pentasaccharide to Human Serum Albumin (HSA) Bonded on Liposome Membrane Surfaces

50 μg of oligomannose-3 pentasaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 μm to complete an amination reaction at the reduction terminal of the sugar chain and obtain 50 μg of glycosylamine compound of the oligomannose-3 pentasaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 μg of the glycosylamine compound of the oligomannose-3 pentasaccharide was added to the liposome solution. The obtained solution was stirred at 25°C for 2 hours; and subsequently stirred at 7°C for one night. Then, the solution was subjected to ultrafiltration by using an XM 300 membrane and a PBS buffer solution (pH 7.2) to bond the oligomannose-3 pentasaccharide to the DTSSP on the human serum albumin bonded on the liposome membrane surface. In this manner, 2 ml of a liposome (referred to as Man3), in which oligomannose-3 pentasaccharide is bonded to the liposome through human serum albumin (FIG. 6) (total lipid mass: 2 mg, total protein mass: 200 μg,

average particle size: 100 nm), were obtained.

EXAMPLE 10

Bonding of Oligomannose-4b hexasaccharide to Human Serum Albumin (HSA) Bonded on Liposome Membrane Surfaces

50 $\mu$g of oligomannose-4b hexasaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination reaction at the reduction terminal of the sugar chain and obtain 50 $\mu$g of glycosylamine compound of the oligomannose-4b hexasaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 $\mu$g of the glycosylamine compound of the oligomannose-4b hexasaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the oligomannose-4b hexasaccharide to the DTSSP on the human serum albumin bonded on the liposome membrane surface. In this manner, 2 ml of a liposome (referred to as Man 4b), in which oligomannose-4b hexasaccharide is bonded to the liposome through human serum albumin (FIG. 7) (total lipid mass: 2 mg, total protein mass: 200 $\mu$g, average particle size: 100 nm), were obtained.

EXAMPLE 11

Bonding of Oligomannose-5 heptasaccharide to Human Serum Albumin (HSA) Bonded on Liposome Membrane Surfaces

50 $\mu$g of oligomannose-5 heptasaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination reaction at the reduction terminal of the sugar chain and obtain 50 $\mu$g of glycosylamine compound of the oligomannose-5 heptasaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 $\mu$g of the glycosylamine compound of the oligomannose-5 heptasaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the oligomannose-5 heptasaccharide to the DTSSP on the human serum albumin bonded on the liposome membrane surface. In this manner, 2 ml of a liposome (referred to as Man 5), in which oligomannose-5 heptasaccharide is bonded to the liposome through human serum albumin (FIG. 8) (total lipid mass: 2 mg, total protein mass: 200 $\mu$g, average particle size: 100 nm), were obtained.

EXAMPLE 12

Bonding of Oligomannose-6 octasaccharide to Human Serum Albumin (HSA) Bonded on Liposome Membrane Surfaces

50 $\mu$g of oligomannose-6 octasaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination reaction at the reduction terminal of the sugar chain and obtain 50 $\mu$g of glycosylamine compound of the oligomannose-6 octasaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 $\mu$g of the glycosylamine compound of the oligomannose-6 octasaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the oligomannose-6 octasaccharide to the DTSSP on the human serum albumin bonded on the liposome membrane surface. In this manner, 2 ml of a liposome (referred to as Man 6), in which oligomannose-6 octasaccharide is bonded to the liposome through human serum albumin (FIG. 9) (total lipid mass: 2 mg, total protein mass: 200 $\mu$g, average particle size: 100 nm), were obtained.

EXAMPLE 13

Bonding of Oligomannose-7 nonasaccharide to Human Serum Albumin (HSA) Bonded on Liposome Membrane Surfaces

50 $\mu$g of oligomannose-7 nonasaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination reaction at the reduction terminal of the sugar chain and obtain 50 $\mu$g of glycosylamine compound of the oligomannose-7 nonasaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 $\mu$g of the glycosylamine compound of the oligomannose-7 nonasaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the oligomannose-7 nonasaccharide to the DTSSP on the human serum albumin bonded on the liposome membrane surface. In this manner, 2 ml of a liposome (referred to as Man 7), in which oligomannose-7 nonasaccharide is bonded to the liposome through human serum albumin (FIG. 10) (total lipid mass: 2 mg, total protein mass: 200 $\mu$g, average particle size: 100 nm), were obtained.

EXAMPLE 14

Bonding of Oligomannose-8 decasaccharide to Human Serum Albumin (HSA) Bonded on Liposome Membrane Surfaces

50 $\mu$g of oligomannose-8 decasaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination reaction at the reduction terminal of the sugar chain and obtain 50 $\mu$g of glycosylamine compound of the oligomannose-8 decasaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 $\mu$g of the glycosylamine compound of the oligomannose-8 decasaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the oligomannose-8 decasaccharide to the DTSSP on the human serum albumin bonded on the liposome membrane surface. In this manner, 2 ml of a liposome (referred to as Man 8), in which oligomannose-8 decasaccharide is bonded to the liposome through human serum albumin (FIG. 11) (total lipid mass: 2 mg, total protein mass: 200 $\mu$g, average particle size: 100 nm), were obtained.

EXAMPLE 15

Bonding of Oligomannose-9 undecasaccharide to Human Serum Albumin (HSA) Bonded on Liposome Membrane Surfaces

50 $\mu$g of oligomannose-9 undecasaccharide (Calbiochem Co.., USA) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination reaction at the reduction terminal of the sugar chain and obtain 50 $\mu$g of glycosylamine compound of the oligomannose-9 undecasaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 µg of the glycosylamine compound of the oligomannose-9 undecasaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the oligomannose-9 undecasaccharide to the DTSSP on the human serum albumin bonded on the liposome membrane surface. In this manner, 2 ml of a liposome (referred to as Man 9), in which oligomannose-9 undecasaccharide is bonded to the liposome through human serum albumin (FIG. 12) (total lipid mass: 2 mg, total protein mass: 200 µg, average particle size: 100 nm), were obtained.

EXAMPLE 16

Bonding of Tris (Hydroxymethyl) Aminomethane to Human Serum Albumin (HSA) Bonded on Liposome Membrane Surfaces

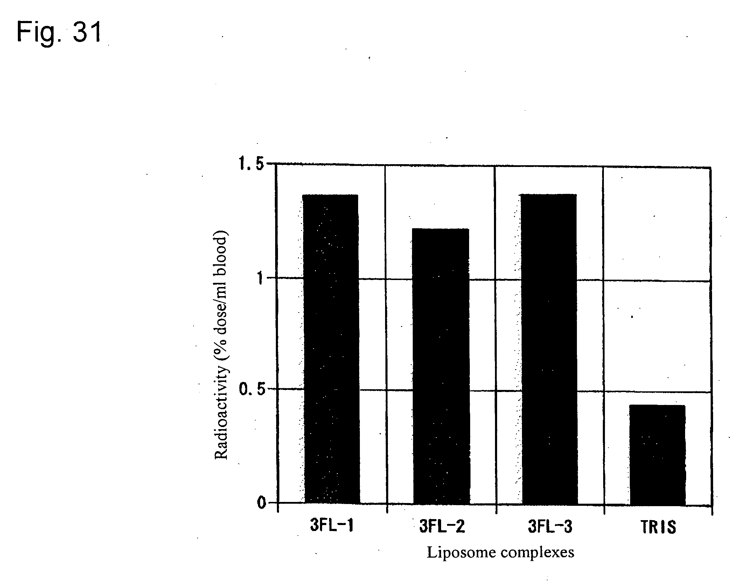

For preparing a liposome as a comparative sample, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was stirred at 25°C for 2 hours, and subsequently stirred at 7°C for one night. The solution was then subjected to ultrafiltration by using an XM 300 membrane and a CBS buffer solution (pH 8.5) to obtain 1 ml of liposome in which the DTSSP was bonded to the HSA on the liposome. Then, 13 mg of tris (hydroxymethyl) aminomethane (Wako Co., Japan) was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the tris (hydroxymethyl) aminomethane to the DTSSP on the human serum albumin bonded on the liposome membrane surface. In this process, an excess amount of tris (hydroxymethyl) aminomethane, that is 13 mg, already exists. Thus, the hydrophilization of the human serum albumin (HSA) bonded on the liposome membrane surface was simultaneously completed. In this manner, 2 ml of the liposome as the comparative sample (TRIS) in which the tris (hydroxymethyl) aminomethane is bonded to human serum albumin (FIG. 31) (total lipid mass: 2 mg, total protein mass: 200 µg, average particle size: 100 nm) was obtained.

EXAMPLE 17

Hydrophilization of Human Serum Albumin Bonded on Liposome Membrane Surfaces

For the 12 types of sugar-modified liposomes prepared in Examples 4 to 15, the respective HSA protein surfaces were separately hydrophilized through the following process. 13 mg of tris (hydroxymethyl) aminomethane was added to each of the 12 types of sugar-modified liposomes (2 ml each). The respective obtained solutions were stirred at 25°C for 2 hours, and subsequently stirred at 7°C for one night. The solutions were then subjected to ultrafiltration by using an XM 300 membrane and a PBS buffer solution (pH 7.2) to remove unreacted materials. In this manner, 2 ml of final product for each of the 12 types of hydrophilized sugar-modified liposome complexes (A2, A3, A4, A6, A36, Man3, Man4, Man5, Man6, Man7, Man8 and Man9) were obtained.

EXAMPLE 18

Measurement of Lectin-Binding Activity Inhibiting Effect in Each Type of Sugar-Modified Liposome Complex

The in vitro lectin-binding activity of each of the 12 types of hydrophilized sugar-modified liposomes prepared in Examples 4 to 15 and 17 were measured through an inhibition test using a lectin-immobilized microplate by methods known in the art (see, e.g., Yamazaki, N., et al., *Drug Delivery System,* 14:498-505 (1999)). More specifically, a lectin (Con A; R&D Systems Co., USA) was immobilized on a 96 well-microplate. Then, 0.1 µg of biotinylated fetuin as a comparative ligand, and various types of sugar-modified liposome complexes having different densities (each including 0.01 µg, 0.04 µg, 0.11 µg, 0.33 µg or 1 µg of protein), were placed on the lectin-immobilized plate, and incubated at 4°C for 2 hours. After washing with PBS (pH 7.2) three times, horseradish peroxidase (HRPO)-conjugated streptavidin was added to each of the wells. The respective test solutions were incubated at 4°C for 1 hour, and then washed with PBS (pH 7.2) three times. Then, peroxidase substrates were added to the test solutions, and incubated at room temperature. Then, the absorbance at 405 nm of each of the test solutions was determined by a microplate reader (Molecular Devices Corp., USA). For the biotinylation of the fucosylated fetuin, each of the test solutions was subject to a sulfo-NHS-biotin reagent (Pierce Chemical Co., USA) treatment and refined by using a Centricon-30 (Amicon Co., USA). HRPO-conjugated streptavidin was prepared by oxidizing HRPO and bonding streptavidin to the oxidized HRPO through a reductive amination method using $NaBH_3CN$. This measurement result is shown in Table 1

[Table 1]

TARGETING LIPOSOME

Table 1: Results of experiments demonstrating inhibitory effect of various sugar chain bonded liposome on lectin binding activity.

| Liposome complex | Inhibitory effect (absorbancy) at each concentration ($\mu$g protein) of liposome complex | | | | |
|---|---|---|---|---|---|
| | 0.006 $\mu$g | 0.02 $\mu$g | 0.06 $\mu$g | 0.17$\mu$g | 0.5 $\mu$g |
| A2 | 0.192 | 0.196 | 0.192 | 0.169 | 0.155 |
| A3 | 0.178 | 0.178 | 0.178 | 0.170 | 0.142 |
| A4 | 0.192 | 0.196 | 0.192 | 0.175 | 0.153 |
| A6 | 0.182 | 0.196 | 0.182 | 0.169 | 0.151 |
| A36 | 0.205 | 0.215 | 0.205 | 0.192 | 0.150 |
| Man3 | 0.201 | 0.211 | 0.201 | 0.177 | 0.144 |
| Man4 | 0.171 | 0.208 | 0.171 | 0.157 | 0.148 |
| Man5 | 0.215 | 0.221 | 0.215 | 0.196 | 0.164 |
| Man6 | 0.210 | 0.222 | 0.210 | 0.207 | 0.125 |
| Man7 | 0.213 | 0.214 | 0.213 | 0.183 | 0.137 |
| Man8 | 0.211 | 0.216 | 0.211 | 0.188 | 0.132 |
| Man9 | 0.208 | 0.211 | 0.208 | 0.186 | 0.135 |

EXAMPLE 19 $^{125}$I-Labeling of Each Type of Sugar-Modified Liposome through the Chloramine T Method

A chloramine T (Wako Pure Chemical Co., Japan) solution and a sodium disulfite solution were prepared at 3 mg/ml and 5 mg/ml, respectively. 50 $\mu$l of the 12 different types of sugar-modified liposomes prepared in Examples 4 to 16, and the tris (hydroxymethyl) aminomethane binding liposome, were put into separate Eppendorf tubes. Then, 15 $\mu$l of $^{125}$I-NaI (NEN Life Science Product, Inc. USA) and 10 $\mu$l of chloramine T solution were added thereto and reacted therewith. 10 $\mu$l of chloramine T solution was added to the respective solutions every 5 minutes. After 15 minutes from the completion of the above procedure repeated twice, 100 $\mu$l of sodium disulfite serving as a reducer was added to the solutions to stop the reaction. Then, each of the resulting solutions was placed on a Sephadex G-50 (Phramacia Biotech. Sweden) column chromatography, and eluted by PBS to purify a labeled compound. Finally, a non-labeled liposome complex was added to each of the solutions to adjust a specific activity ($4 \times 10^6$Bq/mg protein). In this manner, 13 types of $^{125}$I-labeled liposome solutions were obtained.

EXAMPLE 20

Measurement of Distribution Rate of Each Type of Sugar-Modified Liposome Complex to Tissues of Mice with Cancer

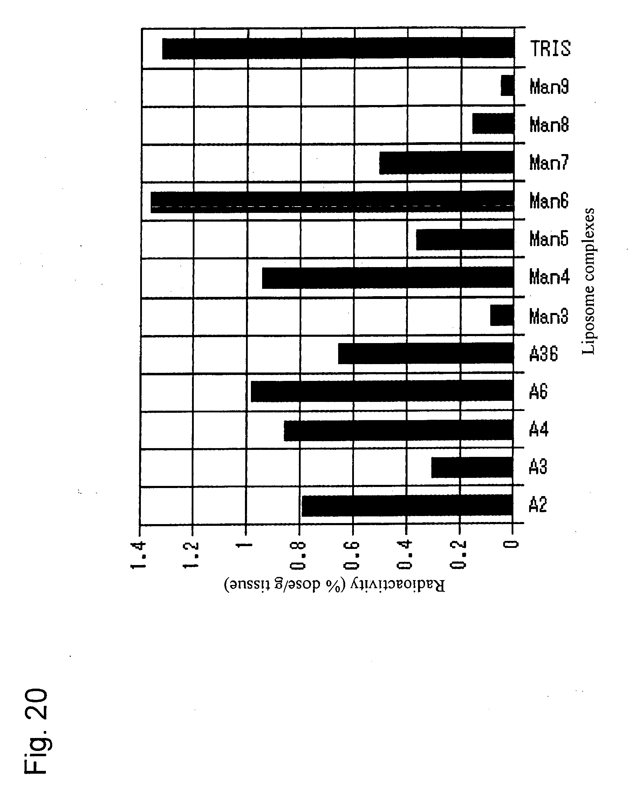

Ehrlich ascites tumor (EAT) cells (about $2 \times 10^7$ cells) were implanted subcutaneously into the femoral region in male ddY mice (7 weeks of age), and the mice were used in this test after the tumor tissues grew to 0.3 to 0.6 g (after 6 to 8 days). Twelve types of the $^{125}$I-labeled, hydrophilized sugar-modified liposome complexes of Example 19 were injected into the tail veins of the mice in an amount of 0.2 ml which is equivalent to 3 $\mu$g of protein per mouse. After 60 minutes, tissues (blood, liver, spleen, lung, brain, inflammatory tissues around cancer, cancer and lymph node) were extracted, and the radioactivity of each of the extracted tissues was measured with a gamma counter (Aloka ARC 300). The distribution rate of the radioactivity in each of the tissues was represented by a ratio of the radioactivity per gram of each of the tissues to the total of given radioactivity (% dose/g tissue). This measurement result is shown in FIGS. 13 to 22.

Example 21

Bonding of 3'-sialyllactose trisaccharide chain (three kinds with different amounts of bound sugar chain) to human serum albumin (HSA) bonded on liposome membrane surfaces

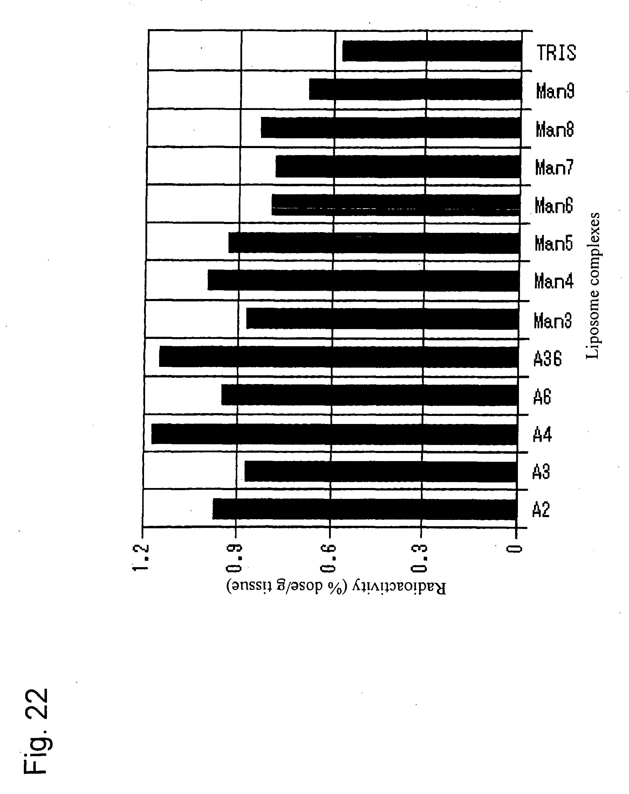

1) 50 $\mu$g, 2) 200 $\mu$g or 3) 1 mg of 3'-sialyllactose trisaccharide (Wako Pure Chemical Co., Japan) was added to 0.5 ml water solution having 0.25g of $NH_4HCO_3$, and the obtained solution was stirred at 37°C for 3 days. Then, the solution was filtered by using a filter of 0.45 $\mu$m filter to complete an amination at the reduced terminal of the sugar chain and obtain 50 $\mu$g of glycosylamine compound of 3'-sialyllactose 3 saccharide. Then, 1 mg of 3,3'-dithiobis (sulfosuccinimidyl) propionate (DTSSP; Pierce Co., USA) serving as a crosslinking reagent was added to 1 ml of a part of the liposome solution obtained in Example 3. The obtained solution was then stirred at 25°C for 2 hours and subsequently stirred at 7°C for one night. Then the solution was subjected to ultrafiltration by using an XM300 membrane and a CBS buffer solution (pH 8.5) to obtain 1 ml of liposome in which DTSPP was bonded to the HSA on the liposome. Then, 50 $\mu$g of the glycosylamine compound of 3'-sialyllactose 3 saccharide was added to the liposome solution. The obtained solution was stirred at 25°C for 2 hours, and subsequently stirred at 7°C for one night. Then, the solution was subjected to ultrafiltration by using an XM300 membrane and a PBS buffer solution (ph 7.2) to bond the 3'-sialyllactose trisaccharide to the DTSSP on the human serum albumin bonded on the liposome membrane surface. As the result, three types of liposomes (2 ml each) differing in the amount of sugar chain bonded thereto (referred to as: 1) 3SL-1, 2) 3SL-2 and 3) 3 SL-3), in which 3'-sialyllactose 3 saccharide is bonded to the liposome through human serum albumin (Fig. 22) (total lipid: 2 mg, total protein: 200 $\mu$g, average particle size: 100 nm), were obtained.

EXAMPLE 22

Bonding of 6'-sialyllactose trisaccharide to Human Serum Albumin (HSA) Bonded on Liposome Membrane Surfaces (3types of liposome differing in the amount of sugar chain bonded)

50 $\mu$g, 200 $\mu$g, or 1 mg of 6'-sialyllactose trisaccharide (Wako Pure Chemical Co., Japan) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination reaction at the reduction terminal of the sugar chain and obtain 50 $\mu$g of glycosylamine compound of the 6'-sialyllactose trisaccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 $\mu$g of the glycosylamine compound of the 6'-sialyllactose trisaccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the 6'-sialyllactose trisaccharide to the DTSSP on the human serum albumin bonded on the liposome membrane surface. In this manner, 3 types of liposomes (2 ml each), differing in the amount of sugar chain bonded thereto (referred to as 6SL-1 (50 $\mu$g), 6SL-2 (200 $\mu$g), and 6SL-3 (1 mg)), in which 6'-sialyllactose trisaccharide is bonded to the liposome through human serum albumin (FIG. 23) (total lipid mass: 2 mg, total protein mass: 200 $\mu$g, average particle size: 100 nm), were obtained.

EXAMPLE 23

Bonding of 3'-sialyllactosamine saccharide to Human Serum Albumin (HSA) Bonded on Liposome Membrane Surfaces (3types of liposome differing in the amount of sugar chain bonded)

50 $\mu$g, 200 $\mu$g, or 1 mg of 3'-sialyllactosamine saccharide (Wako Pure Chemical Co., Japan) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination reaction at the reduction terminal of the sugar chain and obtain 50 $\mu$g of glycosylamine compound of the 3'-sialyllactosamine saccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 μg of the glycosylamine compound of the 3'-sialyllactosamine saccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the 3'-sialyllactosamine saccharide to the DTSSP on the human serum albumin bonded on the liposome membrane surface. In this manner, 3 types of liposomes (2 ml each), differing in the amount of sugar chain bonded thereto (referred to as 3SLN-1 (50 μg), 3SLN-2 (200 μg), and 3SLN-3 (1 mg)), in which 3'-sialyllactosamine saccharide is bonded to the liposome through human serum albumin (FIG. 24) (total lipid mass: 2 mg, total protein mass: 200 μg, average particle size: 100 nm), were obtained.

EXAMPLE 24

Bonding of 6'-sialyllactosamine saccharide to Human Serum Albumin (HSA) Bonded on Liposome Membrane Surfaces (3types of liposome differing in the amount of sugar chain bonded)

50 μg, 200 μg, or 1 mg of 6'-sialyllactosamine saccharide (Wako Pure Chemical Co., Japan) was added to 0.5 ml of water solution having 0.25 g of $NH_4HCO_3$ dissolved therein, and the obtained solution was stirred at 37°C for 3 days. Then, the solution was filtered by using a filter of 0.45 μm to complete an amination reaction at the reduction terminal of the sugar chain and obtain 50 μg of glycosylamine compound of the 6'-sialyllactosamine saccharide. Then, 1 mg of 3,3'-dithiobis (sulfosuccinimidyl propionate) (DTSSP; Pierce Co., USA) serving as a crosslinking reagent was added to 1 ml of a part of the liposome solution obtained in Example 3. The obtained solution was then stirred at 25°C for 2 hours, and subsequently stirred at 7°C for one night. Then, the solution was subjected to ultrafiltration by using an XM 300 membrane and a CBS buffer solution (pH 8.5) to obtain 1 ml of liposome in which the DTSSP was bonded to the HSA on the liposome. Then, 50 μg of the glycosylamine compound of the 6'-sialyllactosamine saccharide was added to the liposome solution. The obtained solution was stirred at 25°C for 2 hours, and subsequently stirred at 7°C for one night. Then, the solution was subjected to ultrafiltration by using an XM 300 membrane and a PBS buffer solution (pH 7.2) to bond the 6'-sialyllactosamine saccharide to the DTSSP on the human serum albumin bonded on the liposome membrane surface. In this manner, 3 types of liposomes (2 ml each), differing in the amount of sugar chain bonded thereto (referred to as 6SLN-1 (50 μg), 6SLN-2 (200 μg), and 6SLN-3 (1mg)), in which 6'-sialyllactosamine saccharide is bonded to the liposome through human serum albumin (FIG. 25) (total lipid mass: 2 mg, total protein mass: 200 μg, average particle size: 100 nm), were obtained.

Example 25

Hydrophilization of Human Serum Albumin bonded on Liposome Membrane (HSA) Surface

For the 12 types of sugar-bonded liposomes prepared in Example 21 to 24, the respective HSA protein surface were separately hydrophilized through the following process. 13 mg of tris(hydroxymethyl)aminomethane was added to each of the 12 types of sugar chain-binding liposomes (2 ml each). The respective obtained solutions were stirred at 25°C for 2 hours, and subsequently stirred at 7°C for one night. The solutions were then subjected to ultrafiltration by using an XM300 membrane and a PBS buffer solution (pH 7.2) to remove unreacted materials. In this manner, 2 ml of final product for each of the 12 types of hydrophilized sugar chain-binding liposome complexes (referred to as: 3SL-2, 3SL-2, 3SL-3, 6SL-1, 6SL-2, 6SL-3, 3SLN-1, 3SLN-2, 3SLN-3, 6SLN-1, 6SLN-2, 6SLN-3) (total lipid mass: 2 mg, total protein mass: 200 μg, average particle size: 100 nm) were obtained.

Example 26

Measurement of Lectin-Binding Activity Inhibiting Effect in Each Type of Sugar Chain-Binding Liposome Complex

The in vitro lectin-binding activity of each of the 12 types of hydrophilized sugar chain-binding liposomes prepared in Examples 21 to 24 was measured through an inhibition test using a lectin-immobilized microplate by methods known in the art (Yamazaki, N. (1999) Drug Delivery System, 14, 498-505). More specifically, a lectin (E-selectin; R&D Systems Co., USA) was immobilized on a 96 well-microplate. Then, 0.1 μg of biotinylated and fucosylated fetuin as a comparative ligand, and various types of sugar chain-binding liposome complexes having different densities (each including 0.01 μg, 0.04 μg, 0.11 μg, 0.33 μg or 1 μg of protein), were placed on the lectin-immobilized plate, and incubated at 4°C for 2 hours. After washing with PBS (pH 7.2) three times, horseradish peroxidase (HRPO)-conjugated streptavidin was added to each of the wells. The respective test solutions were incubated at 4°C for 1 hour, and then washed with PBS (pH 7.2) three times. Then, peroxidase substrates were added to the test solutions, and incubated at room temperature. Then, the absorbance at 405 nm of each of the test solutions was determined by a microplate reader (Molecular Devices Corp., USA). For the biotinylation of the fucosylated fetuin, each of the test solutions was subjected to a sulfo-NHS-biotin reagent (Pierce Co., USA) treatment and refined by using a Centricon-30 (Amicon Co., USA). HRPO-conjugated streptavidin was prepared by oxidizing HRPO and bonding streptavidin to the oxidized HRPO through a reductive amination method using $NaBH_3CN$. This measurement result is shown in Table 2.

[Table 2]

Intestine Tract Absorption Control Liposome

Table 2

| Liposome complex | Inhibiting effect (absorbance) at each concentration ($\mu$g protein) of liposome complex | | | | |
|---|---|---|---|---|---|
| | 0.01 $\mu$g | 0.04 $\mu$g | 0.11 $\mu$g | 0.33 $\mu$g | 1 $\mu$g |
| 3SL-1 | 0.154 | 0.147 | 0.135 | 0.120 | 0.097 |
| 3SL-2 | 0.149 | 0.142 | 0.124 | 0.118 | 0.098 |
| 3 SL-3 | 0.214 | 0.214 | 0.210 | 0.183 | 0.167 |
| 6SL-1 | 0.177 | 0.171 | 0.167 | 0.160 | 0.114 |
| 6SL-2 | 0.196 | 0.184 | 0.169 | 0.160 | 0.159 |
| 6SL-3 | 0.214 | 0.207 | 0.196 | 0.192 | 0.183 |
| 3SLN-1 | 0.219 | 0.198 | 0.180 | 0.164 | 0.119 |
| 3SLN-2 | 0.155 | 0.155 | 0.151 | 0.119 | 0.096 |
| 3SLN-3 | 0.216 | 0.198 | 0.187 | 0.146 | 0.132 |
| 6SLN-1 | 0.257 | 0.246 | 0.233 | 0.200 | 0.151 |
| 6SLN-2 | 0.250 | 0.250 | 0.230 | 0.199 | 0.158 |
| 6SLN-3 | 0.248 | 0.231 | 0.227 | 0.201 | 0.144 |

Example 27

$^{125}$I-Labeling of Each Type of Sugar Chain-Binding Liposome through the Chloramine T Method

A chloramine T (Wako Pure Chemical Co., Japan) solution and a sodium disulfite solution were prepared at 3 mg/ml and 5 mg/ml, respectively. 50 $\mu$l of the 13 different types of hydrophilized sugar chain-binding liposomes prepared in Examples 21 to 24, and Example 16, were put into separate Eppendorf tubes. Then, 15 $\mu$l of $^{125}$I-NaI (NEN Life Science Product, Inc. USA) and 10 $\mu$l of chloramine T solution were added thereto and reacted therewith. 10 $\mu$l of chloramine T solution was added to the respective solutions every 5 minutes. After 15 minutes from the completion of the above procedure repeated twice, 100 $\mu$l of sodium disulfite serving as a reducer was added to the solutions to stop the reaction. Then, each of the resulting solutions was placed on a Sephadex G-50 (Phramacia Biotech. Sweden) column chromatography, and eluted by PBS to purify a labeled compound. Finally, a non-labeled liposome complex was added to each of the solutions to adjust a specific activity ($4 \times 10^6$ Bq/mg protein). In this manner, 13 types of $^{125}$I-labeled liposome solutions were obtained.

Example 28

Measurement of Transfer Rate of Each Type of Sugar Chain-Binding Liposome Complex from Intestine Tract to Blood Stream of Mice

Using an oral sonde, 13 different types of $^{125}$I-labeled, sugar-modified and tris(hydroxymethyl)aminomethane bonded liposome complexes of Example 27 were forcibly administered to the intestinal tract of male ddY mice (7 weeks of age) which had abstained from food, except for water, for one whole day, in an amount of 0.2 ml which is equivalent to 3 µg of protein per mouse. After 10 minutes, 1 ml of blood was taken from descending aorta under Nembutal anesthesia. Then, $^{125}$I-radioactivity in the blood was measured with a gamma counter (Alola ARC300). Further, in order to check the *in vivo* stability of each type of liposome complex, serum from each mouse's blood was subjected to chromatography using a Sephadex G-50. As a result, most of the radioactivity in each sample of serum was found in void fractions having a high molecular weight, and it was proved that each type of liposome complexes has a high *in vivo* stability. The radioactivity transfer rate from intestinal tract to blood was represented by the ratio of the radioactivity per ml of blood to the total of given radioactivity (% dose/ml blood). This measurement result is shown in Figs. 27 to 31.

Example 29

Preparation of Liposome Encapsulating Anti-Cancer Agent Doxorubicin

Liposome was prepared by using the cholate dialysis based method. More specifically, 46.9 mg of sodium cholate was added to 45.6 mg of lipid mixture consisting of dipalmitoylphosphatidylcholine, cholesterol, dicetylphosphate, ganglioside and dipalmitoylphosphatidylethanolamine at a molar ratio of 35:40:5:15:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 10 ml of a TAPS buffered saline solution (pH 8.4), and was subjected to a supersonic treatment to obtain 10 ml of a clear micelle suspension. Then, anticancer drug doxorubicin, completely dissolved in the TAPS buffer solution (pH 8.4) at 3 mg/ml, was added dropwise slowly to this micelle suspension while stirring. After being mixed homogeneously, this micelle suspension containing doxorubicin was subjected to ultrafiltration by using a PM10 membrane (Amicon Co., USA) and the TAPS buffered saline solution(pH 8.4) to prepare 10 ml of a uniform suspension of the liposome encapsulating anticancer drug doxorubicin. The liposome encapsulating anticancer drug doxorubicin in the resulting physiological saline suspension (37°C) thus obtained was subjected to analysis for the particle diameter and zeta potential using a measuring device for zeta potential, particle diameter and molecular weight (Model Nano ZS, Malvern Instrumentss Ltd., UK) and the particle diameter was 50 to 350 nm and the zeta potential was -30 to -10 mV.

Example 30

Hydrophilization of Lipid Membrane Surface of Liposomes Encapsulating Anticancer Drug Doxorubicin

10 ml of the liposome solution containing anticancer drug doxorubicin prepared in Example 29 was subjected to ultrafiltration using an XM300 membrane (Amicon Co., USA) and a CBS buffer solution (pH 8.5) to adjust the pH of the solution to 8.5. Then, 10 ml of bis(sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the reaction between the BS3 and the dipalmitoylphosphatidyletanolamine of the lipid on the liposome membrane. This liposome solution was then subjected to ultrafiltration by using an XM300 membrane and a CBS buffer solution (pH 8.5). Then, 40 mg of tris(hydroxymethyl)aminomethane dissolved in 1 ml of CBS buffer solution (pH 8.5) was added to 10 ml of the liposome solution. The obtained solution was stirred at 25°C for 2 hours, and stirred at 7°C for one night to complete the chemical bonding reaction between the BS3 bonded to the lipid on the liposome membrane and the tris(hydroxymethyl)aminomethane. In this manner, the hydroxyl groups of the tris(hydroxymethyl) aminomethane were coordinated on the lipid dipalmitoylphosphatidyletanolamine of the liposome membrane encapsulating the anticancer drug doxorubcin to achieve the hydrophilization thereof.

Example 31

Bonding of Human Serum Albumin (HSA) to Membrane Surface of Liposomes Encapsulating Anticancer Drug Doxorubicin

A coupling reaction method based on a previously reported method (Yamazaki, N., Kodama, M. and Gabius, H. -J. (1994) Methods Enzymol. 242, 56-65) was used. More specifically, the reaction was carried out through a two-stage reaction method. That is, 43 mg of sodium metaperiodate dissolved in 1 ml of TAPS buffer solution (pH 8.4) was added to 10 ml of the liposome obtained in Example 2, and the obtained solution was stirred at room temperature for 2 hours to periodate-oxidize the ganglioside on the membrane surface of the liposome. Then, the solution was subjected to ultrafiltration by using an XM300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 µl of 2M $NaBH_3CN$ was added to the PBS buffer solution (pH 8.0), and the obtained solution was stirred at 10°C for one night to bond the HSA to the liposome membrane surface through a coupling reaction between the HSA and the ganglioside on the liposome. Then, 10 ml of the solution of the HSA-bonded liposome encapsulating anticancer drug doxorubicin was obtained through an ultrafiltration using an XM300 membrane and a CBS buffer solution (pH 8.5).

Example 32

Bonding of $\alpha$-1-6 Mannobiose disaccharide to Human Serum Albumin (HSA) Bonded on Liposome Membrane Surfaces and Hydrophilization of Linker Protein (HAS)

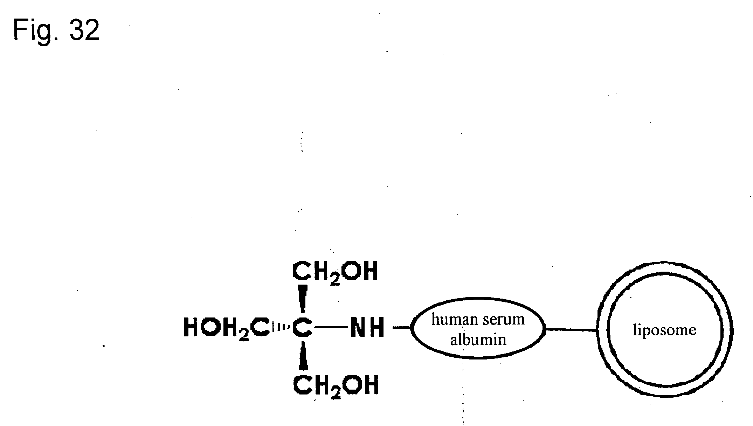

50 $\mu$g of $\alpha$-1,6-mannobiose disaccharide (Calbiochem Co., USA) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination at the reduced terminal of the sugar chain and obtain 50 $\mu$g of glycosylamine compound of the $\alpha$-1,6-mannobiose disaccharide. Then, 1 mg of 3,3'-dithiobis (sulfosuccinimidyl propionate) (DTSSP; Pierce Co., USA) serving as a crosslinking reagent was added to 1 ml of a part of the solution of the liposome encapsulating anticancer drug doxorubicin obtained in Example 31. The obtained solution was then stirred at 25°C for 2 hours, and subsequently stirred at 7°C for one night. Then, the solution was subjected to ultrafiltration by using an XM300 membrane and a CBS buffer solution (pH 8.5) to obtain 1 ml of liposome in which the DTSSP was bonded to the HSA on the liposome. Then, 50 $\mu$g of the glycosylamine compound of the $\alpha$-1,6-mannobiose disaccharide was added to the liposome solution. The obtained solution was stirred at 25°C for 2 hours, and subsequently stirred at 7°C for one night. Then, the solution was subjected to ultrafiltration by using an XM300 membrane and a PBS buffer solution (pH 7.2) to bond the $\alpha$-1,6-mannobiose to the DTSSP on the human serum albumin bonded on the liposome membrane surface. 13 mg of tris(hydroxymethyl)aminomethane was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. As the result, the liposome shown in Fig. 32 was obtained by hydrolizing the linker protein (HSA) in which tris(hydroxymethyl)aminomethane, human serum albumin and liposome were bonded. In this manner, 2 ml of the liposome encapsulating the anticancer drug doxorubicin with the hydrophilized linker protein (HSA), in which $\alpha$-1,6-mannobiose disaccharide, human serum albumin and liposome were bonded, (abbreviated as: DX-A6) (total lipid mass: 2 mg, total protein mass: 200 $\mu$g) shown in Fig. 4 was obtained. The suspension of the liposome encapsulating anticancer drug doxorubicin in a physiological saline (37°C) thus obtained was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

Example 33

Bonding of 3-fucosyllactose trisaccharide to Human Serum Albumin (HSA) Bonded on Liposome Membrane Surfaces and Hydrophilization of Linker Protein (HAS)

50 $\mu$g of 3-fucosyllactose trisaccharide (Calbiochem Co., USA) was added to 0.5 ml of water solution having 0.25 g of $NH_4HCO_3$ dissolved therein, and the obtained solution was stirred at 37°C for 3 days. Then, the solution was filtered by using a filter of 0.45 $\mu$m to complete an amination at the reduced terminal of the sugar chain and obtain 50 $\mu$g of glycosylamine compound of the $\alpha$-1,6-mannobiose disaccharide. Then, 1 mg of 3,3'-dithiobis (sulfosuccinimidyl propionate) (DTSSP; Pierce Co., USA) serving as a crosslinking reagent was added to 1 ml of a part of the solution of the liposome encapsulating anticancer drug doxorubicin obtained in Example 31. The obtained solution was then stirred at 25°C for 2 hours, and subsequently stirred at 7°C for one night. Then, the solution was subjected to ultrafiltration by using an XM300 membrane and a CBS buffer solution (pH 8.5) to obtain 1 ml of liposome in which the DTSSP was bonded to the HSA on the liposome. Then, 50 $\mu$g of the glycosylamine compound of the 3-fucosyllactose trisaccharide was added to the liposome solution. The obtained solution was stirred at 25°C for 2 hours, and subsequently stirred at 7°C for one night. Then, the solution was subjected to ultrafiltration by using an XM300 membrane and a PBS buffer solution (pH 7.2) to bond the 3-fucosyllactose trisaccharide to the DTSSP on the human serum albumin bonded on the liposome membrane surface. 13 mg of tris(hydroxymethyl)aminomethane was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. As the result, the liposome shown in Fig. 32 was obtained by hydrolizing the linker protein (HSA) in which tris(hydroxymethyl)aminomethane, human serum albumin and liposome were bonded. In this manner, 2 ml of the liposome encapsulating the anticancer drug doxorubicin with the hydrophilized linker protein (HSA), in which 3-fucosyllactose trisaccharide, human serum albumin and liposome were bonded, (abbreviated as: DX-3FL) (total lipid mass: 2 mg, total protein mass: 200 μg) shown in Fig. 38 was obtained. The suspension of the liposome encapsulating anticancer drug doxorubicin in a physiological saline (37°C) thus obtained was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

Example 34

Hydrophilization of Linker Protein (HSA) by the Bonding of Tris(hydroxymethyl)aminomethane to Human Serum Albumin (HSA) Bonded on Membrane Surfaces of Liposome Encapsulating Anticancer Drug Doxorubicin

To prepare liposome encapsulating anticancer drug doxorubicin as a comparative sample, 1 mg of 3, 3'-dithiobis (sulfosuccinimidyl) propionate (DTSSP; Pierce Co., USA) serving as a crosslinking reagent was added to 1 ml of a part of the solution of the liposome encapsulating anticancer drug doxorubicin obtained in Example 31. The obtained solution was then stirred at 25°C for 2 hours and subsequently stirred at 7°C for one night. Then the solution was subjected to ultrafiltration by using an XM300 membrane and a CBS buffer solution (pH 8.5) to obtain 1 ml of liposome with the hydrophylized linker protein (HSA) in which DTSPP was bonded to the HSA on the liposome. 13 mg of tris(hydroxymethyl) aminomethane (Wako Co., Japan) was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. In this manner, 2 ml of the liposome encapsulating the anticancer drug doxorubicin with the hydrophilized linker protein (HSA), in which tris(hydroxymethyl)aminomethane, human serum albumin and liposome were bonded, (abbreviated as DX-TRIS) (total lipid mass: 2 mg, total protein mass: 200 μg) shown in Fig. 32 was obtained. The suspension of the liposome encapsulating anticancer drug doxorubicin in a physiological saline (37°C) thus obtained was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

Example 35

Measurement of Carcinostatic Effect by Tail Vein Administration of Each Type of Sugar Chain-Binding Liposome Complex in Mice with Cancer

Ehrlich ascites tumor (EAT) cells (about $2 \times 10^7$ cells) were implanted subcutaneously into the right femoral region in male ddY mice (7 weeks of age), and the 40 mice were used in this test after the tumor tissues grew to 50 to 100 cubic mm (after 6 to 8 days). These mice with cancer were separated into 4 groups, 10 mice in each group. Mice in each group were injected with the sugar chain-binding liposome complex containing anticancer drug doxorubicin prepared in Example 32, the liposome without sugar-modification containing anticancer drug doxorubicin prepared in Example 34, physiological saline or free doxorubicin solution into the tail veins in an amount of 0.2 ml per mouse every 3 to 4 days 4 times (7, 11, 14 and 18 days after implantation of cancer cells). The volume of the cancer was calculated from the following formula by measuring the major axis (L) and the minor axis (S) with a vernier caliper:

$$\text{Cancer Volume (cubic mm)} = 1/2 \times L \times S \times S.$$

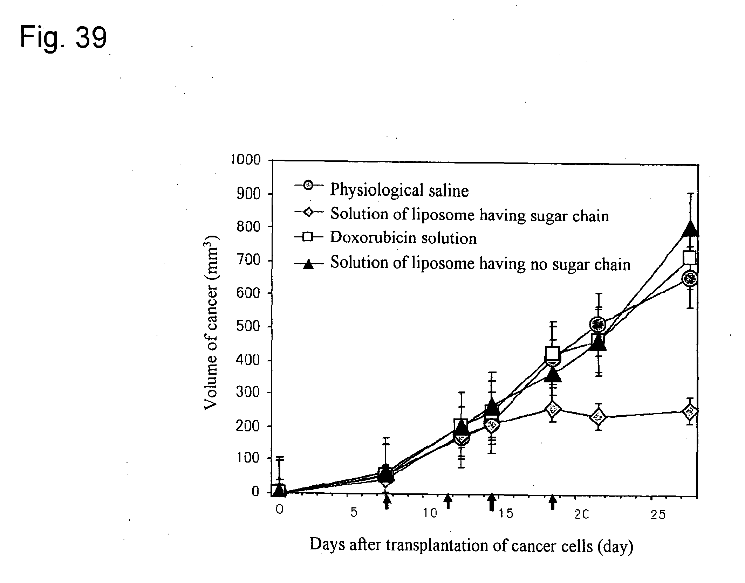

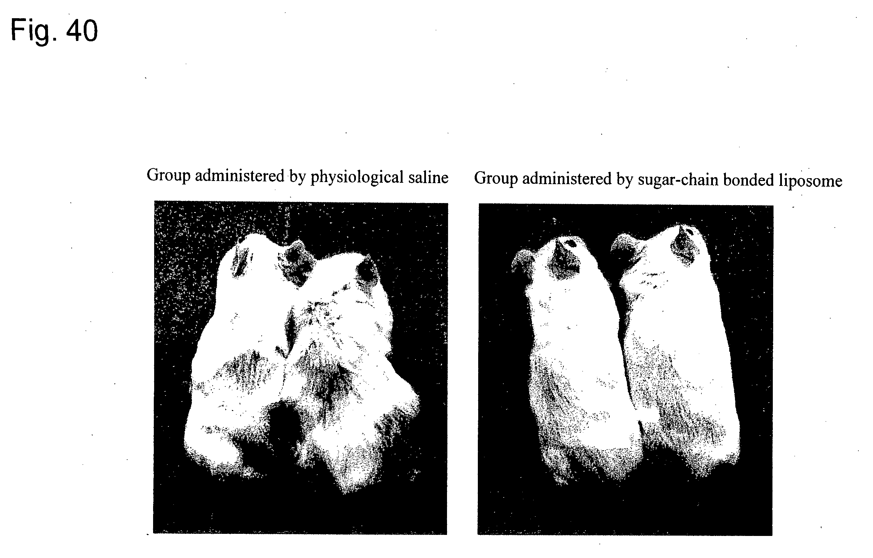

The results are shown in Fig. 39 or Fig. 40. As shown in the figures, the use of the liposome encapsulating doxorubicin of the present invention resulted in suppression of an increase in the cancer volume, and an inhibitory effect on cancer was recognized.

Example 36

Measurement of Carcinostatic Effect by Oral Administration of Each Type of Sugar Chain-Binding Liposome Complex in Mice with Cancer

Ehrlich ascites tumor (EAT) cells (about $2 \times 10^7$ cells) were implanted subcutaneously into the right femoral region in male ddY mice (7 weeks of age), and the 30 mice were used in this test after the tumor tissues grew to 50 to 100 cubic mm (after 6 to 8 days). These mice with cancer were separated into three groups, 10 mice in each group.

Mice in each group were orally administered with the sugar chain-binding liposome complex containing anticancer drug doxorubicin prepared in Example 33, the liposome without sugar-modification containing anticancer drug doxorubicin prepared in Example 34 or physiological saline in an amount of 0.6 ml per mouse every 3 to 4 days 4 times (7, 11, 14 and 18 days after implantation of cancer cells). The volume of the cancer was calculated from the following formula by measuring the major axis (L) and the minor axis (S) with a vernier caliper:

$$\text{Cancer Volume (cubic mm)} = 1/2 \times L \times S \times S.$$

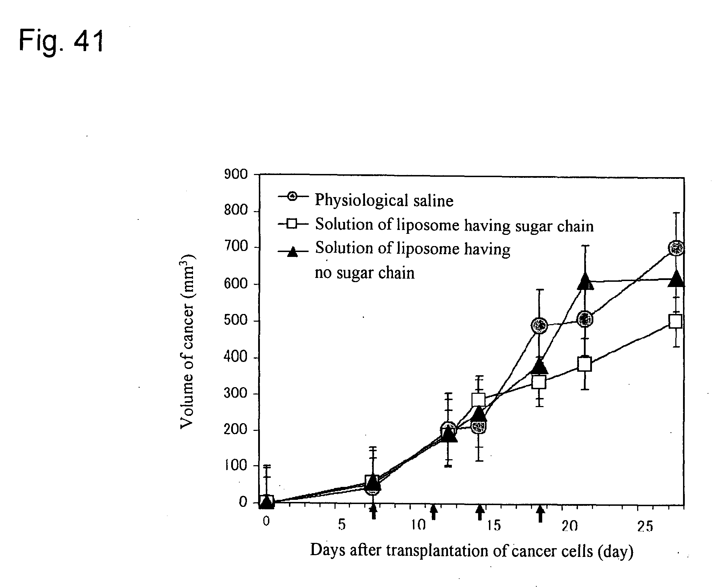

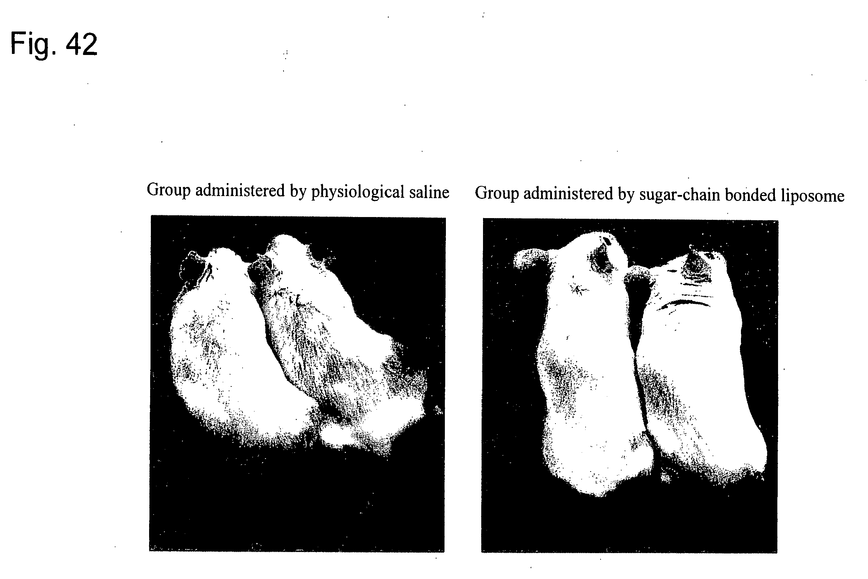

The results are shown in Fig. 41 or Fig. 42. As shown in the figures, the use of the liposome encapsulating doxorubicin of the present invention resulted in suppression of an increase in the cancer volume, and an inhibitory effect on cancer was recognized.

Example 37

Preparation of Targeting Liposome for Leukemia Treatment

(1) Preparation of Liposome Encapsulating Doxorubicin, Assay for Encapsulated Drug and Storage Stability

Liposome was prepared by using the cholate dialysis based method. More specifically, 46.9 mg of sodium cholate was added to 45.6 mg of lipid mixture consisting of dipalmitoylphosphatidylcholine, cholesterol, dicetylphosphate, ganglioside and dipalmitoylphosphatidylethanolamine at a molar ratio of 35:40:5:15:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffered saline solution (pH 8.4), and was subjected to a supersonic treatment to obtain 3 ml of a clear micelle suspension. Then, doxorubicin, completely dissolved in the TAPS buffer solution (pH 8.4) at 3 mg/ml, was added dropwise slowly to this micelle suspension while stirring. After being mixed homogeneously, this micelle suspension containing doxorubicin was subjected to ultrafiltration by using a PM10 membrane (Amicon Co., USA) and a TAPS buffered saline solution (pH 8.4) to prepare 10 ml of a uniform suspension of the liposome encapsulating doxorubicin. The suspension of the liposome encapsulating anticancer drug doxorubicin in a physiological saline (37°C) thus obtained was subjected to analysis for the particle diameter and zeta potential using a measuring device for zeta potential, particle diameter and molecular weight (Model Nano ZS, Malvern Instruments Ltd., UK) and the average particle diameter was 50 to 350 nm and the zeta potential was -30 to -10 mV. The amount of the drug encapsulated in the liposome was measured by the absorbancy at 485 nm and it turned out that doxorubicin was encapsulated at the concentration of about 110 $\mu$g/ml. This liposome encapsulating doxorubicin was stable without precipitation or coagulation after storing 1 year in a refrigerator.

(2) Hydrophilization of Lipid Membrane Surface of Liposomes Containing Anticancer Drug Doxorubicin

10 ml of the liposome solution encapsulating anticancer drug doxorubicin prepared in (1) was subjected to ultrafiltration by using an XM300 membrane (Amicon Co., USA) and a CBS buffer solution (pH 8.5) to adjust the pH of the solution to 8.5. Then, 10 ml of bis (sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the chemical bounding reaction between the BS3 and the dipalmitoylphosphatidyletanolamine of the lipid on the liposome membrane. This liposome solution was then subjected to ultrafiltration by using an XM300 membrane and a CBS buffer solution (pH 8.5). Then, 40 mg of tris(hydroxymethyl)aminomethane dissolved in 1 ml of CBS buffer solution (pH 8.5) was added to 10 ml of the liposome solution. The obtained solution was stirred at 25°C for 2 hours, and stirred at 7°C for one night to complete the chemical bonding reaction between the BS3 bonded to the lipid on the liposome membrane and the tris(hydroxymethyl)aminomethane. In this manner, the hydroxyl groups of the tris (hydroxymethyl)aminomethane were coordinated on the dipalmitoylphosphatidyletanolamine of the lipid on the liposome membrane to achieve the hydrophilization of the lipid membrane surface of the liposome encapsulating anticancer drug doxorubicin.

(3) Bonding of Human Serum Albumin (HSA) to Membrane Surface of Liposomes Containing Anticancer Drug Doxorubicin

A coupling reaction method based on a previously reported method (Yamazaki, N., Kodama, M. and Gabius, H. -J. (1994) Methods Enzymol. 242, 56-65) was used. More specifically, the reaction was carried out through a two-stage reaction method. That is, 43 mg of sodium metaperiodate dissolved in 1 ml of TAPS buffer solution (pH 8.4) was added to 10 ml of the liposome obtained in Example 2, and the obtained solution was stirred at room temperature for 2 hours to periodate-oxidize the ganglioside on the membrane surface of the liposome. Then, the solution was subjected to ultrafiltration by using an XM300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 $\mu$l of 2M $NaBH_3CN$ was added to the PBS buffer solution (pH 8.0), and the obtained solution was stirred at 10°C for one night to bond the HSA to the liposome membrane surface through a coupling reaction between the HSA and the ganglioside on the liposome. Then, 10 ml of the solution of the HSA-bonded liposome encapsulating anticancer drug doxorubicin was obtained through an ultrafiltration using an XM300 membrane and a CBS buffer solution (pH 8.5).

(4) Bonding of Sialyl Lewis X Tetrasaccharide to Human Serum Albumin (HSA) Bonded on Membrane Surface of Liposome Encapsulating Anticancer Drug Doxorubicin and Hydrophilization of Linker Protein (HSA)

50 $\mu$g of sialyl Lewis X tetrasaccharide (Calbiochem Co., USA) was added to 0.5 ml water solution having 0.25 g of $NH_4HCO_3$, and the obtained solution was stirred at 37°C for 3 days. Then, the solution was filtered using a filter of 0.45 $\mu$m filter to complete an amination at the reduced terminal of the sugar chain and obtain 50 $\mu$g of glycosylamine compound of sialyl Lewis X tetrasaccharide. Then, 1 mg of 3,3'-dithiobis(sulfosuccinimidyl) propionate (DTSSP; Pierce Co., USA) serving as a crosslinking reagent was added to 1 ml of a part of the solution of the liposome encapsulating anticancer drug doxorubicin obtained in (3). The obtained solution was then stirred at 25°C for 2 hours and subsequently stirred at 7°C for one night. Then the solution was subjected to ultrafiltration by using an XM300 membrane and a CBS buffer solution (pH 8.5) to obtain 1 ml of liposome in which DTSSP was bonded to the HSA on the liposome. Then, 50 $\mu$g of the glycosylamine compound of sialyl Lewis X tetrasaccharide was added to the liposome solution. The obtained solution was stirred at 25°C for 2 hours, and subsequently stirred at 7°C for one night. Then, the solution was subjected to ultrafiltration by using an XM300 membrane and a PBS buffer solution (pH 7.2) to bond the sialyl Lewis X tetrasaccharide to the DTSSP on the human serum albumin bonded on the liposome membrane surface. 13 mg of tris(hydroxymethyl) aminomethane (Wako Co., Japan) was added to this liposome solution. The obtained solution was stirred at 25°C for 2 hours, and subsequently stirred at 7°C for one night. The solutions were then subjected to ultrafiltration using an XM300 membrane and a PBS buffer solution (pH 7.2) to bond tris(hydroxymethyl)aminomethane to the DTSSP on the human serum albumin bonded on the liposome membrane surface. As the result, the liposome with the hydrophilized linker protein (HSA), in which tris(hydroxymethyl)aminomethane, human serum albumin and liposome were bonded, was obtained. In this manner, 2 ml of the liposome encapsulating the anticancer drug doxorubicin with the hydrophilized linker protein (HSA), in which sialyl Lewis X tetrasaccharide, human serum albumin and liposome were bonded, (total lipid mass: 2 mg, total protein mass: 200 $\mu$g) was obtained. The suspension of the sialyl Lewis X tetrasaccharide bonded liposome encapsulating anticancer drug doxorubicin in a physiological saline (37°C) thus obtained was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

(5) Hydrophilization of Linker Protein (HSA) by the Bonding of Tris(hydroxymethyl)aminomethane to Human Serum Albumin (HSA) Bonded on Membrane Surfaces of Liposome Encapsulating Anticancer Drug Doxorubicin

To prepare liposome encapsulating anticancer drug doxorubicin as a comparative sample (without bonded sugar), 1 mg of 3, 3'-dithiobis (sulfosuccinimidyl) propionate (DTSSP; Pierce Co., USA) serving as a crosslinking reagent was added to 1 ml of a part of the solution of the liposome encapsulating anticancer drug doxorubicin obtained in (3). The obtained solution was then stirred at 25°C for 2 hours and subsequently stirred at 7°C for one night. Then the solution was subjected to ultrafiltration by using an XM300 membrane and a CBS buffer solution (pH 8.5) to obtain 1 ml of liposome with the hydrophylized linker protein (HSA) in which DTSSP was bonded to the HSA on the liposome. 13 mg of tris(hydroxymethyl)aminomethane (Wako Co., Japan) was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. In this manner, 2 ml of the liposome (without bonded sugar) encapsulating the anticancer drug doxorubicin with the hydrophilized linker protein (HSA), in which tris(hydroxymethyl)aminomethane, human serum albumin and liposome were bonded, (total lipid mass: 2 mg, total protein mass: 200 $\mu$g) shown in Fig. 32 was obtained. The suspension of the liposome particle (without bonded sugar) containing anticancer drug doxorubicin in a physiological saline (37°C) thus obtained was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

The liposome prepared in the present example was used in Example 38.

Example 38

Investigation of Leukemia Treatment by Targeting Liposome

(1) Experiments of Cytotoxicity by Doxorubicin HCl Incorporated in Liposome

KG-1a cells established from an acute myeloid leukemia patient were used as human leukemia cells. KG-1a cells were cultured in RPMI 1640 medium containing 10% fetal bovine serum (hereinafter FBS). MRC5 cells established from normal fibroblastic cells were used as normal human cells, and cultured in MEM medium containing 10% FBS. Cell suspensions were prepared from these cells at the concentration of $10^5$ cells/ml and distributed to 96 well plates at $10^4$ cells/well. After 24 hours, free doxorubicin HCl, which was not incorporated in liposome (hereinafter free Dox), doxorubicin HCl incorporated in liposome on which sugar was not bonded (hereinafter L-Dox) and doxorubicin HCl incorporated in liposome on which sialyl Lewis X tetrasaccharide was bonded (hereinafter L-Dox-SLX) were added to each well at appropriate concentrations. At this time the concentration of FBS and the like was adjusted so that all the conditions in each well were the same. Cells were cultured for 72 hours after the addition of the anticancer drug, and surviving cells were quantitated to calculate the killing activities and to obtain 50% growth inhibition concentration (herein after IC50). After removing the media containing the anticancer drug, replacing with 100 $\mu$l of RPMI 1640 or MEM containing 10% WST-8 and 10% FBS and incubating for 3 hours, the quantitation of cells were carried out by measuring absorbance at 485 nm (using the absorbance at 550 nm as a reference).

$$\text{Kiling activity (\%)} = 100 - \text{(absorbance of the well of interest at 485 nm - absorbance of the same well at 550 nm)/(absorbance of the untreated well at 485 nm - absorbance of the untreated well at 550 nm)} \times 100.$$

The killing activity (%) (hereinafter KA) was obtained from the above formula.

The following results were obtained.

IC50 of KG-1 cells for free Dox, L-Dox and L-Dox-SLX were 0.18 $\mu$M, 21.9$\mu$M, and 5.9$\mu$M, respectively. IC50 of NMC5 cells for free Dox, L-Dox and L-Dox-SLX were 8.4 $\mu$M, 520$\mu$M, and 802 $\mu$M, respectively (Table 3).

[Table 3]

| Table 3 | | |
| --- | --- | --- |
| | MRC5 | KG-1a |
| FreeDox | 8.4 | 0.18±0.081 |
| L-Dox | 520 | 21.9±4.83 |
| L-Dox-SLX | 802 | 5.9±1.85 |

Cytotoxicity was represented as 50% growth inhibition concentration (IC50) ($\mu$M).

MRC5 cells were used as normal human cells and KG-1a cells were used as human leukemia cells.

These results revealed the followings.

Incorporation of doxorubicin HCl into the liposome lowered its toxicity to normal cells by about 1/62 in the case of L-Dox and by about 1/95 in the case of L-Dox-SLX. This suggests that the toxicity of the anticancer drug is very much reduced by the incorporation into the liposome. Considering the fact that in many treatment plans currently in use the clinical dose of doxorubicin HCl is 0.4-0.6 mg/Kg per day (0.67-1 $\mu$M) for several days, the IC50 value of over 500 $\mu$M to normal cells appears to offer very high degree of safety.

However, the cell toxicity to leukemia cells was also reduced by the incorporation into the liposome. In KG-1a cells, IC50s of L-dox and free Dox were 21.9 $\mu$M and 0.18 $\mu$M, respectively, indicating the reduction of about 1/120. Since the blood retention time of the liposome formulation was improved compared to free Dox, in the practical *in vivo* administration, the difference in the *in* vivo medicinal effect on KG-1a cells between the free Dox and the L-Dox may not be that great. However, it might be possible that *in vivo* medicinal efficacy of L-Dox may be lower than that of free Dox.

Therefore, we investigated the function of our targeting liposome with sugar chain. In KG-1a cells, IC50 of L-Dox-SLX was 5.9 $\mu$M indicating that the reduction in cytotoxicity was not so great as L-Dox and remained only at the level of about 1/30. Recently, it has been confirmed that leukemia cells including KG-1a cells express L-selectins, and therefore it seems to be possible that the sialyl Lewis X sugar chain bonded to liposome might act as a ligand and bonded to leukemia cells. In MRC5 cells, there was no difference in IC50 value between L-Dox and L-Dox-SLX. This was believed to indicate that L-Dox-SLX were not actively accumulated in MRC5 cells which did not express L-selectin and accumulated only at the same level as L-Dox. It would be expected that the effect of sialyl Lewis X sugar chain would bring about the targeting capacity and regain the cytotoxicity reduced by the incorporation to the liposome.

L-Dox-SLX appears to be the ideal targeting DDS because it possesses a high blood retention and a low cytotoxicity to normal cells due to being incorporated in the liposome, and also it is accumulated actively to leukemia cells through the bonded sugar chain.

(2) Experiments on Enhancement and Inhibition of the Cytotoxicity by Doxorubicin HCl Incorporated in Liposome

The culturing condition by using human leukemia cells KG-1a was the same as in the experiment (1). First, the cytotoxicity of human interferon $\alpha$ (hereinafter IFN) on KG-1a cells was investigated. The KA was obtained in the same way as in (1). Next, the KAs of L-Dox and L-Dox-SLX were obtained in the presence or absence of IFN in the medium and an increase in the KA by IFN was examined. Further, it was investigated whether the KA of L-Dox-SLX in the presence of INF was inhibited or not by the neutralizing antibody to the sugar chain bonding of L-selectin (clone: DREG56). Still further, the KA of the neutralizing antibody itself was measured.

The following results were obtained.

The KA of INF itself to KG-1a cells was 9.4% $\pm$ 7.34 when INF was added to medium at 100 U/ml.

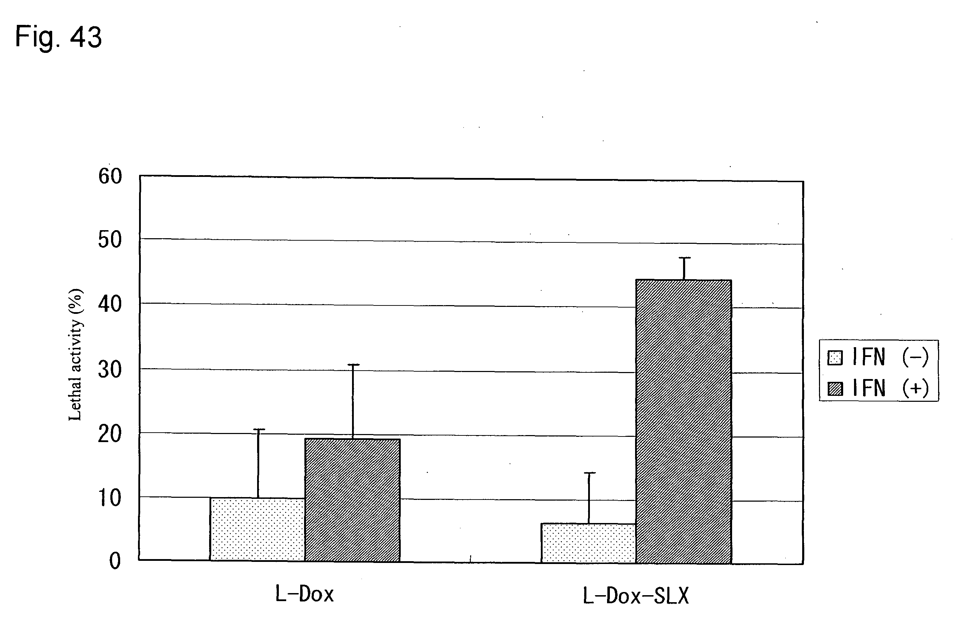

The KAs of L-Dox and L-Dox-SLX were compared in the presence of IFN at 100 U/ml in the medium (Fig. 43). The concentration of doxorubicin HCl in L-Dox and L-Dox-SLX was 17.8 $\mu$M. The KA of L-Dox in the absence of IFN was 10% $\pm$ 10.5 and was increased to only 19.2% $\pm$ 7.99 in the presence of IFN. Considering the fact that the KA of IFN itself was 9.4 %, the increase in the KA of L-Dox in the presence pf IFN was simply an additive. On the other hand, The KA of L-Dox-SLX was 6.3% $\pm$ 11.6 in the absence of IFN and was increased to 44.1% $\pm$ 3.76 in the presence of IFN. The increasing effect was very big and synergic.

A neutralizing antibody to L-selectin was added to the medium at a concentration of 0.3 $\mu$g/ml to measure the KA of the antibody itself. The KA of the antibody at the concentration of 0.3 $\mu$g/ml was 16.3% $\pm$ 25.8.

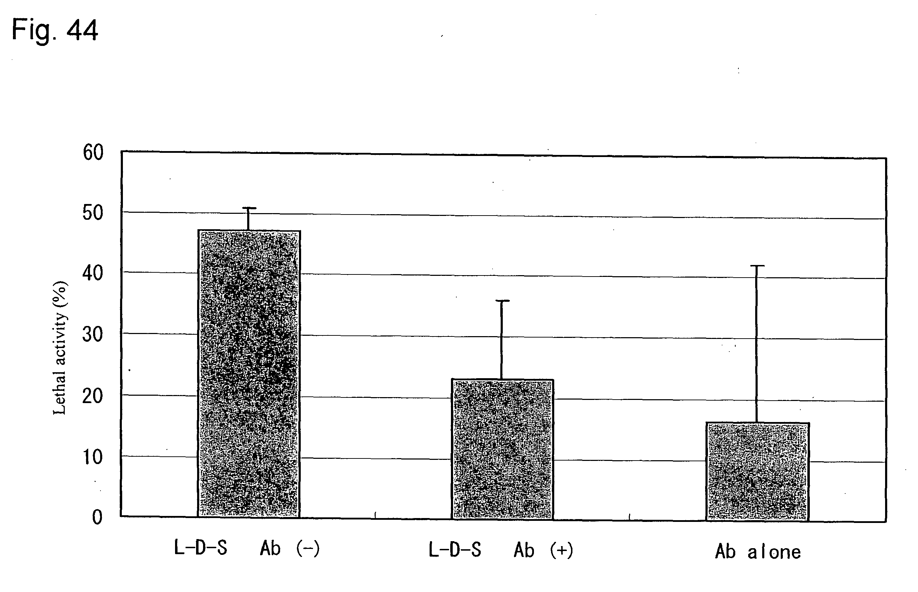

The KA of L-Dox-SLX (the concentration of doxorubicin HCl: 17.8 $\mu$M) in the presence of IFN was 47.2% +3.71. However, addition of the neutralizing antibody reduced the KA to 23.2% + 12.7 (Fig. 44). Considering the KA of the neutralizing antibody itself, the KA of L-Dox-SLX in the presence of INF seemed to be inhibited almost completely.

These results indicate followings.

It has been reported that INF and interferon $\gamma$ and the like stimulate the expression of selectin on leukocytes. It was expected that the stimulation of the selectin expression in human leukemia cells KG-1a, which we used in our experiments, would result in a rise in accumulating ability of L-Dox-SLX in KG-1a cells and an increased cytotoxicity. The experimental results suggest that the KA value of L-Doc without sugar chain in the presence of INF was a simple additive value of the KAs of L-Dox and INF. In contrast, the KA value of L-Dox-SLX was increased synergetically in the presence of INF. This seems to suggest that the prediction described above was correct.

Still further, in the inhibition experiments by the neutralizing antibody against L-selectin, the KA of about 47% was reduced to about 23% by the antibody. Since the KA of the neutralizing antibody itself was about 16%, there was a high probability that the KA of L-Dox-SLX was reduced to a single digit. This seemed to suggest that L-selectin was involved in the expression of the cytotoxicity of L-Dox-SLX. More specifically, it seems that the cytotoxicity of L-Dox-SLX is expressed by the bonding of sialyl Lewis X sugar chain on the liposome to L-selectin on the leukemia cells.

Above results suggest that our DDS has a targeting ability to recognize selectin on human leukemia cells and to accumulate on them and that the sugar chain bonded on the liposome is functioning. Further, the increase in the cytotoxicity of L-Dox-SLX in the presence of IFN and the like is a very interesting phenomenon for considering clinical applications, and it would appear to enhance the potential clinical value of this DDS still more.

Example 39

Investigation of Pharmacokinetics and Carcinostatic Effect in Mice with Cancer

(1) Preparation of Liposome Having Glycolipid Type Sugar Chain and Containing Doxorubicin, and Assay for Encapsulated Drug, and Storage Stability

Liposome was prepared by using the cholate dialysis based method. More specifically, 46.9 mg of sodium cholate was added to 45.6 mg of lipid mixture consisting of dipalmitoylphosphatidylcholine, cholesterol, dicetylphosphate, ganglioside (containing GM1: 13%, GD1a: 38%, GD1b: 9%, GT1b: 16% as glycolipid sugar chain) and at a molar ratio of 35:45:5:15, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffered solution (pH 8.4), and was subjected to a supersonic treatment to obtain 3 ml of a clear micelle suspension. To this micelle suspension, PBS buffer solution (pH 7.2) was added to bring up the volume to 10 ml. Then, anticancer drug doxorubicin, completely dissolved in the TAPS buffer solution (pH 8.4) at 3 mg/ml, was added dropwise slowly to this micelle suspension while stirring. After being mixed homogeneously, this micelle suspension containing doxorubicin was subjected to ultrafiltration by using a PM10 membrane (Amicon Co., USA) and the TAPS buffered saline solution(pH 8.4) to prepare 10 ml of a uniform suspension of the liposome encapsulating anticancer drug doxorubicin. The liposome encapsulating anticancer drug doxorubicin in a physiological saline suspension (37°C) thus obtained was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV. Measuring the amount of encapsulated drug in the liposome with absorbance at 485 nm revealed that doxorubicin was incorporated at the concentration of about 71 μg/ml. This liposome encapsulating doxorubicin was stable in a refrigerator after 1 year storage without precipitating or aggregating.

(2) Measurement of Pharmacokinetics of Liposome Having Glycolipid Type Sugar Chain and Doxorubicin by Tail Vein Administration in Mice with Cancer

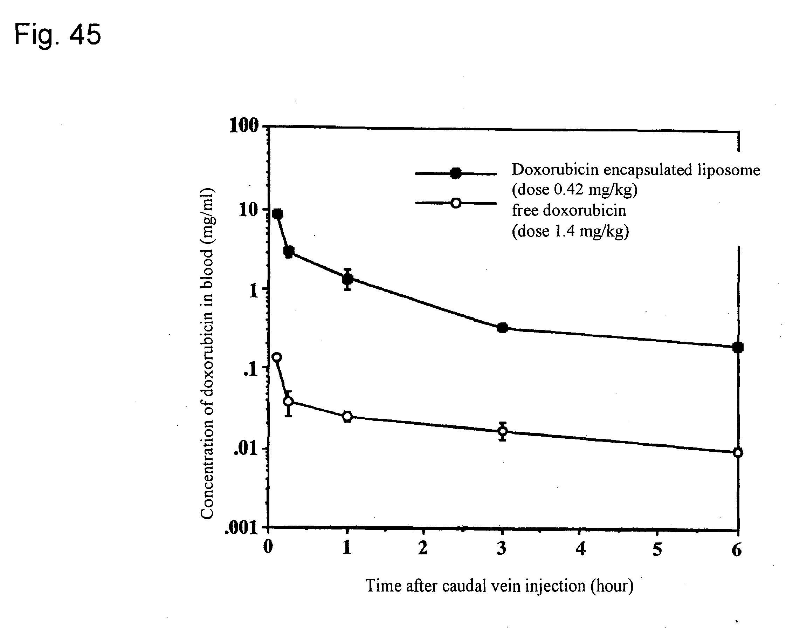

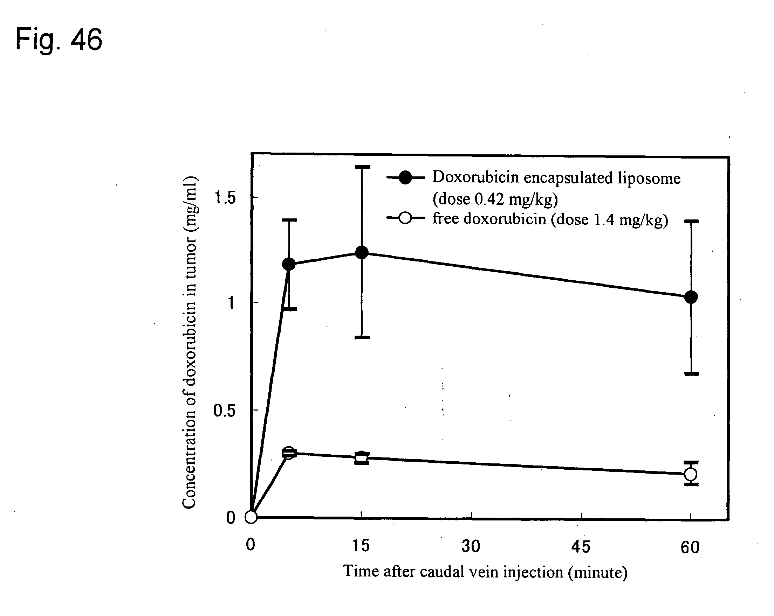

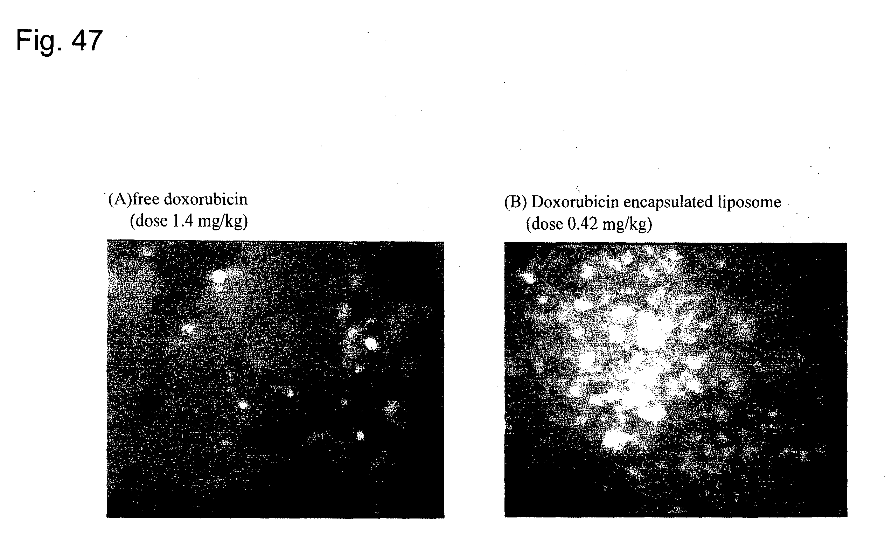

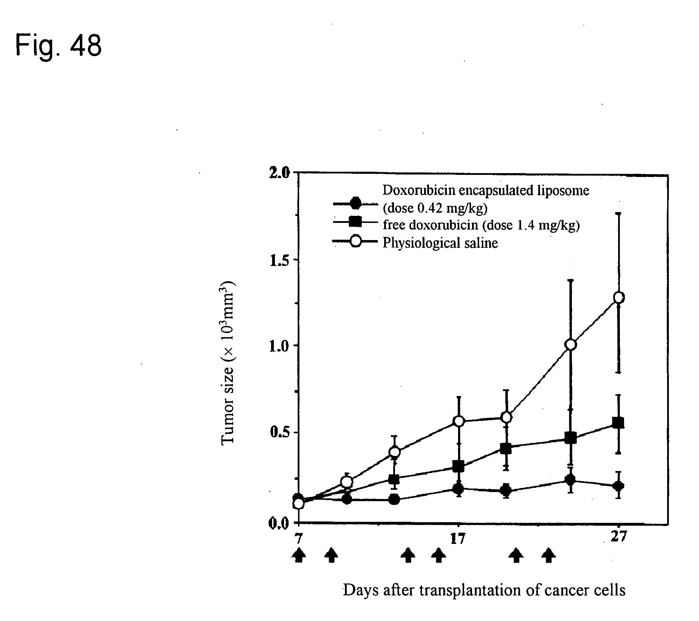

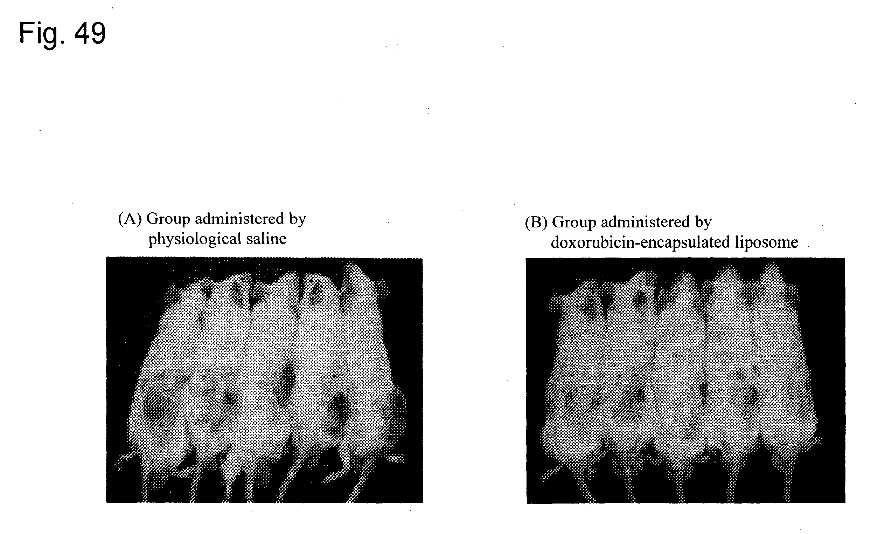

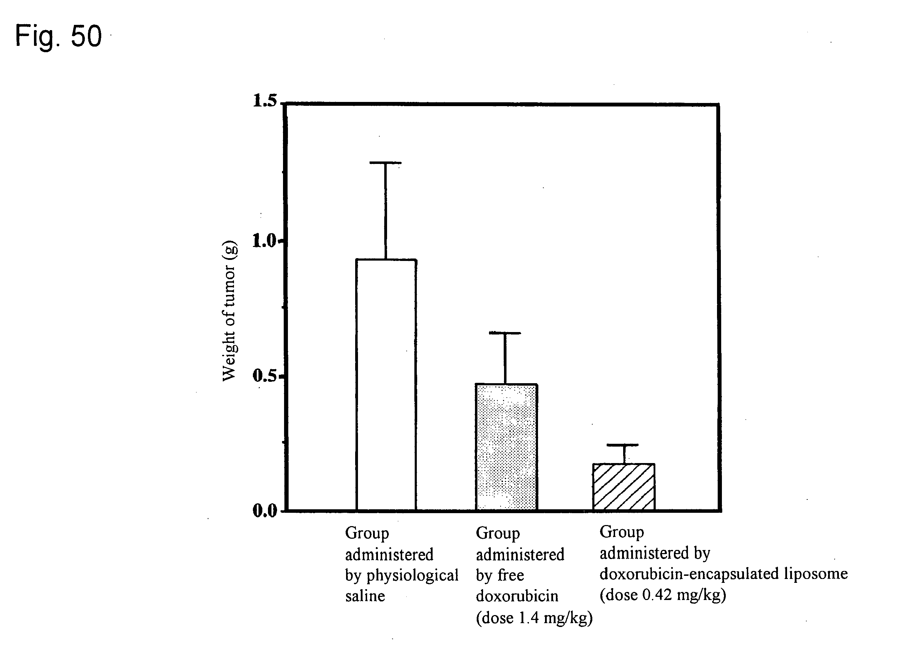

Ehrlich ascites tumor (EAT) cells (about $2 \times 10^7$ cells) were implanted subcutaneously into the right femoral region in male ddY mice (7 weeks of age), and the 50 mice were used in this test after the tumor tissues grew to 50 to 100 cubic mm (after 6 to 8 days).
These mice with cancer were separated into two groups, 25 mice per each group, and the mice in each group were injected with a liposome solution encapsulating having glycolipid type sugar chain and doxorubicin or free doxorubicin solution at a dose of 0.2 ml in tail vein after the tumor implant. Then, at various time intervals, the doxorubicin concentration was measured by fluorescent method (470 nm) in blood and tumor tissue of 5 mice from each group. Also the area under the concentration-time curves (AUC) of the drug in blood was measured and the distribution of doxorubicin in tumor tissues and cells was observed under a fluorescent microscope. As shown clearly in the graphs and photographs in Table 4 and Figs. 45-50, the blood retention of doxorubicin was about 100 times higher and the accumulation of doxorubicin in tumor tissues and cancer cells were about several tens of times higher when the liposome encapsulating doxorubicin of the present invention was compared to the conventional free doxorubicin.

(3) Measurement of Carcinostatic Effect of Liposome Having Glycolipid Type Sugar Chain and Doxorubicin by Tail Vein Administration in Mice with Cancer

Ehrlich ascites tumor (EAT) cells (about $2 \times 10^7$ cells) were implanted subcutaneously into the right femoral region in male ddY mice (7 weeks of age), and the 30 mice were used in this test after the tumor tissues grew to 50 to 100 cubic mm (after 6 to 8 days). These mice with cancer were separated into 3 groups, 10 mice in each group. Mice in each group were injected with liposome having glycolipid type sugar chain and doxorubicin or free doxorubicin solution or physiological saline at a dose of 0.2 ml through tail vein after the tumor implant. The injections were carried out every 3-4 days for total of 6 times. The volume of the cancer was calculated from the following formula by measuring the major axis (L) and the minor axis (S) of transplanted tumor with a vernier caliper:

$$\text{Cancer Volume (cubic mm)} = 1/2 \times L \times S \times S.$$

As shown in the graphs and photographs of Figs. 45-50, by using the liposome encapsulating doxorubicin of the present invention, in comparison with the conventional free doxorubicin, the increase in the tumor volume was inhibited several folds stronger even administering at a low concentration, indicating a marked carcinostatic effect.

[Table 4]

Table 4

| | $AUC_{0\to\infty}$<br>(mg x h/ml) |
|---|---|
| Liposome encapsulating doxorubicin<br>(Amount administered 0.42 mg/kg) | 5.72±0.31 |
| Free doxorubicin<br>(Amount administered 1.4 mg/kg) | 0.13±0.01 |

Example 40

Investigation of Blood Retention of Liposome Treated with 2 Kinds of Hydrophilization

(1) Preparation of Liposome

Liposome was prepared by using the cholate dialysis based method. More specifically, 46.9 mg of sodium cholate was added to 45.6 mg in total of lipid mixture consisting of dipalmitoylphosphatidylcholine, cholesterol, dicetylphosphate, ganglioside and dipalmitoylphosphatidylethanolamine at a molar ratio of 35:40:5:15:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffer solution (pH 8.4), and was subjected to a supersonic treatment to obtain 3 ml of a clear micelle suspension. To this miocelle suspension, PBS buffer solution (pH 7.2) was added to bring up the volume to 5 ml. Then, predonisolone phosphate, completely dissolved in the TAPS buffer solution (pH 8.4) at 2250 mg/6 ml, was added dropwise slowly to this micelle suspension while stirring. After being mixed homogeneously, this micelle suspension containing predonisolone phosphate was subjected to ultrafiltration by using a PM10 membrane (Amicon Co., USA) and the TAPS buffer solution (pH 8.4) to prepare 10 ml of a uniform liposome. The liposome particles in the resulting physiological saline suspension (37°C) was subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

(2) Hydrophilization of Lipid Membrane Surface of Liposomes by Tris(hydroxymethyl)aminomethane

10 ml of the liposome solution prepared in (1) was subjected to ultrafiltration by using an XM300 membrane (Amicon Co., USA) and a CBS buffer solution (pH 8.5) to adjust the pH of the solution to 8.5. Then, 10 ml of bis (sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the chemical bonding reaction between the dipalmitoylphosphatidyletanolamine of the lipid on the liposome membrane and the BS3. This liposome solution was then subjected to ultrafiltration by using an XM300 membrane and a CBS buffer solution (pH 8.5). Then, 40 mg of tris(hydroxymethyl)aminomethane dissolved in 1 ml of CBS buffer solution (pH 8.5) was added to 10 ml of the liposome solution. The obtained solution was stirred at 25°C for 2 hours, and stirred at 7°C for one night to complete the chemical bonding reaction between the BS3 bonded to the lipid on the liposome membrane and the tris (hydroxymethyl)aminomethane. In this manner, the hydroxyl groups of the tris(hydroxymethyl)aminomethane were coordinated on the dipalmitoylphosphatidyletanolamine of the lipid on the liposome membrane to achieve the hydrophilization of the lipid membrane surface of the liposome.

(3) Hydrophilization by Cellobiose on Lipid Membrane Surface of Liposome

10 ml of the liposome solution prepared in (1) was subjected to ultrafiltration by using an XM300 membrane (Amicon Co., USA) and a CBS buffer solution (pH 8.5) to adjust the pH of the solution to 8.5. Then, 10 ml of bis (sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the chemical bonding reaction between the dipalmitoylphosphatidyletanolamine of the lipid on the liposome membrane and the BS3. This liposome solution was then subjected to ultrafiltration by using an XM300 membrane and a CBS buffer solution (pH 8.5). Then, 50 mg of cellobiose dissolved in 1 ml of CBS buffer solution (pH 8.5) was added to 10 ml of the liposome solution. The obtained solution was stirred at 25°C for 2 hours, and stirred at 7°C for one night to complete the chemical bonding reaction between the BS3 bonded to the lipid on the liposome membrane and cellobiose. In this manner, the hydroxyl groups of cellobiose were coordinated on the dipalmitoylphosphatidyletanolamine of the lipid on the liposome membrane to achieve the hydrophilization of the lipid membrane surface of the liposome.

(4) Bonding of Human Serum Albumin (HSA) to Tris(hydroxymethyl)aminomethane Bonded on Liposome Membrane Surface

A coupling reaction method based on a previously reported method (Yamazaki, N., Kodama, M. and Gabius, H. -J. (1994) Methods Enzymol. 242, 56-65) was used. More specifically, the reaction was carried out through a two-stage chemical reaction. That is, 43 mg of sodium metaperiodate dissolved in 1 ml of TAPS buffer solution (pH 8.4) was added to 10 ml of the liposome obtained in (2), and the obtained solution was stirred at room temperature for 2 hours to periodate-oxidize the ganglioside on the membrane surface of the liposome. Then, the solution was subjected to ultrafiltration by using an XM300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 $\mu$l of 2M $NaBH_3CN$ was added to the PBS buffer solution (pH 8.0), and the obtained solution was stirred at 10°C for one night to bond the HSA to the liposome membrane surface through a coupling reaction between the HSA and the ganglioside on the liposome. Then, 10 ml of HSA-bonded liposome solution was obtained through an ultrafiltration using an XM300 membrane and a CBS buffer solution (pH 8.5).

(5) Bonding of Human Serum Albumin (HSA) to Cellobiose Bonded on Liposome Membrane Surface

A coupling reaction method based on a previously reported method (Yamazaki, N., Kodama, M. and Gabius, H. -J. (1994) Methods Enzymol. 242, 56-65) was used. More specifically, the reaction was carried out through a two-stage chemical reaction. That is, 43 mg of sodium metaperiodate dissolved in 1 ml of TAPS buffer solution (pH 8.4) was added to 10 ml of the liposome obtained in Example 3, and the obtained solution was stirred at room temperature for 2 hours to periodate-oxidize the ganglioside on the membrane surface of the liposome. Then, the solution was subjected to ultrafiltration by using an XM300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100 $\mu$l of 2M $NaBH_3CN$ was added to the PBS (pH 8.0), and the obtained solution was stirred at 10°C for one night to bond the HSA to the liposome membrane surface through a coupling reaction between the HSA and the ganglioside on the liposome. Then, 10 ml of HSA-bonded liposome solution was obtained through an ultrafiltration using an XM300 membrane and a CBS buffer solution (pH 8.5).

(6) Hydrophilization of Linker Protein (HSA) by Bonding of Tris(hydroxymethyl)aminomethane on Human Serum Albumin (HSA) bonded to Liposome Membrane Surface

To prepare liposome as one of the hydrophilized samples, 1 mg of 3,3'-dithiobis(sulfosuccinimidyl) propionate (DTSSP; Pierce Co., USA) serving as a crosslinking reagent was added to 1 ml of a part of the liposome solution obtained in (4). The obtained solution was then stirred at 25°C for 2 hours, and subsequently stirred at 7°C for one night. Then, the solution was subjected to ultrafiltration by using an XM300 membrane and a CBS buffer solution (pH 8.5) to obtain 1 ml of hydrophilized liposome in which the DTSSP was bonded to the HSA on the liposome. 13 mg of tris(hydroxymethyl) aminomethane (Wako Co., Japan) was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. In this manner, 2 ml of the liposome with the hydrophilized linker protein (HSA), in which tris(hydroxymethyl)aminomethane, human serum albumin and liposome were bonded, (total lipid mass: 2 mg, total protein mass: 200 μg) was obtained as a comparative sample. The liposome particles in the resulting physiological saline suspension (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

(7) Hydrophilization of Linker Protein (HSA) by Bonding of Cellobiose on Human Serum Albumin (HSA) bonded to Liposome Membrane Surface

To prepare liposome as one of the hydrophilized samples, 1 mg of 3,3'-dithiobis(sulfosuccinimidyl) propionate (DTSSP; Pierce Co., USA) serving as a crosslinking reagent was added to 1 ml of a part of the liposome solution obtained in (5). The obtained solution was then stirred at 25°C for 2 hours, and subsequently stirred at 7°C for one night. Then, the solution was subjected to ultrafiltration by using an XM300 membrane and a CBS buffer solution (pH 8.5) to obtain 1 ml of hydrophilized liposome in which the DTSSP was bonded to the HSA on the liposome. 30 mg of cellobiose was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond cellobiose to the DTSSP on the human serum albumin bonded on the liposome membrane surface. In this manner, 2 ml of the liposome with the hydrophilized linker protein (HSA), in which cellobiose, human serum albumin and liposome were bonded, (total lipid mass: 2 mg, total protein mass: 200 μg) was obtained as a hydrophilized sample. The liposome particles in the resulting physiological saline solution (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

(8) Preparation of Liposome without Hydrophilization

Liposome was prepared by using the cholate dialysis based method. More specifically, 46.9 mg of sodium cholate was added to 45.6 mg of lipid mixture consisting of dipalmitoylphosphatidylcholine, cholesterol, dicetylphosphate and ganglioside (containing 100% of GT1b as glycolipid sugar chain) at a molar ratio of 35:45:5:15, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffer solution (pH 8.4), and was subjected to a supersonic treatment to obtain 3 ml of a clear micelle suspension. Then, to this micelle suspension, PBS buffer solution (pH 7.2) was added to bring up the volume to 10 ml, and doxorubicin, completely dissolved in the TAPS buffer solution (pH 8.4) at 3 mg/l ml, was added dropwise slowly to this micelle suspension while stirring. After being mixed homogeneously, this micelle suspension containing doxorubicin was subjected to ultrafiltration by using a PM10 membrane (Amicon Co., USA) and the TAPS buffer solution (pH 8.4) to prepare 10 ml of a uniform suspension of the liposome particles without hydrophilization. The liposome particles without hydrophilization in the resulting physiological saline suspension (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

(9) $^{125}$I-Labeling of Liposome through the Chloramine T Method

A chloramine T (Wako Pure Chemical Co., Japan) solution and a sodium disulfite solution were prepared at 3 mg/ml and 5 mg/ml, respectively. 50 μl of the 3 different types of liposomes prepared in Examples 6 to 8 were put into separate Eppendorf tubes. Then, 15 μl of $^{125}$I-NaI (NEN Life Science Product, Inc. USA) and 10 μl of chloramine T solution were added thereto and reacted therewith. 10 μl of chloramine T solution was added to the respective solutions every 5 minutes. After 15 minutes from the completion of the above procedure repeated twice, 100 μl of sodium disulfite serving as a reducer was added to the solutions to stop the reaction. Then, each of the resulting solutions was placed on a Sephadex G-50 (Phramacia Biotech. Sweden) column chromatography, and eluted by PBS to purify a labeled compound. Finally, a non-labeled liposome complex was added to each of the solutions to adjust a specific activity (4 × 10$^6$ Bq/mg protein). In this manner, three types of $^{125}$I-labeled liposome solutions were obtained.

(10) Measurement of the Concentration of Different Types of Liposome in Blood Stream of Mice with Cancer

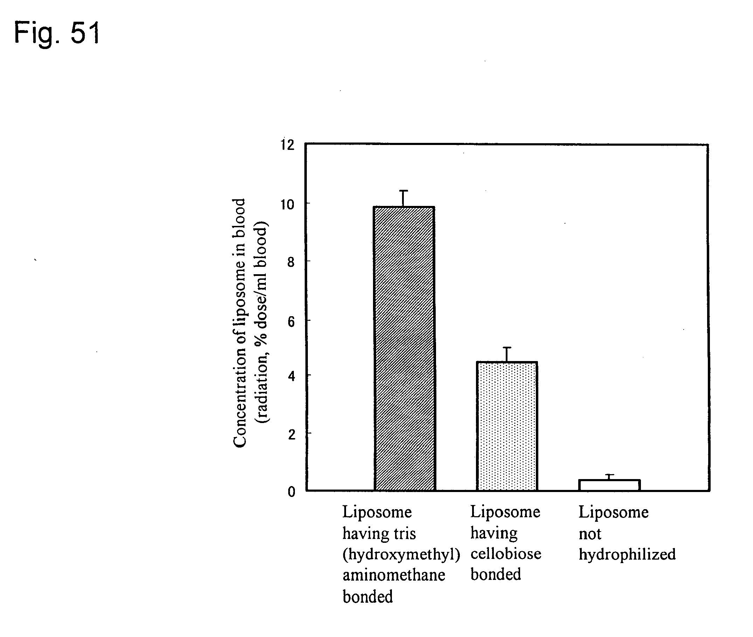

Ehrlich ascites tumor (EAT) cells (about 2 × 10$^7$ cells) were implanted subcutaneously into the femoral region in male ddY mice (7 weeks of age), and the mice were used in this test after the tumor tissues grew to 0.3 to 0.6 g (after 6 to 8 days). To this mice with cancer, 0.2 ml of three types of liposome complex labeled with $^{125}$I according to (9) were injected at a dose of 30 μg/mouse as lipid amount through tail vein. The blood sample was collected after 5 minutes and its radioactivity was measured with a gamma counter (Aloka ARC 300). Distribution of the radioactivity to the blood was expressed as a ratio of the radioactivity per 1 ml of blood to the total radioactivity administered (% administered amount/ml of blood). As shown in Fig. 51, blood retention was increased by the two types of hydrophilization, and among them the liposome hydrophilized with tris(hydroxymethyl)aminomethane had a marked increase in blood retention.

Example 41

Preparation of Liposome Encapsulating Vitamin A and Storage Stability

Liposome was prepared by using the cholate dialysis based method. More specifically, 46.9 mg of sodium cholate was added to 45.6 mg in total of lipid mixture consisting of dipalmitoylphosphatidylcholine, cholesterol, dicetylphosphate, ganglioside, sphingomyelin and dipalmitoylphosphatidylethanolamine at a molar ratio of 35:40:5:5:10:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a PBS buffer solution (pH 7.2), and was subjected to a supersonic treatment to obtain 3 ml of a clear micelle suspension. Then, PBS buffer solution (pH 7.2) was added to bring up the volume to 10 ml, and 6 mg/ml of vitamin A, completely dissolved in 0.3 ml of ethanol and 0.7 ml of PBS buffer solution (pH 7.2), was added dropwise slowly to this micelle suspension while stirring. After being mixed homogeneously, this micelle suspension containing vitamin A was subjected to ultrafiltration by using a PM10 membrane (Amicon Co., USA) and the PBS buffer solution (pH 7.2) to prepare 10 ml of a uniform liposome encapsulating vitamin A (average particle size: 100 nm) suspension. This liposome encapsulating vitamin A was stable without precipitation or coagulation after storing 1 year in a refrigerator.

Example 42

Preparation of Liposome Encapsulating Vitamin E and Storage Stability

Liposome was prepared by using the cholate dialysis based method. More specifically, 46.9 mg of sodium cholate was added to 45.6 mg in total of lipid mixture consisting of dipalmitoylphosphatidylcholine, cholesterol, dicetylphosphate, ganglioside, sphingomyelin and dipalmitoylphosphatidylethanolamine at a molar ratio of 35:40:5:5:10:5, respectively, 6 mg of vitamin E was added and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a PBS bugger solution (pH 7.2), and was subjected to a supersonic treatment to obtain 3 ml of a clear micelle suspension. Then, PBS buffer solution (pH 7.2) was slowly added to this micelle suspension while stirring to bring up the volume to 10 ml, and 6 mg/ml of vitamin A, completely dissolved in 0.3 ml ethanol and 0.7 ml of PBS buffer solution (pH 7.2), was added dropwise slowly to the suspension. After being mixed homogeneously, this micelle suspension containing vitamin A was subjected to ultrafiltration by using a PM10 membrane (Amicon Co., USA) and the PBS buffer solution (pH 7.2) to prepare 10 ml of a uniform liposome encapsulating vitamin A (average particle size: 100 nm) suspension. This liposome encapsulating vitamin E was stable without precipitation or coagulation after storing 1 year in a refrigerator.

Example 42

Preparation of Liposome Encapsulating Predonisolone Phosphate

(1) Preparation of Liposome Eucapsulating Predonisolone Phosphate, Quantitative Determination for Eucapsulated Drug and Storage Stability

Liposome was prepared by using the cholate dialysis based method. More specifically, 46.9 mg of sodium cholate was added to 45.6 mg in total of lipid mixture consisting of dipalmitoylphosphatidylcholine, cholesterol, dicetylphosphate, ganglioside and dipalmitoylphosphatidylethanolamine at a molar ratio of 35:40:5:15:5, respectively, and the lipid mixture was dissolved in 3 ml of chloroform/methanol solution. The solution was then evaporated, and the resulting deposit was dried in vacuo to obtain a lipid membrane. The obtained lipid membrane was suspended in 3 ml of a TAPS buffered saline solution (pH 8.4), and was subjected to a supersonic treatment to obtain 3 ml of a clear micelle suspension. To this miocelle suspension, PBS buffer solution (pH 7.2) was added to bring up the volume to 5 ml. Then, predonisolone phosphate, completely dissolved in the TAPS buffer solution (pH 8.4) at 2250 mg/6 ml, was added dropwise slowly to this micelle suspension while stirring. After being mixed homogeneously, this micelle suspension containing predonisolone phosphate was subjected to ultrafiltration by using a PM10 membrane (Amicon Co., USA) and the TAPS buffer solution(pH 8.4) to prepare 10 ml of a uniform suspension of the liposome encapsulating predonisolone phosphate suspension. The liposome particles encapsulating prednisolove phosphate in the resulting physiological saline suspension (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV. The measurement of encapsulated drug in the liposome with 260 nm absorbance revealed that predonisolone phosphate was incorporated at the concentration of about 280 $\mu$g/ml. This liposome encapsulating predonisolone phosphate was stable without precipitation or coagulation after storing one year in a refrigerator.

(2) Hydrophilization of Lipid Membrane Surface of Liposomes Containing Predonisolone Phosphate

10 ml of the liposome encapsulating predonisolone phosphate solution prepared in (1) was subjected to ultrafiltration by using an XM300 membrane (Amicon Co., USA) and a CBS buffer solution (pH 8.5) to adjust the pH of the solution to 8.5. Then, 10 ml of bis (sulfosuccinimidyl) suberate (BS3; Pierce Co., USA) crosslinking reagent was added to the liposome solution. The obtained solution was stirred at 25°C for 2 hours, and subsequently stirred at 7°C for one night to complete the chemical bonding reaction between the BS3 and the dipalmitoylphosphatidyletanolamine of the lipid on the liposome membrane. This liposome solution was then subjected to ultrafiltration by using an XM300 membrane and a CBS buffer solution (pH 8.5). Then, 40 mg of tris(hydroxymethyl)aminomethane dissolved in 1 ml of CBS buffer solution (pH 8.5) was added to 10 ml of the liposome solution. The obtained solution was stirred at 25°C for 2 hours, and stirred at 7°C for one night to complete the chemical bonding reaction between the BS3 bonded to the lipid on the liposome membrane and the tris(hydroxymethyl)aminomethane. In this manner, the hydroxyl groups of the tris(hydroxymethyl)aminomethane were coordinated on the lipid dipalmitoylphosphatidyletanolamine of the liposome membrane encapsulating the anticancer drug doxorubicin to achieve the hydrophilization thereof.

(3) Bonding of Human Serum Albumin (HAS) to Membrane Surface of Liposome Encapsulating Predonisolone Phosphate

A coupling reaction method based on a previously reported method (Yamazaki, N., Kodama, M. and Gabius, H. -J. (1994) Methods Enzymol. 242, 56-65) was used. More specifically, the reaction was carried out through a two-stage chemical reaction. That is, first, 43 mg of sodium metaperiodate dissolved in 1 ml of TAPS buffer solution (pH 8.4) was added to 10 ml of the gauglioside on the membrane surface of the liposome obtained in (2), and the obtained solution was stirred at room temperature for 2 hours to periodate-oxidize the ganglioside on the membrane surface of the liposome. Then, the solution was subjected to ultrafiltration by using an XM300 membrane and a PBS buffer solution (pH 8.0) to obtain 10 ml of oxidized liposome. 20 mg of human serum albumin (HSA) was then added to the liposome solution, and the obtained solution was stirred at 25°C for 2 hours. Then, 100$\mu$l of 2M NaBH$_3$CN was added to the PBS (pH 8.0), and the obtained solution was stirred at 10°C for one night to bond the HSA to the liposome membrane surface through a coupling reaction between the ganglioside on the liposome and the HSA. Then, 10 ml of HSA-bonded liposome encapsulating predonisolone phosphate solution was obtained through an ultrafiltration using an XM300 membrane and a CBS buffer solution (pH 8.5).

(4) Bonding of Sialyl Lewis X tetrasaccharide to Human Serum Albumin (HSA) Bonded on Membrane Surface of Liposome Containing Predonisolone Phosphate and Hydrophilization of Linker Protein (HSA)

50 $\mu$g of sialyl Lewis X tetrasaccharide (Calbiochem Co., USA) was added to 0.5 ml water solution having 0.25 g of NH$_4$CO$_3$, and the obtained solution was stirred at 37°C for 3 days. Then, the solution was filtered by using a 0.45 $\mu$m filter to complete an amination at the reduced terminal of the sugar chain and obtain 50 $\mu$g of glycosylamine compound of sialyl Lewis X tetrasaccharide. Then, 1 mg of 3, 3'-dithiobis (sulfosuccinimidyl) propionate (DTSSP; Pierce Co., USA) serving as a crosslinking reagent was added to 1 ml of a part of the solution of the liposome encapsulating predonisolone phosphate obtained in (3). The obtained solution was then stirred at 25°C for 2 hours and subsequently stirred at 7°C for one night. Then the solution was subjected to ultrafiltration by using an XM300 membrane and a CBS buffer solution (pH 8.5) to obtain 1 ml of liposome in which DTSPP was bonded to the HSA on the liposome. Then, 50$\mu$g of the glycosylamine compound of sialyl Lewis X tetrasaccharide was added to the liposome solution. The obtained solution was stirred at 25°C for 2 hours, and subsequently stirred at 7°C for one night. Then, the solution was subjected to ultrafiltration by using an XM300 membrane and a PBS buffer solution (ph 7.2) to bond the sialyl Lewis X tetrasaccharide to the DTSSP on the human serum albumin bonded on the liposome membrane surface. 13 mg oftris(hydroxymethyl) aminomethane (Wako Co., Japan) was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. As the result, the liposome with the hydrophilized linker protein (HSA), in which tris(hydroxymethyl)aminomethane, human serum albumin and liposome were bonded, was obtained. In this manner, 2 ml of the liposome encapsulating predonisolone phosphate with the hydrophilized linker protein (HAS), in which sialyl Lewis X tetrasaccharide, human serum albumin and liposome were bonded, (total lipid mass: 2 mg, total protein mass: 200 μg) was obtained. The liposome particles encapsulating prednisolone phosphate in the resulting physiological saline suspension (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.

(5) Hydrophilization of Linker Protein (HAS) by the Bonding of Tris(hydroxymethyl)aminomethane to Human Serum Albumin (HSA) Bonded on Membrane Surfaces of Liposome Encapsulating Predonisolone Phosphate

To prepare liposome encapsulating predonisolone phosphate as a comparative sample (without sugar chain), 1 mg of 3, 3'-dithiobis (sulfosuccinimidyl) propionate (DTSSP; Pierce Co., USA) serving as a crosslinking reagent was added to 1 ml of a part of the solution of the liposome encapsulating predonisolone phosphate obtained in (3). The obtained solution was then stirred at 25°C for 2 hours and subsequently stirred at 7°C for one night. Then the solution was subjected to ultrafiltration by using an XM300 membrane and a CBS buffer solution (pH 8.5) to obtain 1 ml of liposome with the hydrophylized linker protein (HSA) in which DTSPP was bonded to the HSA on the liposome. 13 mg of tris(hydroxymethyl)aminomethane (Wako Co., Japan) was added to this liposome solution. The obtained solution was stirred at 25°C for 2 hours, and subsequently stirred at 7°C for one night. The solutions were then subjected to ultrafiltration by using an XM300 membrane and a PBS buffer solution (pH 7.2) to bond tris(hydroxymethyl)aminomethane to the DTSSP on the human serum albumin bonded on the liposome membrane surface. In this manner, 2 ml of the liposome encapsulating predonisolone phosphate (without sugar chain) with the hydrophilized linker protein (HSA), in which tris (hydroxymethyl)aminomethane, human serum albumin and liposome were bonded, (total lipid mass: 2 mg, total protein mass: 200 μg) was obtained. The liposome particles encapsulating predonisolone phosphate (without sugar chain) in the resulting physiological saline suspension (37°C) were subjected to analysis for the particle diameter and zeta potential using a measuring device for zeta potential, particle diameter and molecular weight (Model Nano ZS, Malvern Instruments Ltd., UK) and the particle diameter was 50 to 350 nm and the zeta potential was -30 to -10 mV.
The liposomes prepared in the present Example were used in the investigations of Examples 15 and 16 below.

Example 43

Investigation of Rheumatic Arthritis Treatment by Targeting Liposome

(1) Preparation of RA Model Mice

Collagen type II induced arthritis (hereinafter CIA) model was prepared as a RA mouse model. The mice, reagents and the like used in the investigation are described as below.
Experimental animal: DBA/1J mouse (8 weeks of age, male)
Antigen inoculated: Bovine collagen type II
Adjuvant: Complete Freund's adjuvant containing killed M. tuberculosis (H37Ra, 2 mg/ml)(CFA) and incomplete Freund's adjuvant without M. tuberculosis (IFA)
An emulsion containing collagen at 200 μg/100 μl was prepared by mixing the collagen solution with CFA at the volume ratio of 2:1 and was injected to mice subcutaneously at the bottom of the tail (Day 0). After 21 days of the treatment (Day 21), an emulsion containing collagen at 200 μg/100 μl was prepared by mixing the collagen solution with IFA at the volume ratio of 2:1 1 and was injected again to the mice subcutaneously at the bottom of the tail.
After the treatment, the limbs of mice were inspected at a frequency of 3 times/week, and the inflammation was quantified by converting to the points according to the following standard. In each limb, normal: 0 point, light inflammation or flare: 1 point, severe flare or swelling or restricted use of limb: 2 points, deformity in palms, soles and joints: 3 points (the lowest is 0 point and the highest is 12 points). The points were processed by the following formula to obtain Inflammatory Activity (hereinafter IA).

$$\text{Inflammatory Activity (\%)} = \text{Total points of the 4 limbs}/12 \times 100.$$

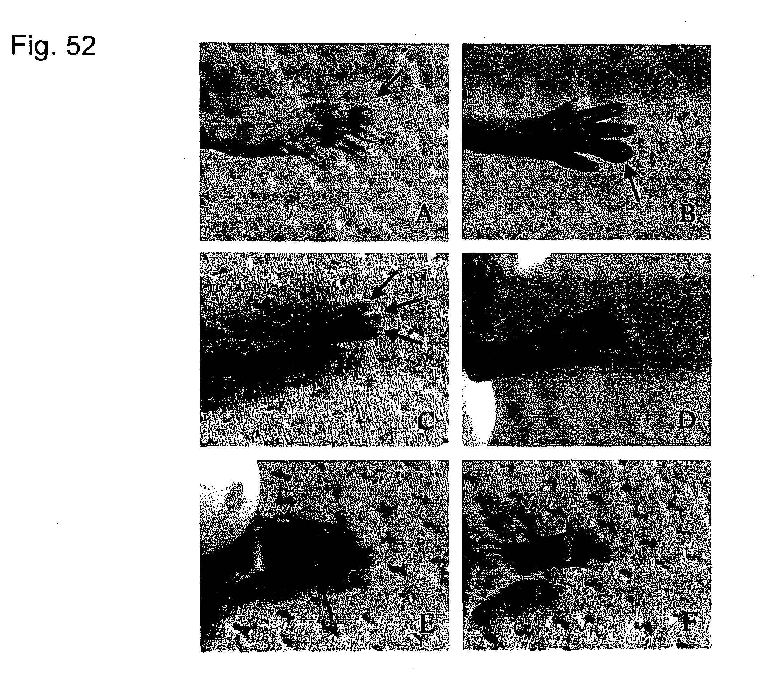

The following results were obtained.
After a few days from Day 21, the number of the mice, in which the flare was confirmed in the fingers of the hind leg, was increased. On Day 28, the IA of mice was 16.7%, and reached 50% on Day 39. Fig. 52 shows photographs of the mouse limbs having inflammation. Observations of the CIA mouse arthritis are as follows. A: swelling of the finger joint of the hind limb (the second digit, arrow), B: swelling of the finger joint of the hind limb (the 4th digit, the arrow), C: flare of the finger joint of the hind limb (arrow), D: normal hind limb, E: swelling of the hand joint of the forelimb (arrow), F: normal fore limb

In this manner, CIA mice were produced as RA mice model according to the method described above.

(2) Experiments for treating RA mice by intravenous administration of DDS

The mice with arthritis were treated by injecting therapeutic drug through the tail vein. The intravenous injections were carried out during Day 28 to Day 46 at the frequency of twice a week.

On Day 28, mice with the inflammation symptom were chosen and divided into 4 groups so that the average of the inflammation points in each group was the same. The 4 groups were: 1) Control: no treatment, 2) free Pred: predonisolone phosphate was dissolved in a physiological saline solution and the mice in this group were injected intravenously with this solution at the dose of 100 $\mu$g per injection and 200 $\mu$g per week, 3) L-Pred: liposome encapsulating predonisolone phosphate without sugar chain was used, and the mice in this group were injected intravenously with this liposome at the dose of 10 $\mu$g per injection and 20 $\mu$g per week, 4) L-Pred-SLX: liposome encapsulating predonisolone phosphate with bonded sialyl Lewis X sugar chain was used, and the mice in this group were injected intravenously with this liposome at the dose of 10 $\mu$g per injection and 20 $\mu$g per week.

The number of the mice in these 4 groups was: Control: 6 mice, free Pred: 7 mice, L-Pred: 8 mice, L-Pred-SLX: 8 mice.

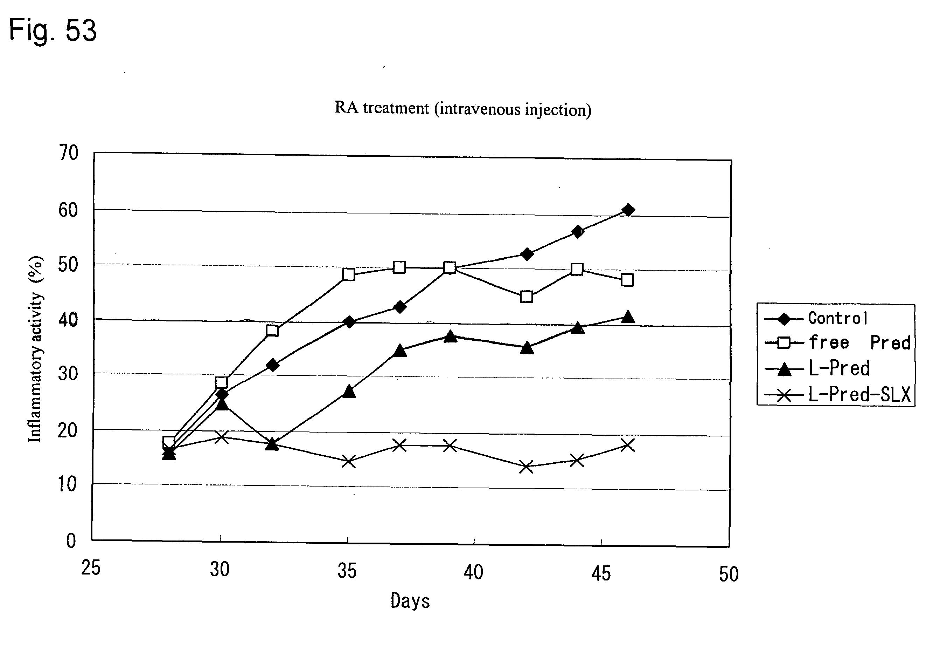

The following results were obtained. In control, IA was increased along with time, and was over 60% on Day 46. In free Pred, IA was about 50% on Day 37 and maintained the equilibrium afterward. In L-Pred, IA started rising from Day 32 and was about 40% on Day 46. In L-Pred-SLX, no big increase in IA was observed, and IA was 20% or below all through the course (Fig. 53).

These results indicate the followings.

In free Pred group, the dose of intravenously injected predonisolone phosphate at 100 $\mu$g/injection was not enough to control the inflammation, and there was no clear difference from the Control group in IA. In L-Pred group, the intravenously injected dose of predonisolone phosphate, 10 $\mu$g/injection, was one tenth of that injected in the Control group but IA was at the same level as in the free Pred group or below. It would appear that the medicinal effect of predonisolone phosphate may be improved due to better blood retention caused by incorporating predonisolone phosphate in liposome. In L-Pred-SLX group, the dose of predonisolone phosphate at 10 $\mu$g/injection was similarly one tenth of that in free Pred group but IA was not increased at all and the progression of the inflammation was suppressed significantly. It seemed that the difference between L-Pred and L-Pred-SLX was brought about by the effect of the presence of sialyl Lewis X sugar chain. More specifically, it seemed to suggest that the liposome was accumulated to the inflamed area effectively by sialyl Lewis X sugar chain.

This DDS was proven to be capable of delivering a therapeutic drug to inflamed area very effectively. The possibility was suggested that by accumulating a therapeutic drug to a lesion site, the side effects of the drug may be suppressed or the medicinal effect of the therapeutic drug may be enhanced.

(3) Experiments of Treating RA Mice by Oral Administration of DDS

Mice with arthritis were treated by oral administration of therapeutic drugs. Drugs were administered from Day 28 to Day 39 at a frequency of 3 times a week.

On Day 28, mice with the inflammation symptom were chosen and divided into 4 groups so that the average of the inflammation points in each group was the same. The 4 groups were: 1) Control: no treatment, 2) free Pred: predonisolone phosphate was dissolved in a physiological saline solution and the mice in this group were administered orally with this solution at the dose of 100 $\mu$g per administration and 300 $\mu$g per week, 3) L-Pred: liposome encapsulating predonisolone phosphate without sugar chain was used, and the mice in this group were administered orally with this liposome at the dose of 10 $\mu$g per administration and 30 $\mu$g per week, 4) L-Pred-SLX: liposome encapsulating predonisolone phosphate with bonded sialyl Lewis X sugar chain was used, and the mice in this group were administered orally with this liposome at the dose of 10 $\mu$g per administration and 30 $\mu$g per week.

The number of the mice in these 4 groups was: Control: 2 mice, free Pred: 3 mice, L-Pred: 3 mice, L-Pred-SLX: 4 mice.

The following results were obtained.

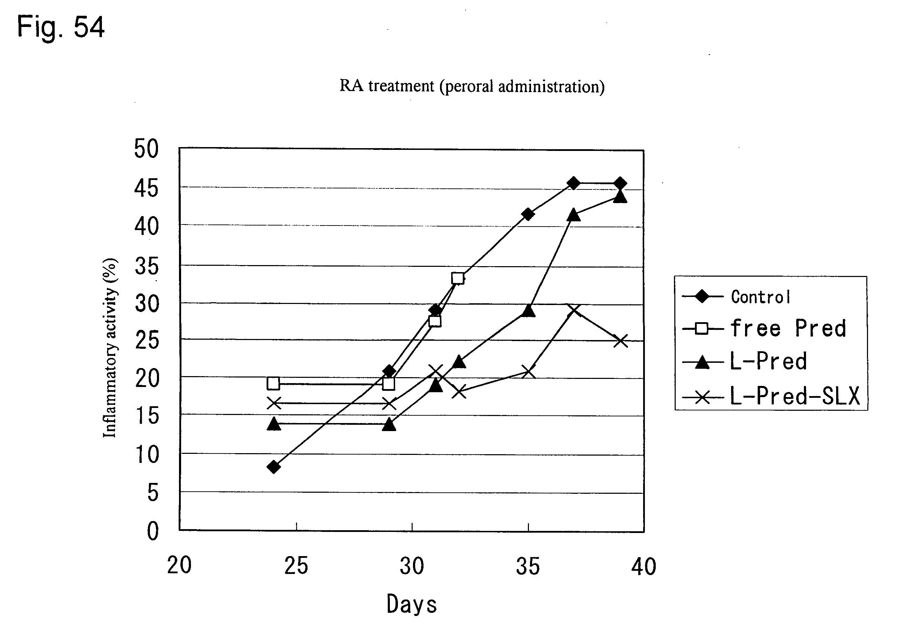

The mice in free Fred group were killed due to gastric perforation by a feeding needle and the experiment was terminated on Day 32. There were no significant difference in IA among the groups of Control, free Pred (up to Day 32) and L-Pred. The progression of inflammation could not be suppressed in L-Pred-SLX group but IA in this group was significantly lower than that in L-Pred on Day 39 (Fig. 54).

These results indicate the followings.

No significant difference was observed among 3 groups of Control, free Pred and L-Pred. It is speculated that in the free Pred group the amount of orally administered predonisolone phosphate, 300 $\mu$g per week, was not enough to suppress the progression of inflammation. Also, in L-Pred group, the dose may be insufficient because predonisolone phosphate may not be absorbed in the intestinal tract, or even if it was absorbed, the dose of 30 $\mu$g/week of predonisolone phosphate was too low. On the other hand, in L-Pred-SLX group, IA was suppressed. This seems to indicate that the liposome was absorbed in the intestinal tract, and considering that the orally administered dose of predonisolone phosphate was only 30 $\mu$g/week, there was a possibility that predonisolone phosphate was delivered to the inflammation lesion in the intact liposome with sugar chain.

The results suggest that this DDS functions efficiently, not only by intravenous administration but also by oral administration. More specifically, it is suggested that by incorporating in liposome, drugs, which could not be absorbed orally in the past, may be made into formulations suitable for oral administration.

(4) Experiments for determining the suitable dose of predonisolone phosphate in the treatment of RA mice.

Mice with arthritis were treated by injecting therapeutic drugs in the tail vein. Intravenous injections were carried out from Day 28 to Day 46 at a frequency of twice a week.

On Day 28, mice with the inflammation symptom were chosen and divided into 3 groups so that the average of the inflammation points in each group was the same.

The three groups were: 1) Control: no treatment, 2) 250 $\mu$g: predonisolone phosphate was dissolved in a physiological saline solution and the mice in this group were injected intravenously with this solution at the dose of 250 $\mu$g per injection and 500 $\mu$g per week, and 3) 500 $\mu$g: predonisolone phosphate was dissolved in a physiological saline solution and the mice in this group were injected intravenously with this solution at the dose of 500 $\mu$g per injection and 1000 $\mu$g per week.

The number of the mice in these 4 groups was: Control: 2 mice, 250 $\mu$g: 2 mice, 500 $\mu$g: 2 mice.

The following results were obtained.

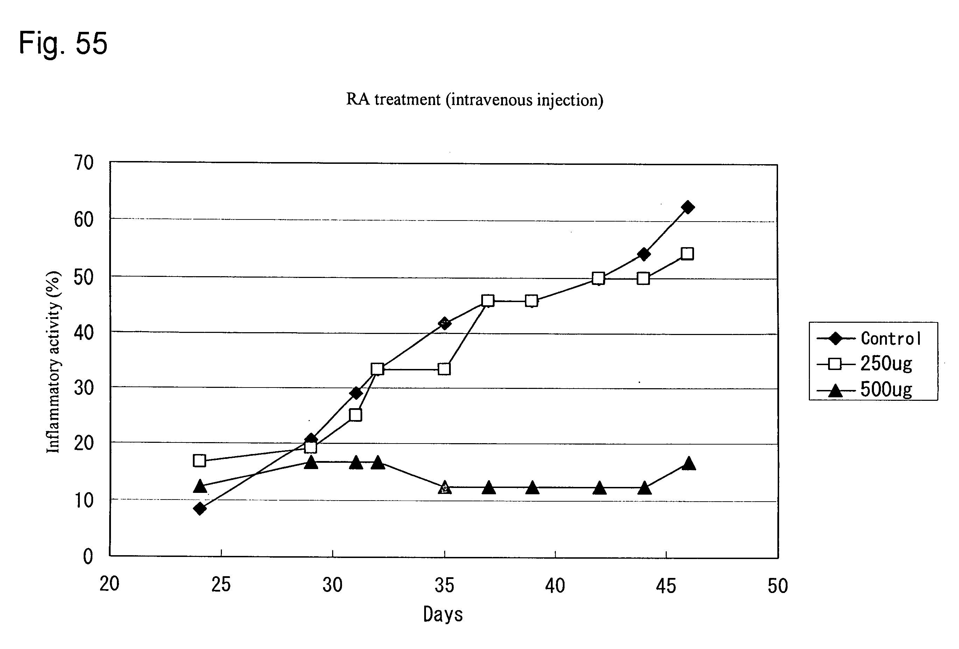

No significant difference was observed between Control group and 250 $\mu$g group. In 500 $\mu$g group, no increase in IA was observed and the progression of inflammation was suppressed (Fig. 55).

These results indicate the followings. It was discovered that to suppress the progression of inflammation in the present RA mouse model, a dose of predonisolone phosphate more than the minimum 500 $\mu$g/week up to 1000 $\mu$g/week was required. Considering the fact that in the experimental treatment by intravenous injection the progression of inflammation was suppressed by L-Pred-SLX group at the dose of 10 $\mu$g/injection, 20 $\mu$g per week, it may be possible to obtain the similar therapeutic effect as the conventional one by using the present DDS with a dose of maximum 1/50.

This suggests the possibility of reducing the side effects by reducing the systemic dose while maintaining the medicinal drug concentration at the lesion site. It may also possible to obtain higher medicinal effect by increasing the medicinal drug concentration at the lesion site by increasing the systemic dose to the level where no side effects would be produced.

The disclosures of all publications, patents and patent applications cited in the specification are entirely incorporated herein by reference.

Industrial Applicability

A liposome having excellent stability was successfully obtained by controlling type and amount of composition constituting a liposome. Furthermore, a liposome having more excellent properties including stability than a conventional liposome was successfully obtained by hydrophilizing the liposome with a specific hydrophilic compound.

As shown in Examples of the present invention, liposomes were prepared by binding various types of sugar chains and a protein (linker) derived from a living organism including a human-derived protein such as human serum albumin, to the liposomes, and then, *in-vivo* dynamic of liposomes to various tissues in mice, particularly uptake of the liposomes into cancer tissues was analyzed by using Ehrlich's mice carrying solid cancer. As a result, it was found that *in-vivo* dynamic of liposomes to various tissues can be controlled (accelerated or suppressed) in an actual living body by using the difference in molecular structure of the sugar chains. Based on this, it was demonstrated that an efficient targeting ability to not only cancer tissues but also tissues of interest (such as blood, liver, spleen, lung, brain, small intestinal tract, heart, thymus gland, kidney, pancreas, muscle, large intestinal tract, bone, bone marrow, eyes, cancer tissue, inflammatory tissue and lymph node) can be added to a DDS material. As described above, the present invention successfully provides a liposome capable of controlling targetability, which is extremely useful in the medical/pharmaceutical field. Furthermore, a sugar-chain bonded liposome exhibiting excellent targetability was obtained by controlling the density of the sugar chain to be bonded to the surface of the liposome.

In addition, the liposome modified by a sugar chain of the present invention has an epoch-making feature that it shows excellent intestinal absorption, in other words, it can be administered through a new administration route not observed in a conventional preparation using a liposome. Furthermore, intestinal absorption and in-vivo dynamics to various tissues (blood, liver, spleen, lung, brain, small intestinal tract, heart, thymus gland, kidney, pancreas, muscle, large intestinal tract, bone, bone marrow, eyes, cancer tissue, inflammatory tissue and lymph node) can be controlled by setting the amount and type of sugar chain bonded to a liposome. As a result, it is possible to efficiently and safely deliver a medicinal drug or a gene by way of the intestinal tract and blood to a living tissue without side effects. Hence, the liposome of the present invention is particularly useful in the medical/pharmaceutical field.

Moreover, when an anticancer drug is encapsulated in he hydrophilic liposome modified and unmodified by a sugar chain according to the present invention and administered to a subject orally or parenterally, the anticancer drug can be targeted to a cancer tissue and accumulated there, thereby suppressing growth of the cancer.

The targeting liposome of the present invention, when it encapsulates a compound having an appropriate medicinal effect therein, recognizes a lectin *in* vivo with the help of a sugar chain bonded on the surface of the liposome, and reaches a tissue/organ expressing the lectin. When the liposome is taken up by the cells of the tissue/organ, a compound having a medicinal effect produces its effects therein and works as a therapeutic drug and diagnostic drug. If an anticancer drug is encapsulated, the liposome is used as a therapeutic drug for cancer. Alternatively, the intestinal-absorption controlled liposome can be easily absorbed from the intestinal track. Therefore, a substance having a medicinal effect and encapsulated in the liposome can be swiftly produce its effect *in vivo* in the same manner as in the targeting liposome.

**Claims**

1. A liposome modified by a sugar chain bonded to the membrane of the liposome.

2. The liposome modified by a sugar chain according to claim 1, wherein a constitutional lipid of the liposome comprises phosphatidylcholines (0 to 70% by mole), phosphatidylethanolamines (0 to 30% by mole); not less than one lipid (0 to 30% by mole) selected from the group consisting of phosphatidic acids, long-chain alkyl phosphates and dicetyl phosphates; not less than one lipid (0 to 40% by mole) selected from the group consisting of gangliosides, glycolipids, phosphatidylglycerols, and sphingomyelins; and cholesterols (0 to 70% by mole).

3. The liposome modified by a sugar chain according to claim 2, wherein the at least one lipid selected from the group consisting of gangliosides, glycolipids, phosphatidylglycerols, sphingomyelins and cholesterols gathers on the surface of the liposome to form a raft.

4. The liposome modified by a sugar chain according to any one of claims 1 to 3, wherein the sugar chain controlled in type and density is bonded.

5. The liposome modified by a sugar chain according to any one of claims 1 to 4, wherein the liposome has a particle diameter of 30 to 500 nm.

6. The liposome modified by a sugar chain according to claim 5, wherein the liposome has a particle diameter of 50 to 350 nm.

7. The liposome modified by a sugar chain according to any one of claims 1 to 6, wherein the liposome has a zeta potential of -50 to 10 mV.

8. The liposome modified by a sugar chain according to claim 7, wherein the liposome has a zeta potential of -40 to 0 mV.

9. The liposome modified by a sugar chain according to claim 8, wherein the liposome has a zeta potential of -30 to -10 mV.

10. The liposome modified by a sugar chain according to any one of claims 1 to 9, wherein the sugar chain is bonded to the membrane of the liposome via a linker protein.

11. The liposome modified by a sugar chain according to claim 10, wherein the linker protein is a protein derived from the living body.

12. The liposome modified by a sugar chain according to claim 11, wherein the linker protein is a protein derived from a human.

13. The liposome modified by a sugar chain according to claim 12, wherein the linker protein is a human serum protein.

14. The liposome modified by a sugar chain according to claim 11, wherein the linker protein is human serum albumin or bovine serum albumin.

15. The liposome modified by a sugar chain according to any one of claims 1 to 14, wherein the linker protein is bonded onto a raft formed of at least one lipid selected from the group consisting of gangliosides, glycolipids, phosphatidylglycerols, sphingomyelins and cholesterols formed on the surface of the liposome.

16. The liposome modified by a sugar chain according to any one of claims 1 to 15, hydrophilized by binding a hydrophilic compound to the membrane of the liposome and/or the linker protein.

17. The liposome modified by a sugar chain according to claim 16, wherein the hydrophilic compound is a low molecular weight substance.

18. The liposome modified by a sugar chain according to claim 16 or 17, wherein the hydrophilic compound rarely causes steric hindrance to the sugar chain and does not prevent proceeding of a reaction of recognizing the sugar chain by a lectin on the membrane surface of a target cell.

19. The liposome modified by a sugar chain according to any one of claims 16 to 18, wherein the hydrophilic compound has a hydroxide group.

20. The liposome modified by a sugar chain according to any one of claims 16 to 19, wherein the hydrophilic compound is an amino alcohol.

21. The liposome modified by a sugar chain according to any one of claims 16 to 20, wherein the hydrophilic compound binds directly to the surface of the liposome membrane.

22. The liposome modified by a sugar chain according to claim 16, hydrophilized by a hydrophilic compound, wherein the hydrophilic compound is represented by the general formula (1):

$$X\text{-}R1(R2OH)n \qquad (1)$$

where R1 denotes a C1 to C40 linear or branched hydrocarbon chain; R2 is absent or represents a C1 to C40 linear or branched hydrocarbon chain; X represents a reactive functional group directly binding to a liposome lipid or a linker protein, or binding to a divalent crosslinking agent; and n is a natural number.

23. The liposome modified by a sugar chain according to claim 16, hydrophilized by a hydrophilic compound, wherein the hydrophilic compound is represented by the general formula (2):

$$H_2N\text{-}R3\text{-}(R4OH)n \qquad (2)$$

where R3 denotes a C1 to C40 linear or branched hydrocarbon chain; R4 is absent or represents a C1 to C40 linear or branched hydrocarbon chain; $H_2N$ represents a reactive functional group directly binding to a liposome lipid or a linker protein, or binding to a divalent crosslinking agent; and n is a natural number.

24. The liposome modified by a sugar chain according to claim 16, hydrophilized by a hydrophilic compound, wherein the hydrophilic compound is represented by the general formula (3):

$$H_2N\text{-}R5\text{-}(OH)n \qquad (3)$$

where R5 denotes a C1 to C40 straight and branched hydrocarbon chain; $H_2N$ represents a reactive functional group directly binding to a liposome lipid or a linker protein, or binding to a divalent crosslinking agent; and n is a natural number.

25. The liposome modified by a sugar chain according to claim 16, wherein the membrane of the liposome and/or the linker protein are hydrophilized by covalently bonding a hydrophilic compound being a tris(hydroxyalkyl)aminoalkane to the membrane of the liposome and/or the linker protein.

26. The liposome modified by a sugar chain according to claim 16, wherein the membrane of the liposome and/or the linker protein are hydrophilized by covalently bonding, to the membrane of the liposome and/or the linker protein, a hydrophilic compound selected from the group consisting of tris(hydroxymethyl)aminoethane, tris(hydroxyethyl) aminoethane, tris(hydroxypropyl)aminoethane, tris(hydroxymethyl)aminomethane, tris(hydroxyethyl)aminomethane, tris(hydroxypropyl)aminomethane, tris(hydroxylmethyl)aminopropane, tris(hydroxyethyl)aminopropane, and tris(hydroxypropyl)aminopropane.

27. The liposome modified by a sugar chain according to any one of claims 1 to 26, wherein the liposome modified by a sugar chain is targeted to a lectin selected from the group consisting of a selectin, DC-SIGN, DC-SGNR, a C-type lectin including colectin and mannose binding lectin, an I-type lectin including siglec, a P-type lectin including a mannose-6-phosphate receptor, an R-type lectin, an L-type lectin, an M-type lectin, and galectin, all serving as a receptor present on the cellular membrane of each tissue.

28. The liposome modified by a sugar chain according to claim 27, wherein the liposome modified by a sugar chain is targeted to a selectin selected from the group consisting ofE-selectin, P-selectin, and L-selectin.

29. The liposome modified by a sugar chain according to any one of claims 1 to 28, wherein a binding density of the sugar chain bonded to the liposome is 1 to 30000 per liposome particle when the linker protein is used, and 1 to 500000 per liposome particle when the linker protein is not used.

30. The liposome modified by a sugar chain according to any one of claims 1 to 28, wherein a binding density of the sugar chain bonded to the liposome is 1 to 60 per protein molecule to be bonded to the liposome.

31. The liposome modified by a sugar chain according to any one of claims 1 to 30, wherein the liposome is capable of being absorbed from the intestinal tract.

32. The liposome modified by a sugar chain according to any one of claims 1 to 31, having high targetability to a tissue or an organ selected from the group consisting of blood, liver, spleen, lung, brain, small intestinal tract, heart, thymus gland, kidney, pancreas, muscle, large intestinal tract, bone, bone marrow, eyes, cancer tissue, inflammatory tissue and lymph node.

33. The liposome modified by a sugar chain according to any one of claims 1 to 32, wherein the sugar chain is bonded to the membrane of the liposome and selected from the group consisting of $\alpha$-1,2-mannobiose disaccharide, $\alpha$-1,3-mannobiose disaccharide, $\alpha$-1,4-mannobiose disaccharide, $\alpha$-1,6-mannobiose disaccharide, $\alpha$-1,3/$\alpha$-1,6-mannotriose trisaccharide, oligomannose-3 pentasaccharide, oligomannose-4b hexasaccharide, oligomannose-5 heptasaccharide, oligomannose-6 octasaccharide, oligomannose-7 nonasaccharide, oligomannose-8 decasaccharide, and oligomannose-9 undecasaccharide, 3'-sialyllactose trisaccharide, 6'-sialyllactose trisaccharide, 3'-sialyllactosamine trisaccharide, 6'-sialyllactosamine trisaccharide, Lewis X trisaccharide, sialyl Lewis X tetrasaccharide, lactose disaccharide, 2'-fucosyllactose trisaccharide, difucosyllactose tetrasaccharide, and 3-fucosyllactose trisaccharide.

34. A liposome preparation prepared by encapsulating a medicinal drug or a gene in the liposome according to any one of claims 1 to 33.

35. The liposome preparation according to claim 34, wherein the medicinal drug is selected from the group consisting of medicinal drugs for tumors such as an anticancer drug based on an alkylating compound, metabolic antagonist, plant derived anticancer drug, anticancer antibiotic, BRM, cytokine, anticancer drug based on a platinum complex, immunotherapeutic drug, hormonal anticancer drug, and monoclonal antibody; medicinal drugs for the central nerve; medicinal drugs for the peripheral nervous system/sensory organ; therapeutic drugs for a respiratory disease; medicinal drugs for a circulatory organ; medicinal drugs for a digestive organ; hormonal medicinal drugs; medicinal drugs for a urinary/genital organ; medicinal drugs for external application; vitamins/analeptics; medicinal drugs for blood and body fluid; metabolic medicine; antibiotic/chemotherapeutic agents; medicinal drugs for medical check; anti-inflammatory agents; medicinal drugs for eye disorder; medicinal drugs for the central nervous system; medicinal drugs for the autoimmune system; medicinal drugs for the circulatory organ; medicinal drugs for lifestyle-related diseases such as diabetes and hyperlipemia; various oral, pulmonary, transdermal or transmucosal drugs; adenocortical hormones; immunosuppressive agents; antibiotics; anti-viral agents; neovascularization inhibitors; cytokines;

chemokines; anti-cytokine antibodies; anti-chemokine antibodies; anti-cytokine/chemokine receptor antibodies; nucleic acid preparations involved in gene therapy such as siRNA, miRNA, smRNA, antisense ODN and DNA; neuroprotective factors; and various antibody medicines.

**36.** The liposome preparation according to claim 34 or 35, which is an oral preparation.

**37.** The liposome preparation according to claim 34 or 35, which is a parenteral preparation.

**38.** The liposome preparation according to claim 35, wherein the medicinal drug comprising a liposome modified by a sugar chain is doxorubicin.

**39.** An anticancer drug comprising the liposome preparation according to claim 35, wherein the medicinal drug is a drug for a tumor.

**40.** The anticancer drug according to claim 39 which is to be orally administered.

**41.** The anticancer drug according to claim 39 which is to be parenterally administered.

**42.** A liposome whose membrane is hydrophilized and which has no sugar chain bonded onto surface thereof.

**43.** The liposome according to claim 42, wherein a constitutional lipid of the liposome comprises phosphatidylcholines (0 to 70% by mole), phosphatidylethanolamines (0 to 30% by mole); not less than one lipid (0 to 30% by mole) selected from the group consisting of phosphatidic acids, long-chain alkyl phosphates and dicetyl phosphates; not less than one lipid (0 to 40% by mole) selected from the group consisting of gangliosides, glycolipids, phosphatidylglycerols, and sphingomyelins; and cholesterols (0 to 70% by mole).

**44.** The liposome according to claim 42 or 43, further comprising a protein.

**45.** The liposome according to any one of claims 42 to 44, wherein the membrane of the liposome and/or the linker protein are hydrophilized by binding a hydrophilic compound thereto.

**46.** The liposome according to claim 45, wherein the hydrophilic compound is a low molecular weight substance.

**47.** The liposome according to claim 45 or 46, wherein the hydrophilic compound rarely causes steric hindrance to the sugar chain and does not prevent proceeding of a reaction of recognizing the sugar chain by a lectin on the membrane surface of a target cell.

**48.** The liposome according to any one of claims 45 to 47, wherein the hydrophilic compound has a hydroxide group.

**49.** The liposome according to any one of claims 45 to 48, wherein the hydrophilic compound is an amino alcohol.

**50.** The liposome according to any one of claims 45 to 49, wherein the hydrophilic compound binds directly to the surface of the liposome membrane.

**51.** The liposome according to claim 45, wherein the hydrophilic compound of a low molecular weight has at least one OH group.

**52.** The liposome according to claim 45, wherein the hydrophilic compound is represented by the general formula (1):

$$X\text{-}R1(R2OH)n \qquad (1)$$

where R1 denotes a C1 to C40 linear or branched hydrocarbon chain; R2 is absent or represents a C1 to C40 linear or branched hydrocarbon chain; X represents a reactive functional group directly binding to a liposome lipid or a linker protein or binding to a divalent crosslinking agent; and n is a natural number.

**53.** The liposome according to claim 45, wherein the hydrophilic compound is represented by the general formula (2):

$$H_2N\text{-}R3\text{-}(R4OH)n \qquad (2)$$

where R3 denotes a C1 to C40 linear or branched hydrocarbon chain; R4 is absent or represents a C1 to C40 linear or branched hydrocarbon chain; $H_2N$ represents a reactive functional group directly binding to a liposome lipid or a linker protein or binding to a divalent crosslinking agent; and n is a natural number.

54. The liposome according to claim 45, wherein the hydrophilic compound is represented by the general formula (3):

$$H_2N\text{-}R5\text{-}(OH)n \qquad (3)$$

where R5 denotes a C1 to C40 linear or branched hydrocarbon chain; $H_2N$ represents a reactive functional group directly binding to a liposome lipid or a linker protein or binding to a divalent crosslinking agent; and n is a natural number.

55. The liposome according to claim 45, wherein the membrane of the liposome and/or the linker protein are hydrophilized by covalently bonding a hydrophilic compound being a tris(hydroxyalkyl)aminoalkane to the membrane of the liposome and/or the linker protein.

56. The liposome according to claim 55, wherein the membrane of the liposome is hydrophilized by covalently bonding, to the membrane of the liposome, a hydrophilic compound selected from the group consisting of tris(hydroxymethyl) aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxylmethyl)aminopropane, tris(hydroxyethyl)aminopropane, and tris(hydroxypropyl)aminopropane.

57. A liposome preparation prepared by encapsulating a medicinal drug or a gene in the liposome according to any one of claims 42 to 56.

58. The liposome preparation according to claim 57, wherein the medicinal drug is selected from the group consisting of medicinal drugs for tumors such as an anticancer drug based on an alkylating compound, metabolic antagonist, plant derived anticancer drug, anticancer antibiotic, BRM, cytokine, anticancer drug based on a platinum complex, immunotherapeutic drug, hormonal anticancer drug, and monoclonal antibody; medicinal drugs for the central nerve; medicinal drugs for the peripheral nervous system/sensory organ; therapeutic drugs for a respiratory disease; medicinal drugs for a circulatory organ; medicinal drugs for a digestive organ; hormonal medicinal drugs; medicinal drugs for a urinary/genital organ; medicinal drugs for external application; vitamins/analeptics; medicinal drugs for blood and body fluid; metabolic medicine; antibiotic/chemotherapeutic agents; medicinal drugs for medical check; anti-inflammatory agents; medicinal drugs for eye disorder; medicinal drugs for the central nervous system; medicinal drugs for the autoimmune system; medicinal drugs for the circulatory agent; medicinal drugs for lifestyle-related diseases such as diabetes and hyperlipemia; various oral, pulmonary, transdermal or transmucosal drugs; adenocortical hormones; immunosuppressive agents; antibiotics; anti-viral agents; neovascularization inhibitors; cytokines; chemokines; anti-cytokine antibodies; anti-chemokine antibodies; anti-cytokine/chemokine receptor antibodies; nucleic acid preparations involved in gene therapy such as siRNA, miRNA, smRNA, antisense ODN and DNA; neuroprotective factors; and various antibody medicines.

59. The liposome preparation according to claim 57 or 58, being a preparation for peroral administration.

60. The liposome preparation according to claim 57 or 58, which is a parenteral preparation.

61. An anticancer drug comprising the liposome preparation according to claim 58, wherein the medicinal drug is a drug for a tumor.

62. The liposome preparation according to claim 61, wherein the medicinal drug is doxorubicin.

63. The anticancer drug according to claim 61 or 62, which is to be orally administered.

64. The anticancer drug according to claim 61 or 62, which is to be parentally administered.

65. A cosmetic composition comprising a liposome preparation prepared by encapsulating a cosmetic in the liposome modified by a sugar chain according to any one of claims 1 to 33.

**66.** The cosmetic composition according to claim 65, which is a preparation to be transdermally administered.

**67.** The cosmetic composition according to claim 65 or 66, wherein the cosmetic is vitamin A or vitamin E.

**68.** A food composition comprising a liposome preparation prepared by encapsulating a food in the liposome modified by a sugar chain according to any one of claims 1 to 33.

**69.** The food composition according to claim 68, which is a preparation for peroral administration.

**70.** The food composition according to claim 68 or 69, wherein the food is a nutritional supplement.

**71.** The food composition according to claim 69 or 70, wherein the food is vitamin A or vitamin E.

**72.** A cosmetic composition comprising a liposome preparation prepared by encapsulating a cosmetic in the liposome according to any one of claims 42 to 56.

**73.** The cosmetic composition according to claim 72, being a preparation for transdermal administration.

**74.** The cosmetic composition according to claim 72 or 73, wherein the cosmetic is vitamin A or vitamin E.

**75.** A food composition comprising a liposome preparation prepared by encapsulating a food in the liposome according to any one of claims 42 to 56.

**76.** The food composition according to claim 75, which is a preparation for oral administration.

**77.** The food composition according to claim 75 or 76, wherein the food is a nutritional supplement.

**78.** The food composition according to claim 76 or 77, wherein the food is vitamin A or vitamin E.

Fig. 1

EP 1 655 038 A1

Fig. 2

Fig. 3

Fig. 4

Fig. 5

EP 1 655 038 A1

Fig. 6

Fig. 7

# Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

EP 1 655 038 A1

Fig. 21

Fig. 22

Fig. 23

# Fig. 24

Fig. 25

EP 1 655 038 A1

Fig. 26

Fig. 27

EP 1 655 038 A1

Fig. 28

Fig. 29

Fig. 30

EP 1 655 038 A1

Fig. 31

Radioactivity (% dose/ml blood)

3FL-1   3FL-2   3FL-3   TRIS

Liposome complexes

EP 1 655 038 A1

Fig. 32

Fig. 33

Fig. 34

Fig. 35

Fig. 36

Fig. 37

Fig. 38

## Fig. 39

EP 1 655 038 A1

## Fig. 40

Group administered by physiological saline     Group administered by sugar-chain bonded liposome

EP 1 655 038 A1

Fig. 41

Fig. 42

Group administered by physiological saline    Group administered by sugar-chain bonded liposome

EP 1 655 038 A1

Fig. 43

EP 1 655 038 A1

Fig. 44

Fig. 45

EP 1 655 038 A1

Fig. 46

# Fig. 47

(A)free doxorubicin
(dose 1.4 mg/kg)

(B) Doxorubicin encapsulated liposome
(dose 0.42 mg/kg)

EP 1 655 038 A1

## Fig. 48

Days after transplantation of cancer cells

Fig. 49

(A) Group administered by physiological saline

(B) Group administered by doxorubicin-encapsulated liposome

Fig. 50

Weight of tumor (g)

1.5

1.0

0.5

0.0

Group administered by physiological saline

Group administered by free doxorubicin (dose 1.4 mg/kg)

Group administered by doxorubicin-encapsulated liposome (dose 0.42 mg/kg)

Fig. 51

EP 1 655 038 A1

Fig. 52

## Fig. 53

RA treatment (intravenous injection)

EP 1 655 038 A1

# Fig. 54

RA treatment (peroral administration)

EP 1 655 038 A1

## Fig. 55

RA treatment (intravenous injection)

Legend:
- Control
- 250ug
- 500ug

X-axis: Days (20, 25, 30, 35, 40, 45, 50)

Y-axis: Inflammatory activity (%) (0, 10, 20, 30, 40, 50, 60, 70)

## INTERNATIONAL SEARCH REPORT

| International application No. |
| --- |
| PCT/JP2004/011291 |

| A. CLASSIFICATION OF SUBJECT MATTER |
| --- |
| Int.Cl$^7$ A61K9/127, 47/24, 47/26, 47/28, 47/36, 47/42, 7/00, A61P35/00, A23L1/302, 1/00 |

According to International Patent Classification (IPC) or to both national classification and IPC

| B. FIELDS SEARCHED |
| --- |
| Minimum documentation searched (classification system followed by classification symbols) |
| Int.Cl$^7$ A61K9/127, 47/24, 47/26, 47/28, 47/36, 47/42, 7/00, A61P35/00, A23L1/302, 1/00 |

Documentation searched other than minimum documentation to the extent that such documents are included in the fields searched

Jitsuyo Shinan Koho 1922–1996 Toroku Jitsuyo Shinan Koho 1994–2004
Kokai Jitsuyo Shinan Koho 1971–2004 Jitsuyo Shinan Toroku Koho 1996–2004

Electronic data base consulted during the international search (name of data base and, where practicable, search terms used)
CAPLUS(STN), REGISTRY(STN), MEDLINE(STN), BIOSIS(STN), EMBASE(STN)

| C. DOCUMENTS CONSIDERED TO BE RELEVANT | | |
| --- | --- | --- |
| Category* | Citation of document, with indication, where appropriate, of the relevant passages | Relevant to claim No. |
| X | US 2003/0143267 A1 (YAMAZAKI, Noboru), 31 July, 2003 (31.07.03), Full text; particularly, Claims 1 to 15; examples 1 to 17 & JP 2003-226638 A & JP 2003-226647 A | 1–78 |
| X Y | YAMAZAKI, Noboru et al., Prepatation and Characterization of Neoglycoprotein-liposome conjugates: a promising approach to developing drug deliverly materials applying sugar chain ligands, Trends in Glycoscience and Glycotechnology, 2001, Vol.13, No.71, pages 319 to 329, full text | 1–15,27–41 16–26,65–78 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 20 October, 2004 (20.10.04) | 09 November, 2004 (09.11.04) |

| Name and mailing address of the ISA/ Japanese Patent Office | Authorized officer |
| --- | --- |
| Facsimile No. | Telephone No. |

Form PCT/ISA/210 (second sheet) (January 2004)

**INTERNATIONAL SEARCH REPORT**

International application No.

PCT/JP2004/011291

| C (Continuation). DOCUMENTS CONSIDERED TO BE RELEVANT | | |
|---|---|---|
| Category* | Citation of document, with indication, where appropriate, of the relevant passages | Relevant to claim No. |
| X | KOLE, Labanyamoy et al., Synergistic effect of interferon-γ and mannosylated loposome-incorporated doxorubisin in the therapy of experimental visceral leishmaniasis, 1999, Vol.180, pages 811 to 820, full text | 1-5,7-9,27, 29,32,34,35, 37,38 |
| Y | | 6,10-26,28, 31,33,36, 39-41,65-78 |
| X | JP 2001-81044 A (Tokai University), 27 March, 2001 (27.03.01), Full text; particularly, Claims 1, 2 | 1-9,27,29, 31-34,37 |
| Y | | 10-26,28,30, 35,36,38-41, 65-78 |
| X | TAKEUCHI, Hirofumi et al., Passive targeting of doxorubicine with polymer coated liposomes in tumor bearing rat, Biol.Pharm.Bull., 2001, Vol. 24, pages 795 to 799, full text | 42,43,45,48, 50,57,58, 60-62,64 |
| Y | | 44,46,47,49, 51-56,59,63 |
| X | KAMPS, Jan A.A.M. et al., Massive targeting of liposomes, surfase-modified with anionized albumins, to hepatic endothelial cells, Proc. Natl.Acas.Sci. U.S.A., 1997, Vol.94, pages 11681 to 11685, full text | 42-45,50 |
| Y | | 46-49,51-64 |
| X | HONG, Ruey-Long et al., Direct comparison of liposomal doxorubicin with or without poluethylene glycol coating in C-26 tumor-bearing mice: Is suface coating with polyethylene glycol beneficial?, Clinical Cancer Research, 1999, Vol.5, pages 3645 to 3652, full text | 42,43,45,48, 57,58,60-62, 64 |
| Y | | 44,46,47, 49-56,59,63 |

Form PCT/ISA/210 (continuation of second sheet) (January 2004)

# INTERNATIONAL SEARCH REPORT

International application No.

PCT/JP2004/011291

**Box No. II     Observations where certain claims were found unsearchable (Continuation of item 2 of first sheet)**

This international search report has not been established in respect of certain claims under Article 17(2)(a) for the following reasons:

1. ☐ Claims Nos.:

   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:
         (See extra sheet.)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**          ☐ The additional search fees were accompanied by the applicant's protest.

                               ☐ No protest accompanied the payment of additional search fees.

Form PCT/ISA/210 (continuation of first sheet (2)) (January 2004)

**INTERNATIONAL SEARCH REPORT**

International application No.

PCT/JP2004/011291

Continuation of Box No.III of continuation of first sheet(2)

It appears that the special technical feature of the invention according to claim 1 relating to "a sugar chain-modified liposome having a sugar chain bound to the liposome membrane" resides in bonding sugar chain to the liposome membrane, while the special technical feature of the invention according to claim 42 relating to "a liposome wherein the liposome membrane has been made hydrophilic and no sugar chain is bound to the surface thereof" resides in making the liposome membrane hydrophilic without binding to any sugar chain. Thus, no technical feature common to them can be found.

Such being the case, these inventions are not considered as being so linked as to form a single general inventive concept.

Form PCT/ISA/210 (extra sheet) (January 2004)